# EUROPEAN PATENT APPLICATION

(11) **EP 4 674 439 A1**
(43) Date of publication of application: **07.01.2026**
(21) Application number: 24764053.5
(22) Date of filing: 01.03.2024
(51) Int. Cl.: A61K 47/68, A61K 31/395, A61K 39/395, A61K 47/60, A61K 47/65, A61K 51/10, A61P 35/00, A61P 43/00, G01N 33/553, A61K 101/02, A61K 103/00, A61K 103/20, A61K 103/32, A61K 103/40

(54) **RADIOACTIVE METAL LABELED ANTIBODY, RADIOPHARMACEUTICAL, AND COMPOUND**

(30) Priority: 02.03.2023 JP 2023032317; 30.11.2023 JP 2023203279
(71) Applicant: Nihon Medi-Physics Co., Ltd., Tokyo 136-0075 (JP); Kyoto University, Kyoto-shi, Kyoto 606-8501 (JP); Shoken Gakuen, Maebashi-shi, Gunma 371-0846 (JP)
(72) Inventor: MAYA, Yoshifumi, Tokyo 136-0075 (JP); ONO, Masahiro, Kyoto-shi, Kyoto 606-8501 (JP); WATANABE, Hiroyuki, Kyoto-shi, Kyoto 606-8501 (JP); NAKASHIMA, Kazuma, Kyoto-shi, Kyoto 606-8501 (JP); TSUJI, Shoutaro, Maebashi-shi, Gunma 371-0823 (JP)
(74) Representative: Hoffmann Eitle
(86) International application number: PCT/JP2024/007907
(87) International publication number: WO 2024/181576

(57) **Abstract**

The antibody is a radioactive metal-labeled antibody including an antibody labeled with a radioactive metal. The radioactive metal-labeled antibody further includes a functional chelate linker conjugated to the antibody, and, the functional chelate linker includes: a chelating moiety capable of coordinating to a radioactive metal ion; and a functional unit having a function of enhancing intracellular retention. The functional unit preferably has a structure in which ethyleneimine or ethylene glycol is polymerized or a structure capable of binding to cathepsin B. The functional unit preferably has a structure in which ethyleneimine is polymerized with a polymerization degree of 1 to 7.

## Description

### Technical Field

The present invention relates to a radioactive metal-labeled antibody, a radioactive pharmaceutical, and a compound.

### Background Art

Radioactively labeled antibodies (hereinafter also referred to as "radioactively labeled antibodies") are expected to be useful as therapeutic agents in internal radiation therapy or as diagnostic agents in PET and the like. For example, ibritumomab tiuxetan is a combination of ibritumomab, which is an antibody specific to anti-CD20 antibody, with tiuxetan modified with DTPA, and the compound to which yttrium-90 is added has been approved in Japan as a therapeutic agent for CD20-positive lymphoma. In addition, although not approved in Japan, ¹³¹I-Tositumomab has been approved overseas as a therapeutic agent for CD20-positive lymphoma.

Further, although trastuzumab is an antibody specific to HER2, there have been reports of it being labeled with various radioactive nuclides such as indium-111, lutetium-177, copper-64, zirconium-89, astatine-211, and radioactive iodine, and its clinical application to non-invasive imaging and targeted radiotherapy of tumors that overexpress HER2 is being investigated.

As a challenge of radioiodine-labeled antibodies, it is known that iodotyrosine rapidly diffuses out of cells due to endocytosis and lysosomal degradation. In response to such a challenge, Non Patent Literature 1 describes that by focusing on the accumulation of In-111DOTA-lysine in target cells and combining iodotyrosine labeled with radioactive iodine with DOTA, the intracellular retention of trastuzumab labeled with radioactive iodine was enhanced. In addition, Non Patent Literature 2 describes that by introducing a guanidino group, which has high basicity, into trastuzumab, the intracellular retention of radioactive iodine-labeled trastuzumab can be enhanced.

In addition, Non Patent Literature 3 describes that by introducing a structure capable of binding to cathepsin B into a peptide targeting the gastrin-releasing peptide receptor (GRPR), the retention of the peptide within tumor cells can be enhanced.

Furthermore, Non Patent Literature 4 describes that a peptide having an epoxysuccinyl group exhibits excellent affinity for the active site of cathepsin B.

### Citation List

### Non Patent Literature

Non Patent Literature 1: C. Andrew Boswell et al., J. Med. Chem. 2013, 56, 9418-9426
Non Patent Literature 2: Satish K. Chitneni et al. Molecules, 2019, 24, 3907
Non Patent Literature 3: Wenting Zhang et al., J. Nucl. Med., 2020, 61, 443-450
Non Patent Literature 4: N. Schaschke et al., FEBS Lett., 2000, 482, 91-96

### Summary of Invention

As described in Non Patent Literature 1 and 2, conventionally, antibodies labeled with radioactive metal ions (hereinafter also referred to as "radioactive metal-labeled antibodies") have been considered to be retained within cells. However, among antibodies, there are some that are promptly excreted from inside the cell to the outside. Also in radioactive metal-labeled antibodies, it is expected that enhancing the radioactivity accumulation within target cells will further improve the therapeutic efficacy.

In addition, as described above, Non Patent Literature 3 describes that the retention of a peptide targeting GRPR in tumor cells is enhanced by introducing a structure capable of binding to cathepsin B, but the introduction of a structure capable of binding to cathepsin B to a radioactive metal-labeled antibody was not studied in the same document.

Therefore, an object of the present invention is to provide a radioactive metal-labeled antibody capable of enhancing the radioactivity accumulation within target cells.

According to the present invention, there is provided a radioactive metal-labeled antibody comprising an antibody labeled with a radioactive metal, wherein
the radioactive metal-labeled antibody further comprises a functional chelate linker conjugated to the antibody, and
the functional chelate linker includes:
   a chelating moiety capable of coordinating to a radioactive metal ion; and
   a functional unit having a function of enhancing intracellular retention.

In addition, according to the present invention, there is provided a compound including, in its structure:
a chelating moiety capable of coordinating to a radioactive metal ion;
a functional unit having a function of enhancing intracellular retention; and
an antibody binding site including a binding site with an antibody.

### Brief Description of Drawings

[Fig. 1] Fig. 1 is a graph showing a percentage of radioactivity in an internalized fraction and a medium fraction, as well as the ratio thereof, in Examples 4-1 to 4-3 and Comparative Examples 4-1 to 4-3.
[Fig. 2] Fig. 2 is a graph showing a percentage of radioactivity in a dissociated fraction and a degraded fraction, as well as the ratio thereof, in Examples 4-1 to 4-3 and Comparative Examples 4-1 to 4-3.
[Fig. 3] Fig. 3 is a graph showing an area under the curve (AUC) in blood and tumor, as well as the ratio thereof, in Examples 5-1 to 5-3 and Comparative Examples 5-1 to 5-3.
[Fig. 4] Fig. 4 is a SPECT/CT image of [¹¹¹In]In-TMDPEI2 in Example 7-1.
[Fig. 5] Fig. 5 is a graph showing an area under the curve (AUC) in blood and tumor, as well as the ratio thereof, in Examples 5-4 to 5-6.
[Fig. 6] Fig. 6 is an autoradiogram of gel after polyacrylamide gel electrophoresis in Example 4-7 and Comparative Example 4-4.
[Fig. 7] Fig. 7 is a graph showing a percentage of radioactivity in an internalized fraction and a degraded fraction in Example 5-7 and Comparative Example 5-4.
[Fig. 8] Fig. 8 is an autoradiogram of gel after polyacrylamide gel electrophoresis in Example 6-7.
[Fig. 9] Fig. 9 is an enlarged view of a molecular weight around 100 kDa to 250 kDa in the autoradiogram shown in Fig. 8.
[Fig. 10] Fig. 10 is a graph showing a tumor/blood ratio of radioactivity accumulation and an area under the curve (AUC) in blood and tumor in Example 6-7 and Comparative Example 4-4.

### Description of Embodiments

Hereinafter, a radioactive metal-labeled antibody and a compound of the present invention will be described based on preferable embodiments thereof. In the following description, "T to U [V]" (T and U are any numbers and [V] is a unit) means "not less than T [V] and not more than U [V]" unless otherwise specified. In addition, when a chiral carbon atom is present in the structure, unless otherwise specified, each may independently be in the S configuration or the R configuration.

The radioactive metal-labeled antibody of the present invention is a radioactive metal-labeled antibody including an antibody labeled with a radioactive metal, and further includes a functional chelate linker conjugated to the antibody. The functional chelate linker includes a chelating moiety capable of coordinating to a radioactive metal ion and a functional unit having a function of enhancing intracellular retention.

In addition, the compound of the present invention is a functional chelate linker prior to conjugation to an antibody, and its chemical structure can be broadly classified as including: a chelating moiety capable of coordinating to a radioactive metal ion; a functional unit having a function of enhancing intracellular retention; and an antibody binding unit including a binding site with an antibody.

The compound of the present invention may be a precursor compound used for labeling with a radioisotope such as a radioactive metal ion, that is, a precursor for radioactive labeling. The radioactive metal ion will be described later.

In one embodiment, in a compound of the present invention, a functional unit having a function of enhancing intracellular retention; and an antibody binding unit including a binding site with an antibody are bound via a chelating moiety. In other words, the chelating moiety is interposed between the antibody binding unit and the functional unit. Thus, when the compound of this embodiment is used as a radioactive metal-labeled antibody, the compound of this embodiment results in a radioactive metal-labeled antibody in which the chelating moiety is interposed between the antibody and the functional unit.

Such a compound is preferably represented by the following general Formula (I).

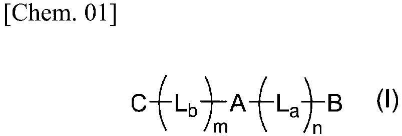

In Formula (I), A represents a chelating moiety capable of coordinating to a radioactive metal ion, B represents a functional unit having a function of enhancing intracellular retention, and C represents an antibody binding unit including a binding site with an antibody.

Lₐ represents a linker structure.

L_{b} represents a linker structure that is the same as or different from Lₐ.

m and n each independently represent 0 (zero) or 1.

When n represents 1, Lₐ is bound to any site of A.

When n represents 0, B is bound to any site of A.

When m represents 1, L_{b} is bound to A at a site different from the site where B or Lₐ is bound to A.

When m represents 0, C is bound to A at a site different from the site where B or Lₐ is bound to A.

In the general Formula (I), A and B and A and C may each independently have a linker structure and be indirectly bound, or may be directly bound without having a linker structure. In Formula (I), when a linker structure is provided between A and B and between A and C, the linker structures may be the same or different. One or more B are directly or indirectly bound to A.

Details of the linker structure will be described later.

In the general Formula (I), it is preferable that the chelating moiety represented by the symbol A have first and second amino acid residues, the chelating moiety be bound to the antibody binding unit represented by the symbol C via the first amino acid residue, and the chelating moiety be bound to the functional unit represented by the symbol B via the second amino acid residue.

Preferably, the first amino acid residue is a lysine residue, and the second amino acid residue is a glutamine residue.

The fact that the compound of the present invention has such a configuration has an advantage that both easiness of synthesis and stability of a chemical structure can be achieved.

Next, another embodiment of the compound of the present invention will be described. In the compound of the present invention in another embodiment, the antibody binding unit including the binding site with the antibody and the chelating moiety are bound via the functional unit having a function of enhancing intracellular retention. In other words, a functional unit is interposed between the antibody binding unit and the chelating moiety.

Such a compound is preferably represented by the following general Formula (II).

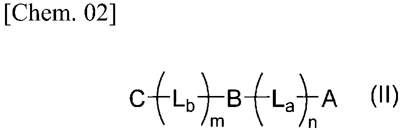

In Formula (II), A represents a chelating moiety capable of coordinating to a radioactive metal ion, B represents a functional unit having a function of enhancing intracellular retention, and C represents an antibody binding unit including a binding site with an antibody.

Lₐ represents a linker structure.

L_{b} represents a linker structure that is the same as or different from Lₐ.

m and n each independently represent 0 (zero) or 1.

When n represents 1, Lₐ is bound to any site of B.

When n represents 0, A is bound to any site of B.

When m represents 1, L_{b} is bound to A at a site different from the site where A or Lₐ is bound to B.

When m represents 0, C is bound to A at a site different from the site where A or Lₐ is bound to B.

In the general Formula (II), A and B and B and C may each independently have a linker structure and be indirectly bound, or may be directly bound without having a linker structure. In Formula (II), when a linker structure is provided between A and B and between B and C, the linker structures may be the same or different.

From the viewpoint of easiness of production and economy, it is particularly preferable that the compound of the present invention be represented by the general Formula (I), that is, the functional unit and the antibody binding unit be bound via a chelating moiety.

Hereinafter, matters applicable to each of the above-described embodiments will be described.

Unless otherwise specified, the following description is appropriately applied to the description of the general Formula (I) and the general Formula (II), and may be appropriately combined and employed.

When the compound of the present invention is used as a radioactive metal-labeled antibody, from the viewpoint of sufficiently enhancing the affinity between the compound of the present invention and the radioactive metal ion to suppress release of the radioactive metal ion from the radioactive metal-labeled antibody before being taken up by the target cell, it is preferable that in the Formulae (I) and (II), A have a cyclic structure, the cyclic structure have two or more nitrogen atoms, and each nitrogen atom be linked with two or more adjacent carbon atoms interposed therebetween, or A have a chain structure, the chain structure have two or more nitrogen atoms, and each nitrogen atom be linked with two or more adjacent carbon atoms interposed therebetween.

In the Formulae (I) and (II), when A has a cyclic structure, the skeleton of the cyclic structure may be composed of only nitrogen atoms and carbon atoms, or may be composed of oxygen atoms in addition to nitrogen atoms and carbon atoms. The bonds between carbon atoms in the cyclic structure may be a chain or may form a cyclic structure.

In the Formulae (I) and (II), when A has a chain structure, the bonds between carbon atoms in the chain structure may be divided by nitrogen atoms. The bonds between carbon atoms in the chain structure may be a chain or may form a cyclic structure.

In addition, in the Formulae (I) and (II), when A has a cyclic structure or a chain structure, it is preferable that A have a nitrogen-binding group directly bound to a nitrogen atom constituting the cyclic structure or the chain structure. As a specific example of the nitrogen-binding group, an atomic group containing one or more of a carboxy group, a phosphate group, an amide group, a benzene ring, and a pyridine ring is preferable, and the atomic group is more preferably a chain.

Further, in the Formulae (I) and (II), when A has a cyclic structure or a chain structure, provided that B is bound to any site of A and C is bound to any site of A different from the binding site of B, when B is bound to the above-described nitrogen-binding group via Lₐ or not via Lₐ, C is preferably bound to a site other than the nitrogen-binding group to which B is bound via L or not via L.

Specifically, in the Formulae (I) and (II), the chelating moiety capable of coordinating to the radioactive metal represented by the symbol A preferably has a structure derived from a compound represented by any one of the following Formulae (A1) to (A9), and more preferably has a structure derived from a compound represented by the following Formula (A1). That is, the compound of the present invention is preferably a derivative of a compound represented by any one of the following Formulae (A1) to (A9), and more preferably a derivative of a compound represented by the following Formula (A1). These structures can be appropriately selected according to the type of radioactive metal described later. Irrespective of a structure of the chelating moiety, when the compound of the present invention is used as a radioactive metal-labeled antibody, the affinity between the compound of the present invention and a radioactive metal ion can be sufficiently enhanced, and thus the release of the radioactive metal ion from the radioactive metal-labeled antibody before being taken up by the target cell is suppressed.

In the Formulae (I) and (II), examples of the chelating moiety represented by the symbol A include structures derived from the following compounds, but are not limited thereto and are applicable to others.

### <DOTA or Derivative Thereof>

1,4,7,10-Tetraazacyclododecane-1,4,7,10-tetraacetic acid (DOTA)
1,4,7,10-Tetraazacyclododecane-1,4,7,10-tetrapropionic acid (DOTPA)
1,4,7,10-Tetraazacyclododecane-1,4,7,10-tetramethylenephosphonic acid (DOTMP)
Hydroxypropyl-tetraazacyclododecanetriacetic acid (HP-DO3A)
(1R,4R,7R,10R)-α,α',α",α‴-Tetramethyl-1,4,7,10-tetraazacyclododecane-1,4,7,10-tetraacetic acid (DOTAMA)
1,4,7,10-Tetrakis(carbamoylmethyl) -1,4,7,10-tetraazacyclododecane (DOTAA)
1,4,7,10-Tetraazacyclododecane-1,4,7,10-tetrakis(acetamidomethylene)phosphonic acid (DOTA-A-AMP)
Tetraazacyclododecanedimethylenephosphonic acid (DO2P)
α-(2-Carboxyethyl)-1,4,7,10-tetraazacyclododecane-1,4,7,10-tetraacetic acid (DOTAGA)

### <HOPO or Derivative Thereof>

N,N',N",N‴-Tetra(1,2-dihydro-1-hydroxy-2-oxo-6-pyridinecarbonyl)-1,5,10,14-tetraazatetradodecane (1,2-HOPO)

### <TETA, PEPA, or Derivatives Thereof>

1,4,8,11-Tetraazacyclotetradecane-1,4,8,11-tetraacetic acid (TETA)
1,4,8,11-Tetraazacyclotetradecane-1,4,8,11-tetrapropionic acid (TETPA)
1,4,7,10,13-Pentaazacyclopentadecane-N,N',N",N‴,Nʺʺ-pentaacetic acid (PEPA)

### <Chain Structure (Octapa, Neunpa, or Derivatives Thereof)>

### Ethylenediaminetetraacetic acid (EDTA)

6,6'-((Ethane-1,2-diylbis((carboxymethyl)azanediyl))bis(methylene))dipicolinic acid (H₄octapa)
6,6'-({9-Hydroxy-1,5-bis(methoxycarbonyl)-2,4-di(pyridin-2-yl)-3,7-diazabicyclo[3.3.1]nonan-3,7-diyl}bis(methylene))dipicolinic acid (H₂bispa2)
1,2-[{6-(Carboxy)pyridin-2-yl}methylamino]ethane (H₂dedpa)
N,N"-Bis(6-carboxy-2-pyridylmethyl)-diethylenetriamine-N,N',N"-triacetic acid (H₅decapa)
N,N'-(Methylenephosphonate)-N,N'-[6-(methoxycarbonyl)pyridin-2-yl]methyl-1,2-diaminoethane (H₆phospa)
6,6'-(((((4-Isothiocyanatophenethyl)azanediyl)bis(ethane-2,1-diyl))bis((carboxymethyl)azanediyl))bis(methylene))dipicolinic acid (p-SCN-Bn-H₄neunpa)
6,6'-(((((4-Nitrophenethyl)azanediyl)bis(ethane-2,1-diyl))bis((carboxymethyl)azanediyl))bis(methylene))dipicolinic acid (p-NO₂-Bn-H₄neunpa)
6,6'-(((Azanediylbis(ethane-2,1-diyl))bis((carboxymethyl)azanediyl))bis(methylene))dipicolinic acid (H₅neunpa)

### <Macropa and Derivative Thereof>

6-(1,4,10,13-Tetraoxa-7,16-diazacyclooctadecane-N,N'-dimethyl)picolinic acid (H₂macropa)

### <NOTA or Derivative Thereof>

· 2-[4,7-Bis(carboxymethyl)-1,4,7-triazonan-1-yl]acetic acid (NOTA)

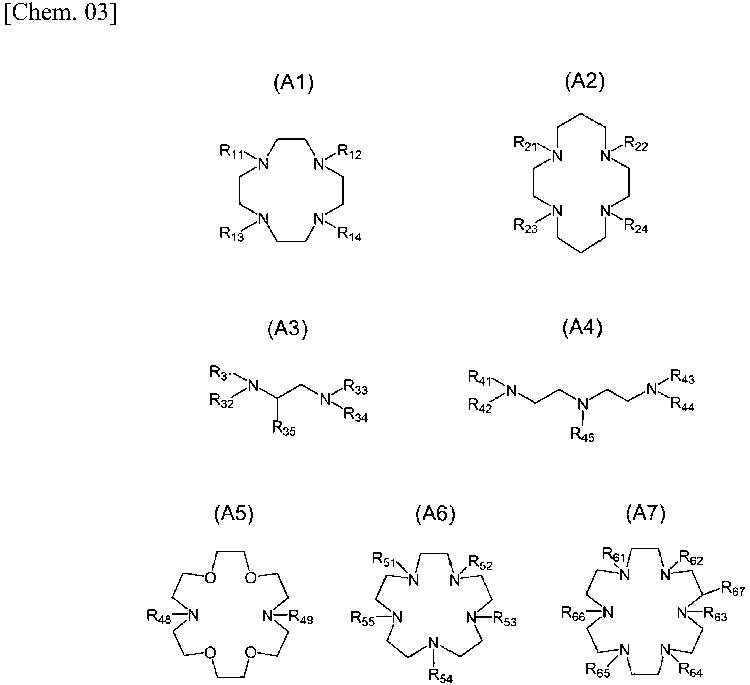

In Formula (A1), R₁₁, R₁₂, R₁₃, and R₁₄ each independently represent any of groups consisting of -(CH₂)ₚCOOH, -(CH₂)ₚC₅H₄N, -(CH₂)ₚPO₃H₂, -(CH₂)ₚCONH₂, and - (CHCOOH)(CH₂)ₚCOOH, and p represents an integer of 0 to 3.

In Formula (A2), R₂₁, R₂₂, R₂₃, and R₂₄ each independently represent a carboxy group or a carboxyalkyl group having 2 or 3 carbon atoms.

In Formula (A3), R₃₁, R₃₂, R₃₃, and R₃₄ each independently represent an atomic group having a hydrogen atom and 2 or more and 10 or less carbon atoms and optionally containing a nitrogen atom or an oxygen atom, and R₃₅ represents a hydrogen atom, a carboxy group, or a carboxyalkyl group having 2 or 3 carbon atoms.

In Formula (A4), R₄₁, R₄₂, R₄₃, and R₄₄ each independently represent an atomic group having a hydrogen atom and 2 or more and 10 or less carbon atoms and optionally containing a nitrogen atom or an oxygen atom, and R₄₅ represents a hydrogen atom, a carboxy group, or a carboxyalkyl group having 2 or 3 carbon atoms.

In Formula (A5), R₄₈ and R₄₉ each independently represent an atomic group having a hydrogen atom and 2 or more and 10 or less carbon atoms and optionally containing a nitrogen atom or an oxygen atom.

In Formula (A6), R₅₁, R₅₂, R₅₃, and R₅₄ each independently represent an atomic group having a hydrogen atom and 2 or more and 10 or less carbon atoms and optionally containing a nitrogen atom or an oxygen atom.

In Formula (A7), R₆₁, R₆₂, R₆₃, R₆₄, R₆₅, and R₆₆ each independently represent an atomic group having a hydrogen atom and 2 or more and 10 or less carbon atoms and optionally containing a nitrogen atom or an oxygen atom, and R₆₇ represents a hydrogen atom, a carboxy group, or a carboxyalkyl group having 2 or 3 carbon atoms.

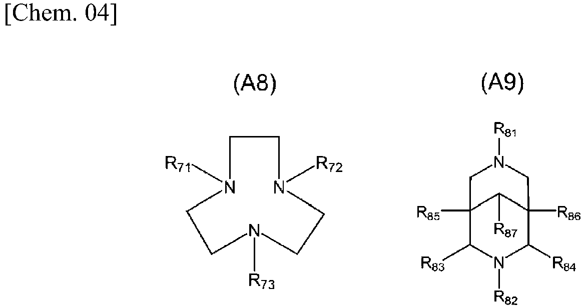

In Formula (A8), R₇₁, R₇₂, and R₇₃ each independently represent an atomic group having a hydrogen atom and 2 or more and 10 or less carbon atoms and optionally containing a nitrogen atom or an oxygen atom.

In Formula (A9), R₈₁ and R₈₂ each independently represent an alkyl group having 1 to 5 carbon atoms, the terminal of the alkyl group may be substituted with a pyridyl group substituted with 1 or more carboxy groups, R₈₇ represents an oxygen atom (= O) of a hydroxyl group or a carbonyl group, R₈₃ and R₈₄ represent a substituted or unsubstituted pyridyl group, R₈₅ and R₈₆ each independently represent -COO-Rₐ, and Rₐ represents an alkyl group having 1 to 5 carbon atoms.

Examples of the specific structure represented by Formula (A1) include structures represented by the following Formulae (A1-1) to (A1-7).

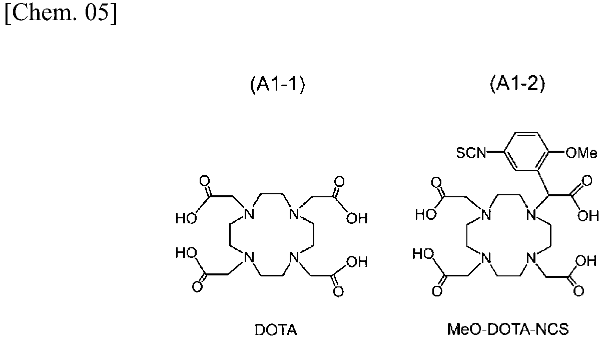

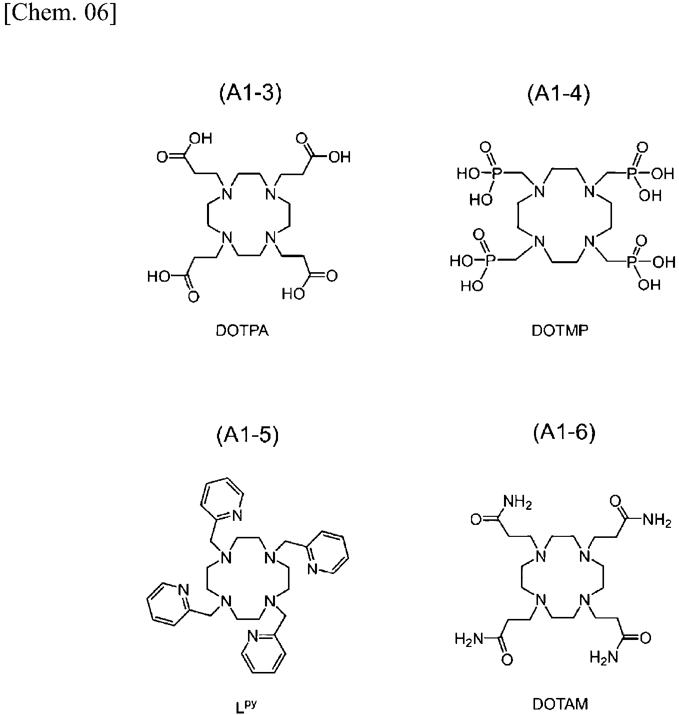

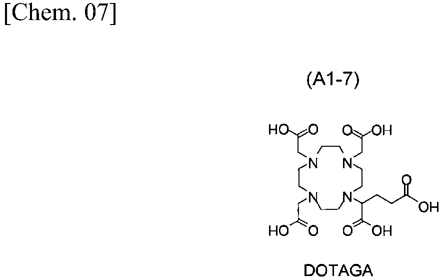

Examples of the specific structure represented by Formula (A2) include structures represented by the following Formulae (A2-1) and (A2-2).

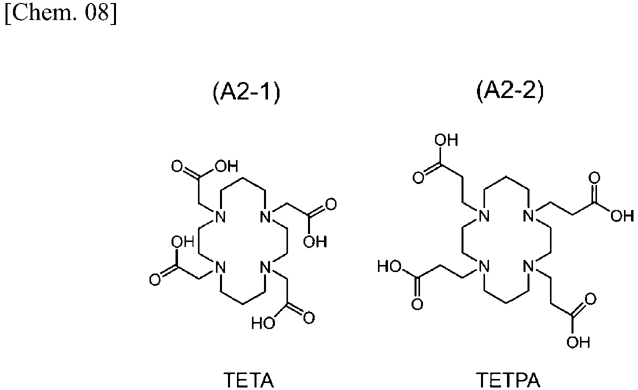

Examples of the specific structure represented by Formula (A3) include structures represented by the following Formulae (A3-1) to (A3-7).

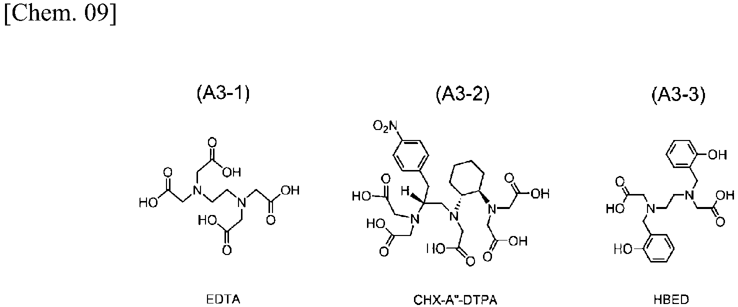

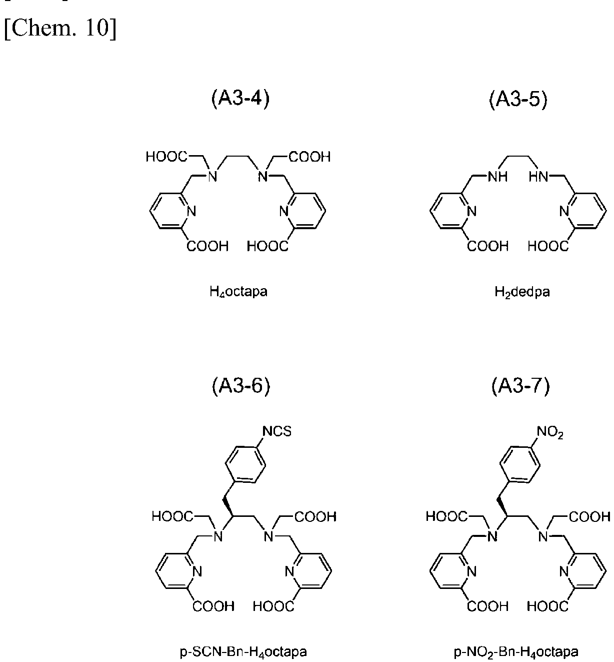

Examples of the specific structure represented by Formula (A4) include structures represented by the following Formulae (A4-1) and (A4-2).

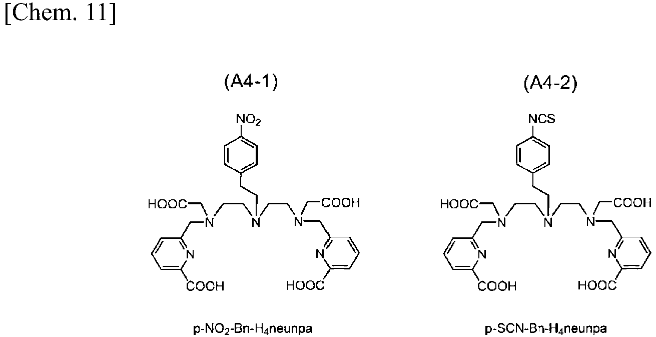

Examples of the specific structure represented by Formula (A5) include structures represented by the following Formulae (A5-1) to (A5-3).

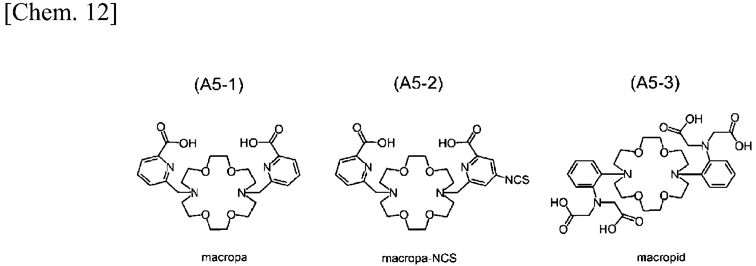

Examples of the specific structure represented by Formula (A6) include a structure represented by the following Formula (A6-1).

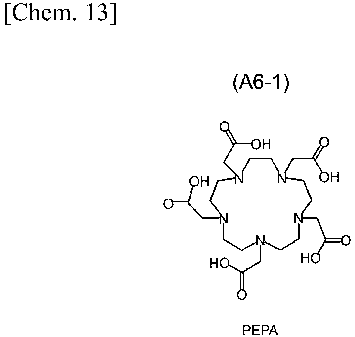

Examples of the specific structure represented by Formula (A7) include structures represented by the following Formulae (A7-1) and (A7-2).

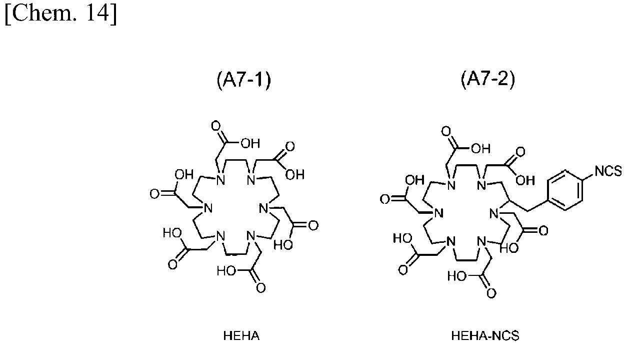

Examples of the specific structure represented by Formula (A8) include structures represented by the following Formulae (A8-1) to (A8-3).

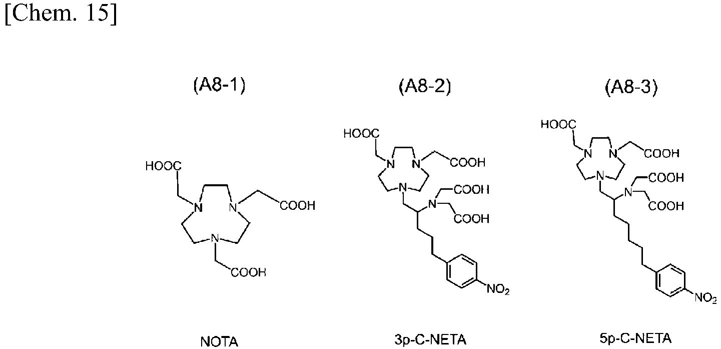

Examples of the specific structure represented by Formula (A9) include structures represented by the following Formulae (A9-1) to (A9-4).

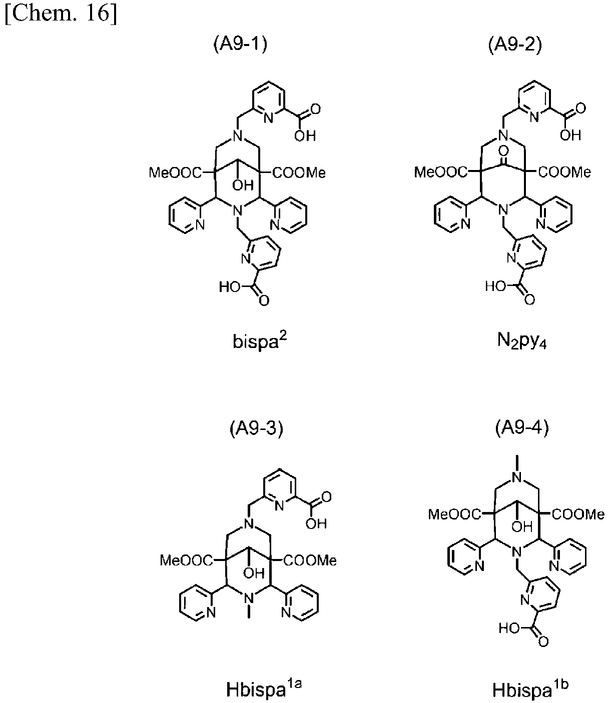

The chelating moiety is preferably DOTA or a derivative thereof, and in the Formulae (I) and (II), the chelating moiety represented by the symbol A preferably has a structure derived from DOTA or a derivative thereof (Al-2 to A1-7) or a compound represented by any one of the following Formula (A) from the viewpoint of sufficiently enhancing the affinity between the compound of the present invention and a radioactive metal ion.

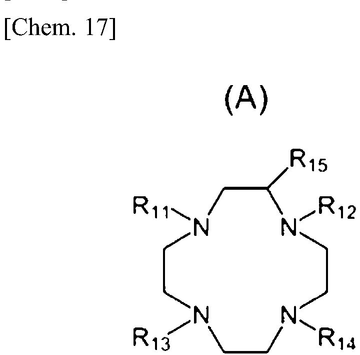

In Formula (A), R₁₁ and R₁₄ each independently represent a group consisting of - (CH₂)ₚCOOH, -(CH₂)ₚC₅H₄N, -(CH₂)ₚPO₃H₂, -(CH₂)ₚCONH₂, and - (CHCOOH)(CH₂)ₚCOOH.

When the compound of the present invention is represented by the Formula (I), for example, R₁₂ may be a substituent for binding to an antibody binding unit, R₁₃ may be a substituent for binding to a functional unit (B), R₁₅ may be a hydrogen atom, and p may be an integer of 0 to 3. Preferably, an amino acid may be introduced such that a carboxy group remains in R₁₂ and R₁₃, and it is more preferable that lysine be introduced into R₁₂ and glutamine be introduced into R₁₃.

When the compound of the present invention is represented by Formula (II), one of R₁₂ and R₁₅ is a hydrogen atom, a carboxy group, or a carboxyalkyl group having 2 or 3 carbon atoms, the other is a substituent for binding to the functional unit (B), p is an integer of 0 to 3, R₁₅ is a hydrogen atom when R₁₂ is a substituent for binding to the functional unit (B), and R₁₅ is a substituent for binding to the functional unit (B) when R₁₂ is not a substituent for binding to the functional unit (B).

As the radioactive metal coordinated in an ionic state to the chelating moiety of the radioactive metal-labeled antibody of the present invention or the compound of the present invention, a metal nuclide that emits radiation of α-rays, β-rays, γ-rays, or a combination thereof can be used. Examples of the nuclide of such a radioactive metal include a radioisotope of an alkali metal, an alkaline earth metal, a lanthanoid, an actinoid, a transition metal, or a metal other than these metals.

Among them, it is preferable to use ⁴⁴Sc, ⁵¹Cr, ⁵⁷Co, ⁵⁸Co, ⁶⁰Co, ⁵⁹Fe, ⁶⁷Ga, ⁶⁸Ga, ⁶⁴Cu, ⁶⁷Cu, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁷Th, or ²²⁵Ac as a radioactive metal nuclide from the viewpoint of being commercially available and enhancing the complex-forming property.

When the radioactive metal-labeled antibody of the present invention is used for the purpose of treating a disease, it is preferable to use an α-ray-emitting nuclide or a β⁻-ray-emitting nuclide as the radioactive metal from the viewpoint of enhancing the therapeutic effect. The α-ray-emitting nuclide may be any nuclide that emits α-rays in the decay process of the radioactive metal, and specifically, ²¹²Bi, ²¹³Bi, ²²⁷Th, ²²⁵Ac or the like is preferably used, ²²⁷Th or ²²⁵Ac is more preferably used, and ²²⁵Ac is still more preferably used.

The β⁻-ray-emitting nuclide may be any nuclide that emits a β⁻-ray in the decay process of the radioactive metal, and specifically, ⁶⁰Co, ⁵⁹Fe, ⁶⁴Cu, ⁶⁷Cu, ⁸⁹Sr, ⁹⁰Y, ^{99m}Tc, ¹⁰³Ru, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰³Hg, ²¹²Bi, ²¹³Bi, ²¹²Pb, or the like is preferably used, and ⁶⁴Cu, ⁶⁷Cu, ⁸⁹Sr, ⁹⁰Y, or ¹⁷⁷Lu is more preferably used.

In addition, when the radiolabeled compound is used for the purpose of diagnosis of a disease or detection of a lesion, it is preferable to use a β⁺-ray-emitting nuclide, an electron-capturing decay nuclide, or a γ-ray-emitting nuclide as the radioactive metal from the viewpoint of enhancing diagnostic performance. The β⁺-ray-emitting nuclide may be a nuclide that emits positrons in the decay process of the radioactive metal, and ⁴⁴Sc, ⁵⁸Co, ⁶⁸Ga, ⁶⁴Cu, ⁸⁹Zr, or the like is preferably used, and ⁶⁴Cu or ⁸⁹Zr is more preferably used.

The electron-capturing decay nuclide may be any nuclide that emits Auger electrons or characteristic X-rays in the decay process of a radioactive metal, and ⁵¹Cr, ⁵⁷Co, ⁵⁸Co, ⁶⁷Ga, ⁶⁸Ga, ⁶⁴Cu, ⁸⁹Zr, ¹¹¹In, ¹⁸⁶Re, ²⁰¹Tl, ¹⁹⁷Hg or the like is preferably used.

The γ-ray-emitting nuclide may be any nuclide that emits γ-rays by γ decay, and ^{99m}Tc, ⁶⁸Ga, or ²⁰¹Tl is preferably used as the nuclide that emits γ-rays by y decay.

When the radioactive metal coordinated to the radioactive metal complex in an ionic state is selected based on the ionic radius, examples of the radioactive metal having an ionic radius of approximately 70 to 130 pm include ⁶⁷Ga, ⁶⁸Ga, ⁶⁴Cu, ⁶⁷Cu, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, and ²²⁵Ac, and these preferably can form a complex of the compound of the present invention having a chelating moiety having the structure represented by the above Formula (A) and a radioactive metal ion.

The radioactive metal coordinated to the chelating moiety of the radioactive metal-labeled antibody of the present invention or the compound of the present invention is preferably one or more selected from Al¹⁸F, ⁶⁴Cu, ⁶⁷Cu, ⁶⁸Ga, ⁸⁹Zr, ⁹⁰Y, ^{99m}Tc, ¹¹¹In, ¹⁷⁷Lu, and ²²⁵Ac.

In Formulae (I) and (II), the functional unit represented by the symbol B is an atomic group having a function of enhancing the intracellular retention of a radioactive metal when the compound of the present invention is used as a radioactive metal-labeled antibody, and is preferably an atomic group containing carbon and hydrogen and further containing nitrogen or oxygen. Examples of the functional unit suitably used in the present invention include a structure having a positive charge under acidic conditions and a structure including a unit for increasing the mass of a radioactive metal-labeled antibody.

When the functional unit has a structure that carries a positive charge under acidic conditions, the structure generates a positive charge within the lysosome inside the cell, making it less likely to be excreted outside the cell. Therefore, it is considered that the radioactive metal coordinated to the functional chelate linker is likely to be retained within the cell. Examples of such an atomic group include a structure having one or a plurality of primary, secondary, or tertiary amino groups, and preferably include an atomic group containing a structure in which ethyleneimine is polymerized (hereinafter also referred to as a "PEI structure").

In addition, it is considered that when the functional unit includes a unit for increasing the mass of the radioactive metal-labeled antibody, the metabolite derived from the radioactive metal-labeled antibody of the present invention is less likely to be excreted outside the cell. As such an atomic group, for example, an atomic group having an atomic weight of 400 or more may be designed, and an atomic group having an atomic weight of 400 or more and 10,000 or less may be more preferable. As a more preferable example, an atomic group containing a structure in which ethylene glycol is polymerized (hereinafter also referred to as a "PEG structure") can be mentioned.

Furthermore, when the functional unit has a structure capable of binding to a molecule present in the target cell, the binding increases the mass of the radioactive metal-labeled antibody, and thus metabolites derived from the radioactive metal-labeled antibody are less likely to be excreted outside the cell. Therefore, as the functional unit, an atomic group having a structure capable of binding to a molecule present in a target cell can be used.

When the functional unit has a PEI structure, the PEI structure is preferably a structure in which ethyleneimine is polymerized with a polymerization degree of 1 to 7.

Specific examples of the structure of the functional unit including the PEI structure include a structure represented by the following Formula (1).

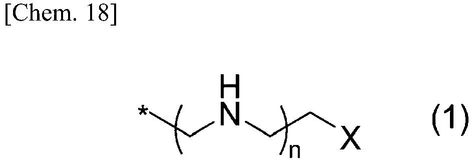

In Formula (1), X represents a hydrogen atom, an alkyl group having 1 to 4 carbon atoms, a hydroxy group, an amino group, or a bond.

n represents the polymerization degree of ethyleneimine.

* (asterisk) represents a bond.

Examples of the alkyl group having 1 to 4 carbon atoms represented by X in Formula (1) include a methyl group, an ethyl group, a propyl group, an isopropyl group, a butyl group, an isobutyl group, a sec-butyl group, and a tert-butyl group.

In Formula (1), n is preferably an integer of 1 to 7, more preferably 1 to 5, and even more preferably 1 to 3.

The functional unit represented by Formula (1) is protonated under acidic conditions such as in a lysosome, and becomes a (poly)cation having a high ionic charge density. Therefore, the radioactive metal coordinated to the functional chelate linker has high intracellular retention.

When the functional unit has a PEG structure, the PEG structure is preferably a structure in which ethylene glycol is polymerized with a polymerization degree of 11 to 48.

Specific examples of the structure of the functional unit containing a PEG structure include a structure represented by the following Formula (2).

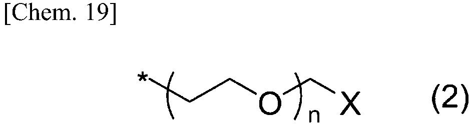

In Formula (2), X represents a hydrogen atom, an alkyl group having 1 to 5 carbon atoms, a hydroxy group, an amino group, a hydroxymethyl group, or a bond.

n represents the polymerization degree of ethylene glycol.

* (asterisk) represents a bond.

Examples of the alkyl group having 1 to 5 carbon atoms represented by X in Formula (2) include a methyl group, an ethyl group, a propyl group, an isopropyl group, a butyl group, a pentyl group, an isobutyl group, a sec-butyl group, a tert-butyl group, an isopentyl group, a sec-pentyl group, a tert-pentyl group, and a neopentyl group.

When the compound having the functional unit represented by Formula (2) is used as a radioactive metal-labeled antibody, the radioactive metal coordinated to the functional chelate linker has high intracellular retention due to the large mass of the functional unit.

From this viewpoint, in Formula (2), n is preferably an integer of 11 to 48, more preferably 12 to 48, and still more preferably 24 to 48.

Next, another embodiment of the functional unit will be described. In the present embodiment, the functional unit is an atomic group having a structure capable of binding to a molecule present in a target cell. For example, when the target cell is a cancer cell, it is preferable to select a protein overexpressed in the cancer cell as the "molecule present in the target cell". Examples of such a protein include cathepsin B. Therefore, the functional unit of the present embodiment preferably has a structure capable of binding to cathepsin B.

There is no limitation on the binding mode between the functional unit and the molecule present in the target cell, and it may be a non-covalent bond and a covalent bond such as an ionic bond, a hydrogen bond, a dipolar interaction, and a dispersing force. Among them, from the viewpoint of enhancing the affinity for the molecule present in the target cell and the binding specificity, it is preferable that the binding between the functional unit and the molecule present in the target cell include a covalent bond. The number of covalent bonds formed between the functional unit and the molecule present in the target cell may be one or more. The functional unit and the molecule present in the target cell may have one or more non-covalent bonds in addition to a covalent bond.

The binding site with the functional unit in the molecule present in the target cell is not particularly limited, but when the molecule present in the target cell is an enzyme, the functional unit preferably binds to the binding site between the enzyme and its substrate, that is, the active site of the enzyme. This is because the structure of the functional unit having excellent affinity for the enzyme and binding specificity can be easily designed by mimicking the structure of the substrate of the enzyme.

In summary, when an atomic group capable of binding to a molecule present in a target cell is used as a functional unit, the functional unit preferably has a structure capable of forming a covalent bond with cathepsin B, and more preferably has a structure capable of forming a covalent bond with an active site of cathepsin B (hereinafter also referred to as "structure A").

For example, when a thiol group present in the active site of cathepsin B and a functional unit form a covalent bond, the structure A preferably has an epoxy group.

Furthermore, considering affinity with cathepsin B and stability in a bound state with cathepsin B, structure A is preferably an epoxysuccinyl peptide. The epoxysuccinyl peptide refers to a peptide having a structure in which trans-epoxysuccinic acid is dehydration-condensed with two same or different amines.

Preferable specific examples of the structure A include structures represented by the following Formulae (X-1) to (X-3).
*-HN-(CH₂)ᵣ-HN-Gly-Aa-Bb-X-Cc-Dd (X-1)
*-HN-(CH₂)ᵣ-HN-Gly-Aa-Bb-X-Cc (X-2)
*-HN-(CH₂)ᵣ-HN-Gly-Aa-Bb-X (X-3)

In Formulae (X-1) to (X-3), X represents an epoxide derivative.

r represents an integer of 1 to 30, and preferably represents 6.

Aa represents one selected from the group consisting of Gly, Phe, Asp, Glu, and 1-aminoadipic acid, and preferably represents Gly.

Bb represents an aliphatic or aromatic hydrophobic amino acid.

Cc represents an aliphatic or aromatic hydrophobic amino acid.

Dd represents an aliphatic or aromatic hydrophobic amino acid, or an aliphatic or aromatic amine.

X represents an epoxide derivative.

* represents a bond.

Gly, Aa, Bb, X, Cc, and Dd are bound to each other via an amide bond.

In Formulae (X-1) to (X-3), Bb preferably represents one selected from the group consisting of Leu, Ile, Phe, Tyr, Val, 3-(2-naphthyl)alanine, 3-cyclohexylalanine, 3-(4-bromophenyl)alanine, and amino acids represented by the following Formulae (X-4) to (X-7), and more preferably represents Leu.

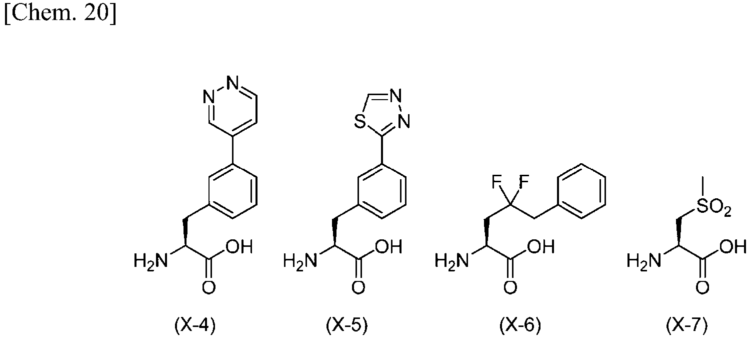

In Formulae (X-1) and (X-2), Cc preferably represents one selected from the group consisting of Leu, Ile, Phe, Tyr, Val, Trp, and Nle, and more preferably represents Leu.

In Formula (X-1), Dd preferably represents one selected from the group consisting of Trp, Val, Ile, Phe, Tyr, Ala, Ser, Thr, Pro, -NH-(CH₂)ₙ-CH₃ (n represents an integer of 0 to 4), -NH-CH(CH₃)₂, -NH-CH₂-CH(CH₃)₂, and -NH-(CH₂)ₘPh (m represents 1 or 2), and more preferably represents Pro.

In Formulae (X-1) to (X-3), the epoxide derivative represented by X is preferably a dicarboxylic acid having an epoxy group or an amino acid having an epoxy group, and more preferably trans-epoxysuccinic acid.

The functional unit may have only one or two or more of the above-described PEI structure, PEG structure, and/or structure capable of binding to a molecule present in the target cell. For example, the functional unit may have one PEI structure and one PEG structure.

The radioactive metal-labeled antibody of the present invention may have only one functional unit or two or more functional units.

When the functional unit has a PEI structure and/or a PEG structure, the functional unit preferably has a structure represented by the following Formula (c).

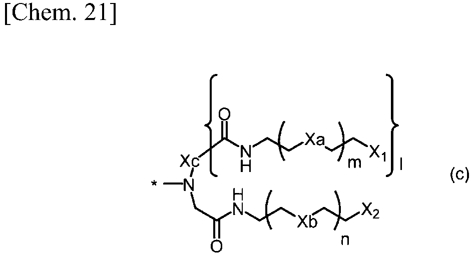

In Formula (c), X₁ and X₂ each independently represent a hydrogen atom, an alkyl group having 1 to 4 carbon atoms, a hydroxy group, an amino group, or a bond.

Xₐ and X_{b} each independently represent O or NH.

l represents 0 or 1.

Xc represents a hydrogen atom when l is 0, and represents CH₂ when l is 1.

m and n each independently represent the polymerization degree of ethyleneimine or ethylene glycol.

* represents a bond.

The compound of the present invention (functional chelate linker) preferably contains a structure represented by the following Formula (d). The structure represented by the following Formula (d) is a structure including a chelating moiety and one or a plurality of functional units.

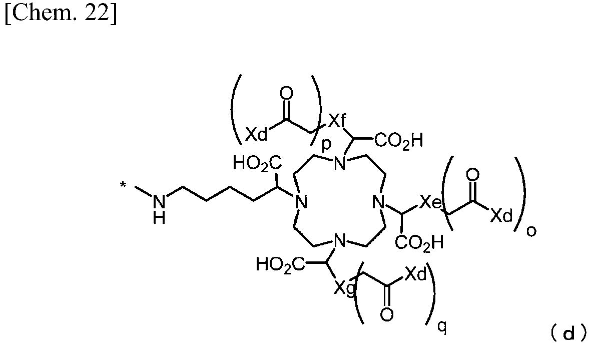

In Formula (d), o, p, and q each independently represent 0 or 1, and satisfy 1 ≤ o + p + q ≤ 3.

Xd represents the structure represented by the Formula (c).

Xe represents a hydrogen atom when o is 0, and represents CH₂ when o is 1.

Xf represents a hydrogen atom when p is 0, and represents CH₂ when p is 1.

Xg represents a hydrogen atom when q is 0, and represents CH₂ when q is 1.

* (asterisk) represents a bond.

In the compound of the present invention, the structure represented by Formula (d) is directly or indirectly bound to the antibody binding unit via a bond (*).

In addition, in the radioactive metal-labeled antibody containing the compound of the present invention and an antibody, the structure represented by the Formula (d) is directly or indirectly bound to the antibody via a bond (*). The term "indirectly bound" as used herein refers to the presence of a linker between the bond and the antibody.

In Formulae (I) and (II), the antibody binding unit represented by the symbol C is an antibody binding unit including a binding site with an antibody. The binding mode between the antibody binding unit and the antibody is not particularly limited, and may be, for example, the following binding mode.
(a) Binding between the antibody and the antibody binding unit via a conjugate addition reaction
(b) Binding between the antibody and the antibody binding unit via a click reaction
(c) Binding between the antibody and the antibody binding unit via amine coupling Hereinafter, these will be described in order.

### <Binding via Conjugate Addition Reaction>

The antibody and the compound of the present invention can be bound by a conjugate addition reaction between a thiol group derived from a side chain of an amino acid constituting the antibody and an antibody binding unit. For this purpose, it is preferable that the antibody binding unit have a Michael acceptor as the binding site with the antibody. As such a Michael acceptor, it is particularly preferable to use a maleimide group from the viewpoint of a balance between stability and reactivity to a conjugate addition reaction, and from the viewpoint of being able to be inexpensively introduced into the antibody binding unit. In other words, it is particularly preferable that the binding site with the antibody be a maleimide group.

Instead of the thiol group derived from the side chain of the amino acid constituting the antibody, a thiol group may be introduced onto the surface of the antibody by appropriate chemical modification, and then the antibody binding unit and the antibody may be bound by a conjugate addition reaction between the thiol group and a Michael acceptor. Examples of the method for introducing a thiol group into an antibody include a method in which an antibody is treated with 2-iminothiolane hydrochloride.

As the antibody binding unit having a maleimide group, for example, one selected from atomic groups represented by the following Formulae (3) to (8) can be used.

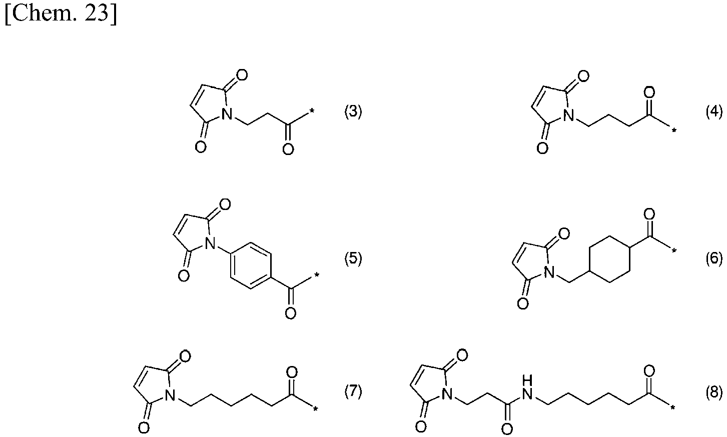

The atomic group represented by any one of the Formulae (3) to (8) can be easily introduced into the compound of the present invention using commercially available reagents BMPS, GMBS, MBS, SMCC, EMCS, or SMPH, and thus can be particularly preferably used as an antibody binding unit.

### <Binding via Click Reaction>

When the antibody and the antibody binding unit are bound via a click reaction, it is preferable to use an antibody binding unit containing an atomic group capable of click reaction as a binding site with the antibody. By reacting such an antibody binding unit with an antibody into which a different atomic group capable of click reaction has been introduced (hereinafter also referred to as a "modified antibody"), the antibody and the antibody binding unit can be bound.

In the present invention, as the combination of atomic groups capable of click reaction, an appropriate combination is selected according to the type of click reaction, and examples thereof include a combination of alkyne and azide and a combination of 1,2,4,5-tetrazine and alkene. In these atomic groups, the antibody binding unit may have one of the atomic groups, and the antibody may have an atomic group that forms a combination with the antibody binding units. Specific examples of click reactions using such combinations of atomic groups include the Huisgen cycloaddition reaction and the inverse electron-demand Diels-Alder reaction.

From the viewpoint of simplicity of the click reaction step, the atomic group capable of click reaction is preferably an atomic group that can be used for the metal catalyst-free click reaction.

Specific examples of the combination of the antibody binding unit and the modified antibody include a combination of an atomic group (Formula (11a)) containing dibenzocyclooctyne (DBCO) as the alkyne and an atomic group (Formula (12a)) containing an azide group as the azide as shown in the following formula, or a combination of an atomic group (Formula (11b)) containing 1,2,4,5-tetrazine and an atomic group (Formula (12b)) containing trans-cyclooctene (TCO) as the alkene.

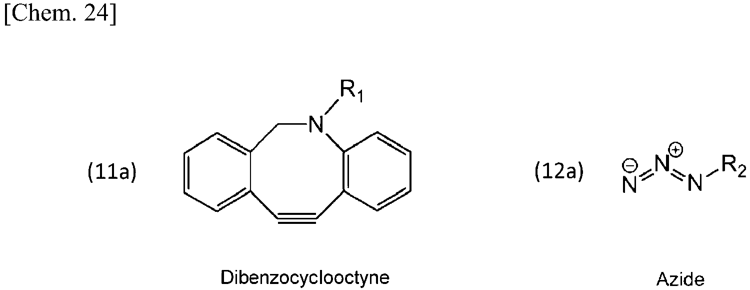

In Formula (11a), R₁ represents a moiety obtained by removing an atomic group capable of click reaction from the antibody binding unit or the modified antibody.

In Formula (12a), R₂ represents a moiety obtained by removing an atomic group capable of click reaction from the antibody binding unit or the modified antibody.

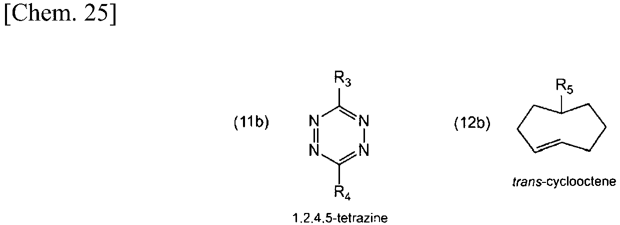

In Formula (11b), one of R₃ and R₄ represents a moiety obtained by removing an atomic group capable of click reaction from the antibody binding unit or the modified antibody, and the other represents a hydrogen atom, a methyl group, a phenyl group, or a pyridyl group.

In Formula (12b), R₅ represents a moiety obtained by removing an atomic group capable of click reaction from the antibody binding unit or the modified antibody.

When an atomic group capable of click reaction is introduced into the antibody binding unit, various commercially available reagents can be used for introduction. Specifically, when an atomic group containing dibenzocyclooctyne (DBCO) is introduced as an atomic group capable of click reaction, for example, DBCO reagents such as DBCO-C6-Acid, DBCO-Amine, DBCO-Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, and DBCO-mPEG can be used. In addition, as a modified antibody corresponding thereto, a modified antibody into which an azide group is introduced may be prepared. Examples of the method for introducing an azide group into an antibody include a method for introducing an azide group into an N-terminal of an amino acid sequence or an amino side chain of a lysine residue.

### <Binding via Amine Coupling>

When the antibody binding unit and the antibody are bound via amine coupling, it is preferable to use an antibody binding unit having a functional group capable of reacting with a primary amine as a binding site with the antibody. By using such an antibody binding unit, an amino group present at the N-terminal of the peptide chain constituting the antibody, the side chain of a lysine residue in the peptide chain, or the like reacts with a binding site of the antibody to form a bond.

Examples of the binding site with an antibody having a functional group capable of reacting with a primary amine include an atomic group having a structure such as isothiocyanate, isocyanate, acyl azide, N-hydroxysuccinimide (NHS) ester, sulfonyl chloride, aldehyde, glyoxal, epoxide, carbonate, aryl halide, imido ester, carbodiimide, anhydride, and fluoroester.

The linker structure represented by Lₐ or L_{b} in Formulae (I) and (II) may have a structure represented by the following Formula (P). The structure is a structure derived from ethylene glycol, and in Formula (P), k is preferably an integer of 2 to 10, more preferably an integer of 2 to 8, and still more preferably an integer of 2 to 5.

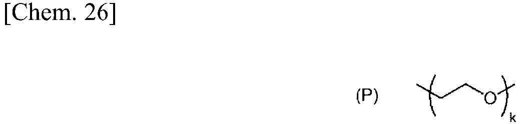

The linker structure represented by Lₐ or L_{b} may be composed of one type of linker structure, or may have a structure in which one type of linker structure is repeated, or a plurality of types of linker structures are combined, and these are bound in a linear or branched manner.

By reacting the compound of the present invention (functional chelate linker) with a radioactive metal ion and an antibody, a radioactive metal-labeled antibody in which the functional chelate linker is conjugated to the antibody can be obtained. The compound of the present invention may be first reacted with a radioactive metal ion and then reacted with an antibody, or may be first reacted with an antibody and then reacted with a radioactive metal ion. In addition, the compound of the present invention, a radioactive metal ion, and an antibody may be reacted simultaneously.

As the radioactive metal ion, one type of the radioactive metals listed above can be preferably used.

The antibody used in the present invention is a polyclonal antibody or a monoclonal antibody. As the immunoglobulin class of the antibody, any of the IgG, IgA, IgM, IgD, and IgE classes can be used without particular limitation, but among these classes, IgG is a main class in the secondary response of the immune response, and is preferably used because IgG is most present in the blood and has high recognition specificity for an antigen. Furthermore, mammalian IgG is preferable, and specific examples of the mammal include humans and primates such as chimpanzees; experimental animals such as rats, mice, and rabbits; livestock animals such as pigs, cows, horses, sheep, and goats; and companion animals such as dogs and cats, and a chimeric antibody of a monoclonal antibody, a humanized antibody, or a human antibody is further preferable. In addition, subclasses of these antibodies are not particularly limited, and for example, when human IgG is used, the antibody may be at least one of IgG1, IgG2, IgG3, and IgG4. The antibody is a full-length antibody or an antibody fragment (examples: F(ab')₂, Fab', Fab, Fv, single chain antibody), but a full-length antibody is preferable.

An antibody is an antibody against any antigen. Examples of such an antigen include proteins, glycans, nucleic acids, and low molecular weight compounds, but an antibody having a protein as an antigen may be preferable. Examples of the protein include a cell membrane receptor, a cell membrane protein other than the cell membrane receptor, a ligand, and a soluble receptor, and more specifically, may be a disease target protein.

For example, an antibody that recognizes an antigen specifically expressed in a tumor cell can be used. Specifically, for example, it is preferable to use antibodies against antigens such as PD-L1, GD2, PDGFRα (platelet-derived growth factor receptor), CD3, CD16A, CD19, CD20, CD22, CD25, CD27, CD30, CD32B, CD33, CD37, CD38, CD39, CD40, CD70, CD73, CD74, CD79b, CD100 (SEMA4D), CD105 (endoglin), CD123, CD137 (4-1BB), CD138, CD157 (Bst1), CD166, CD200, HER2, HER3, phosphatidylserine (PS), EpCAM, fibronectin, PD-1, VEGF, VEGFR-2, VEGF-A, HGF, gpNMB, DEC-205, folate receptor, sialylated HEG1, HEG1, Trop2, CEACAM5, S1P, IGF-1R, DLL4, TNT-1/B, CPAAs, PSMA, ICAM-1, MUC1, MUC5AC, EGFR, EGFRvIII, KIR2DL1, KIR3DL2, NKG2A, tenascin-C, IGF (insulin-like growth factor), CTLA-4, mesothelin, c-Met, Ang2, ENPD3, folate receptor α, TEM-1, GM2, glypican-3, macrophage inhibitory factor, Notch1, Notch2, Notch3, TLR-2, CSF-1R, FGFR2b, HLA-DR, GM-CSF, EphA3, B7-H3, gpA33, Frizzled7 receptor, DLL4, RSPO, LIV-1, SLITRK6, Nectin-4, MET, Tissue Factor, IL-8, P-cadherin, CEA, GITR, TAM (tumor-associated macrophage), DLL4, Ang2, FGFR2, FGFR3, FGFR4, CXCR4, LAG-3, GITR, Fucosyl-GM1, IGF-1, angiopoietin 2, CSF-1R, OX40, BCMA, ErbB3, PTK7, EFNA4, FAP, DR5, CEA, Ly6E, CA6, CAIX, CA72-4 (TAG-72)CEACAM5, LAMP1, tissue factor, EPHA2, DR5, B7-H3, α2-PI, A33, GDF15, ROR1, GITR, BCMA, TBA, LAG-3, EphA2, TIM-3, PIGF, Axl, MICA/B, Thomsen-Friedenreich, CLEC12A, Lgr3, transferrin receptor, TGFβ, IL-17, 5T4, RTK, immune suppressor protein, NaPi2b, Lewis blood group B antigen, A34, lysil-oxidase, DLK-1, or α9 integrin.

Examples of antibodies having HER2 as an antigen include trastuzumab and pertuzumab.

In addition, as antibodies using sialylated HEG1 as an antigen, there can be mentioned antibodies that bind to HEG1 protein with glycosylation derived from mesothelioma, antibodies that bind to HEG1 protein on the cell membrane of mesothelioma, and antibodies that bind to peptides expressed in mesothelioma cell lines (for example, peptides having the amino acid sequence SKSPSLVSLPT). More specific examples thereof include the SKM9-2 antibody described in WO2017/141604 and a humanized antibody thereof. As the SKM9-2 antibody, for example, an anti-sialylated HEG1 monoclonal antibody of a mouse species is commercially available from Nichirei Biosciences. Examples of the humanized SKM9-2 antibody include a humanized antibody capable of binding to human HEG1 protein expressed in mesothelioma cells. The humanized SKM9-2 antibody preferably includes an antibody including a heavy chain variable region and a light chain variable region shown in Table 1 described in WO2023/277113 (hereinafter also referred to as "antibody A"). The "Reference number" described below is a Sequence ID No. of WO2023/277113.

**[Table 1]**

| Table 1: Heavy chain variable region and light chain variable region of humanized SKM9-2 antibody (antibody A) | | | |
|---|---|---|---|
| | Amino acid sequence | Sequence ID No. | Reference number |
| Heavy chain CDR1 | GFSFXTYWIT (X is T or R) | 1 | 37 |
| Heavy chain CDR2 | MIHPSDXETRLSQTFKD (X is A or S) | 2 | 43 |
| Heavy chain CDR3 | DFDV | 3 | 51 |
| Light chain CDR1 | RSSQNIVHXXXXTYLG (The 9th X is N or Q; the 10th X is N or T; the 11th X is A, R, or G; and the 12th X is N, Q, or G) | 4 | 62 |
| Light chain CDR2 | FXVSNRFX (The 2nd X is K or R; and the 8th X is S or L) | 5 | 75 |
| Light chain CDR3 | FQGSHXPRT (X is I, N, or H) | 6 | 82 |

Here, the heavy chain CDR1 of the antibody A is preferably an amino acid sequence (Sequence ID No. 7, Reference number: 104) in which X is T (Thr) in Sequence ID No. 1. In addition, the heavy chain CDR2 of the antibody A is preferably an amino acid sequence (Sequence ID No. 8, Reference number 105) in which X is A (Ala) in Sequence ID No. 2.

Here, the light chain CDR1 of the antibody A is preferably such that both the 9th and 12th X in Sequence ID No. 4 are the amino acid sequence of Q (Gln) (Sequence ID No. 9, Reference number 63), and more preferably such that the 10th X in Sequence ID No. 4 is N (Asn) and the 11th X is the amino acid sequence of G (Gly) (Sequence ID No. 10, Reference number 149). In addition, the light chain CDR2 of the antibody A is preferably an amino acid sequence (Sequence ID No. 11, Reference number 150) in which the 2nd X in Sequence ID No. 5 is R (Arg) and the 8th X is S (Ser). In addition, the light chain CDR3 of the antibody A is preferably an amino acid sequence (Sequence ID No. 12, Reference number 151) in which X in Sequence ID No. 6 is I (Ile).

The heavy chain variable region of the antibody A may have framework regions 1 to 4 shown in Table 2.

**[Table 2]**

| Table 2: Framework region of heavy chain variable region of humanized SKM9-2 antibody (antibody A) | | | |
|---|---|---|---|
| Region | Heavy chain amino acid sequence | Sequence ID No. | Reference number |
| 1 | QVQLXQXGAEXXXPGASVKXSCKAS (The 5th X is Q or V; the 7th X is S or P; the 11th X is V or L; the 12th X is V or K; the 13th X is R or K; and the 20th X is L or V) | 13 | 32 |
| 2 | WVXQXPGQGLEWIG (The 3rd X is K or R; and the 5th X is R or A) | 14 | 40 |
| 3 | KATLTXDXSXXTAYMXFSSXXSEDXAVYYCXR (The 6th X is V or A; the 8th X is K or E; the 10th X is S or T; the 11th X is N or S; the 16th X is E or Q; the 20th X is P or L; the 21st X is T or R; the 25th X is S or T; and the 31st X is V or A) | 15 | 46 |
| 4 | WGXGTXVTVSS (The 3rd X is A or G; and the 6th X is T or L) | 16 | 54 |

The light chain variable region of the antibody A may have framework regions 1 to 4 shown in Table 3.

**[Table 3]**

| Table 3: Framework region of light chain variable region of humanized SKM9-2 antibody (antibody A) | | | |
|---|---|---|---|
| Region | Light chain amino acid sequence | Sequence ID No. | Reference number |
| 1 | DVXMTQXPLSLPVXXGXXASISC (The 3rd X is L or V; the 7th X is T or S; the 14th X is S or T; the 15th X is L or P; the 17th X is D, Q, or E; and the 18th X is Q or P) | 17 | 57 |
| 2 | WYLQKPGQSPXLLI (X is K or Q) | 18 | 72 |
| 3 | GVPDRFSGSGSGTDFTLKISRVEAEDXGVYYC (X is L or V) | 19 | 79 |
| 4 | FGXGTRLEIK (X is G or Q) | 20 | 86 |

The antibody A more preferably has an amino acid sequence in which the heavy chain variable region is selected from the group consisting of Sequence ID No. 21 to 28 shown in Table 4, and further preferably has an amino acid sequence of Sequence ID No. 18 in the heavy chain variable region.

**[Table 4]**

| Table 4: Amino acid sequences of heavy chain variable region of humanized SKM9-2 antibody (antibody A) | | | |
|---|---|---|---|
| Clone name | Sequence ID No. | Heavy chain amino acid sequence | Reference number |
| xiH | 21 | QVQLQQPGAELVRPGASVKLSCKASGFSFTTYWITWVKQRPGQ GLEWIGMIHPSDSETRLSQTFKDKATLTVDKSSNTAYMQFSSPTS EDSAVYYCVRDFDVWGAGTTVTVSS | 1 |
| zuH3 | 22 | QVQLVQSGAEVKKPGASVKLSCKASGFSFTTYWITWVRQAPGQ GLEWIGMIHPSDSETRLSQTFKDKATLTADKSTNTAYMELSSLRS EDTAVYYCARDFDVWGQGTLVTVSS | 2 |
| zuH5 | 23 | QVQLVQSGAEVKKPGASVKLSCKASGFSFTTYWITWVRQAPGQ GLEWIGMIHPSDSETRLSQTFKDKATLTADESTNTAYMELSSLRSE DTAVYYCARDFDVWGQGTLVTVSS | 3 |
| zuH3a | 24 | QVQLVQSGAEVKKPGASVKLSCKASGFSFTTYWITWVRQAPGQ GLEWIGMIHPSDAETRLSQTFKDKATLTADKSTNTAYMELSSLRS EDTAVYYCARDFDVWGQGTLVTVSS | 4 |
| zuH3b | 25 | QVQLVQSGAEVKKPGASVKVSCKASGFSFTTYWITWVRQAPGQ GLEWIGMIHPSDAETRLSQTFKDKATLTADKSTNTAYMELSSLRS EDTAVYYCARDFDVWGQGTLVTVSS | 5 |
| zuH3c | 26 | QVQLVQSGAEVKKPGASVKVSCKASGFSFTTYWITWVRQAPGQ GLEWIGMIHPSDAETRLSQTFKDKATLTADKSTSTAYMELSSLRS EDTAVYYCARDFDVWGQGTLVTVSS | 6 |
| zuH3e | 27 | QVQLVQSGAEVKKPGASVKVSCKAS GFHFTTYWITWVRQAPGQGLEWIGMIHPSDAETRLSQTFKDKAT LTADKSTNTAYMELSSLRSEDTAVYYCARDFDVWGQGTLVTVSS | 8 |
| zuH3f | 28 | QVQLVQSGAEVKKPGASVKVSCKASGFSFRTYWITWVRQAPGQ GLEWIGMIHPSDAETRLSQTFKDKATLTADKSTNTAYMELSSLRS EDTAVYYCARDFDVWGQGTLVTVSS | 9 |

The antibody A more preferably has an amino acid sequence in which the light chain variable region is selected from the group consisting of Sequence ID No. 29 to 41 shown in Table 5, and further preferably has an amino acid sequence of Sequence ID No. 30 in the light chain variable region.

**[Table 5]**

| Table 5: Amino acid sequences of light chain variable region of humanized SKM9-2 antibody (antibody A) | | | |
|---|---|---|---|
| Clone name | Sequence ID No. | Light chain amino acid sequence | Reference number |
| xiL | 29 | DVLMTQTPLSLPVSLGDQASISCRSSQNIVHNNGNTYLGWYLQK PGQSPKLLIFKVSNRFSGVPDRFSGSGSGTDFTLKISRVEAEDLG VYYCFQGSHIPRTFGGGTRLEIK | 10 |
| zuL3 | 30 | DVVMTQTPLSLPVTLGQPASISCRSSQNIVHNNGNTYLGWYLQK PGQSPQLLIFKVSNRFSGVPDRFSGSGSGTDFTLKISRVEAEDVG VYYCFQGSHIPRTFGQGTRLEIK | 12 |
| zuL4 | 31 | DVVMTQTPLSLPVTPGEPASISCRSSQNIVHNNGNTYLGWYLQK PGQSPQLLIFKVSNRFSGVPDRFSGSGSGTDFTLKISRVEAEDVG VYYCFQGSHIPRTFGQGTRLEIK | 13 |
| zuL5 | 32 | DVVMTQSPLSLPVTLGQPASISCRSSQNIVHNNGNTYLGWYLQK PGQSPQLLIFKVSNRFSGVPDRFSGSGSGTDFTLKISRVEAEDVG VYYCFQGSHIPRTFGQGTRLEIK | 14 |
| zuL5a | 33 | DVVMTQSPLSLPVTLGQPASISCRSSQNIVHQNGNTYLGWYLQK PGQSPQLLIFKVSNRFSGVPDRFSGSGSGTDFTLKISRVEAEDVG VYYCFQGSHIPRTFGQGTRLEIK | 15 |
| zuL5c | 34 | DVVMTQSPLSLPVTLGQPASISCRSSQNIVHNNGQTYLGWYLQK PGQSPQLLIFKVSNRFSGVPDRFSGSGSGTDFTLKISRVEAEDVG VYYCFQGSHIPRTFGQGTRLEIK | 17 |
| zuL5d | 35 | DVVMTQSPLSLPVTLGQPASISCRSSQNIVHNNGNTYLGWYLQK PGQSPQLLIFRVSNRFSGVPDRFSGSGSGTDFTLKISRVEAEDVGV YYCFQGSHIPRTFGQGTRLEIK | 18 |
| zuL5e | 36 | DVVMTQSPLSLPVTLGQPASISCRSSQNIVHQNAQTYLGWYLQK PGQSPQLLIFKVSNRFSGVPDRFSGSGSGTDFTLKISRVEAEDVG VYYCFQGSHIPRTFGQGTRLEIK | 19 |
| zuL5f | 37 | DVVMTQSPLSLPVTLGQPASISCRSSQNIVHQNAQTYLGWYLQK PGQSPQLLIFRVSNRFSGVPDRFSGSGSGTDFTLKISRVEAEDVGV YYCFQGSHIPRTFGQGTRLEIK | 20 |
| zuL5g | 38 | DVVMTQSPLSLPVTLGQPASISCRSSQNIVHQNGQTYLGWYLQK PGQSPQLLIFRVSNRFSGVPDRFSGSGSGTDFTLKISRVEAEDVGV YYCFQGSHIPRTFGQGTRLEIK | 21 |
| zuL5o | 39 | DVVMTQSPLSLPVTLGQPASISCRSSQNIVHQNGQTYLGWYLQK PGQSPQLLIFRVSNRFSGVPDRFSGSGSGTDFTLKISRVEAEDVGV YYCFQGSHNPRTFGQGTRLEIK | 29 |
| zuL5p | 40 | DVVMTQSPLSLPVTLGQPASISCRSSQNIVHQNGQTYLGWYLQK PGQSPQLLIFRVSNRFSGVPDRFSGSGSGTDFTLKISRVEAEDVGV YYCFQGSHHPRTFGQGTRLEIK | 30 |
| zuL5q | 41 | DVVMTQSPLSLPVTLGQPASISCRSSQNIVHQNGQTYLGWYLQK PGQSPQLLIFRVSNRFLGVPDRFSGSGSGTDFTLKISRVEAEDVG VYYCFQGSHIPRTFGQGTRLEIK | 31 |

The radioactive metal-labeled antibody of the present invention can be used as a radioactive pharmaceutical containing the labeled antibody as an active ingredient. The radioactive pharmaceutical containing the radioactive metal-labeled antibody of the present invention as an active ingredient can be formulated by a known pharmaceutical method. For example, the radioactive pharmaceutical of the present invention may contain a pharmaceutically acceptable excipient. The excipient can be an excipient that can be appropriately administered to give an effective amount of the radioactive metal-labeled antibody of the present invention as an active ingredient to a subject. In an aspect, the radioactive pharmaceutical of the present invention can be an injection, and the injectable excipient can be a sterile aqueous solution, for example, a saline solution or an isotonic solution containing an auxiliary agent such as a pharmaceutically acceptable buffer solution, a stabilizer, or a solubilizer. The radioactive pharmaceutical of the present invention can be administered parenterally (for example, intravenous or intrapleural administration) in the form of an injection, and is used for treatment of a disease, diagnosis of a disease, detection of a lesion, or the like. The radioactive pharmaceutical using an antibody that recognizes an antigen specifically expressed in tumor cells is suitably used for diagnosis of cancer or internal radiation therapy of cancer by selecting a radioactive metal.

In each of the above-described embodiments, examples of the substituent capable of substituting each atomic group and each chemical structure of the compound include a halogen atom, a saturated or unsaturated alkyl group, a hydroxy group, a formyl group, a carboxy group, an acyl group, an amino group, a nitro group, an ester group, an isothiocyanate group, a thioxy group, a cyano group, an amide group, an imide group, a phosphate group, a phenyl group, a benzyl group, a pyridyl group, and a naphthyl group. These substituents may be one alone or a group in which two or more substituents are combined.

Although the present invention has been described above based on the preferable embodiments thereof, the present invention is not limited to the above-described embodiments. For example, in each of the above-described embodiments, a compound having one chelating moiety, one functional unit, and one antibody binding unit has been described, but as long as the present invention is embodied, the compound of the present invention may have a plurality of at least one of the functional unit and the antibody binding unit.

The above embodiments of the present invention include the following technical ideas.
[1] A radioactive metal-labeled antibody comprising an antibody labeled with a radioactive metal, wherein
   the radioactive metal-labeled antibody further comprises a functional chelate linker conjugated to the antibody, and
   the functional chelate linker includes:
      a chelating moiety capable of coordinating to a radioactive metal ion; and
      a functional unit having a function of enhancing intracellular retention.
[2] The radioactive metal-labeled antibody according to [1], in which the functional unit has a structure in which ethyleneimine or ethylene glycol is polymerized.
[3] The radioactive metal-labeled antibody according to [2], in which the functional unit has a structure in which ethyleneimine is polymerized with a polymerization degree of 1 to 7.
[4] The radioactive metal-labeled antibody according to [2], in which the functional unit has a structure in which ethylene glycol is polymerized with a polymerization degree of 11 to 48.
[5] The radioactive metal-labeled antibody according to any one of [1] to [4], in which the chelating moiety is interposed between the antibody and the functional unit.
[6] The radioactive metal-labeled antibody according to [5], in which
   the functional chelate linker further includes an antibody binding unit having a binding site with the antibody,
   the chelating moiety has first and second amino acid residues, and
   the chelating moiety is bound to the antibody binding unit via the first amino acid residue, and the chelating moiety is bound to the functional unit via the second amino acid residue.
[7] The radioactive metal-labeled antibody according to any one of [1] to [6], in which the chelating moiety is DOTA or a derivative thereof.
[8] The radioactive metal-labeled antibody according to any one of [1] to [7], in which the radioactive metal is one selected from Al¹⁸F, ⁶⁴Cu, ⁶⁷Cu, ⁶⁸Ga, ⁸⁹Zr, ⁹⁰Y, ^{99m}Tc, ¹¹¹In, ¹⁷⁷Lu, and ²²⁵Ac.
[9] The radioactive metal-labeled antibody according to any one of [1] to [8], in which the functional chelate linker is introduced into a thiol group of the antibody.
[10] The radioactive metal-labeled antibody according to any one of [1] to [9], in which the antibody is an antibody that recognizes an antigen specifically expressed in a tumor cell.
[11] A radioactive metal-labeled antibody according to any one of [1] to [10], in which
   a chelating moiety capable of being coordinated to the radioactive metal ion has a structure represented by the Formula (A), and
   the functional unit has a structure in which ethyleneimine is polymerized or a structure in which ethylene glycol is polymerized with a polymerization degree of 11 to 48.
[12] The radioactive metal-labeled antibody according to any one of [1] to [11], in which the functional chelate linker has a structure represented by the following Formula (a) or (b). 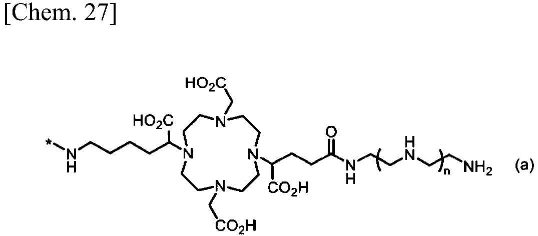 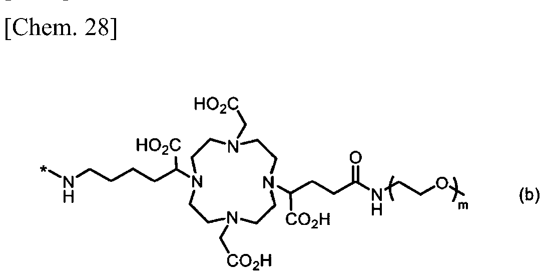 In Formulae (a) and (b), * (asterisk) represents a bond, n represents 1 or more and 7 or less, and m represents 11 or more and 48 or less.
[13] The radioactive metal-labeled antibody according to any one of [1] to [12], in which the functional unit has a structure represented by the Formula (c).
[14] The radioactive metal-labeled antibody according to any one of [1] to [13], in which the functional chelate linker has a structure represented by the Formula (d).
[15] A radioactive pharmaceutical including the radioactive metal-labeled antibody according to any one of [1] to [14] as an active ingredient.
[16] The radioactive pharmaceutical according to [15], which is used for diagnosis of cancer or internal radiation therapy for cancer.
[17] A compound including, in its structure:
   a chelating moiety capable of coordinating to a radioactive metal ion;
   a functional unit having a function of enhancing intracellular retention; and
   an antibody binding unit including a binding site with an antibody.
[18] The compound according to [17], in which the chelating moiety is interposed between the antibody binding unit and the functional unit.
[19] The compound according to [17] or [18], in which the functional unit has a structure in which ethyleneimine is polymerized with a polymerization degree of 1 to 7.
[20] The compound according to [17] or [18], in which the functional unit has a structure in which ethylene glycol is polymerized with a polymerization degree of 11 to 48.
[21] The compound according to any one of [17] to [20], in which the binding site with the antibody is a maleimide group.
[22] A compound according to any one of [17] to [21], in which
   a chelating moiety capable of being coordinated to the radioactive metal ion has a structure represented by the Formula (A), and
   the functional unit has a structure in which ethyleneimine is polymerized or a structure in which ethylene glycol is polymerized with a polymerization degree of 11 to 48.
[23] The compound according to any one of [17] to [22], including a structure represented by the Formula (a) or (b).
[24] The compound according to any one of [17] to [23], in which the functional unit has a structure represented by the Formula (c).
[25] The compound according to any one of [17] to [24], including a structure represented by the Formula (d).
[26] The radioactive metal-labeled antibody according to any one of [1] and [5] to [10], in which the functional unit has a structure capable of forming a covalent bond with a cathepsin B active site.
[27] The radioactive metal-labeled antibody according to [26], in which the structure capable of forming the covalent bond with the cathepsin B active site is an epoxysuccinyl peptide.
[28] The compound according to [17] or [18], in which the functional unit has a structure including an epoxysuccinyl peptide.
[29] The radioactive metal-labeled antibody according to any one of [1] to [14], in which the antibody is trastuzumab or pertuzumab.
[30] The radioactive metal-labeled antibody according to any one of [1] to [14], in which the antibody is an antibody that binds to HEG1 protein having glycosylation obtained from mesothelioma, preferably a humanized antibody thereof, and more preferably the antibody A.
[31] The radioactive metal-labeled antibody according to any one of [1] to [14], [29], and [30], in which the functional chelate linker is represented by the general Formula (I).
[32] The radioactive metal-labeled antibody according to any one of [17] to [25] and [28], in which the functional chelate linker is represented by the general Formula (II).

### Examples

Hereinafter, the present invention will be described in more detail with reference to Examples. However, the scope of the present invention is not limited to such examples.

All reagents used were commercially available reagents, and were used as they were unless otherwise specified.

LCMS-2020 manufactured by Shimadzu Corporation was used for mass spectrometry (MS), and LCMS-IT-TOF manufactured by Shimadzu Corporation was used for high-resolution mass spectrometry (HRMS).

¹H and ¹³C nuclear magnetic resonance (NMR) spectra were recorded using the JNM-ECS400 system or the JNM-ECA500 system from JEOL Ltd., with tetramethylsilane (0 ppm) used as an internal standard. Coupling of peaks is described as s for the singlet, t for the triplet, and m for the multiplet.

Reverse phase (RP) HPLC was performed using a Shimadzu system (LC-20AT pump with SPD-20A UV detector, detection wavelength at 220 or 254 nm) equipped with a COSMOSIL C₁₈ column (5C₁₈-AR-II, 4.6 mm I.D. × 150 mm or 10 mm I.D. × 250 mm) or a COSMOSIL PBr column (3PBr, 4.6 mm I.D. × 150 mm) from Nacalai Tesque, Inc. Size exclusion (SEC)-HPLC was performed using a Shimadzu system (LC-20A pump with SPD-20A UV detector, detection wavelength at 254 nm) equipped with a TSKgel G3000SW_{XL} column (5 µm, 7.8 mm I.D. × 300 mm) from Tosoh Corporation.

The concentration of the antibody was calculated by UV absorption measurement at 280 nm using NanoVue PLUS from Central Scientific Commerce, Inc.

Autoradiographic imaging was measured using the Amersham Typhoon scanner from Global Life Science Technologies Japan Co., Ltd.

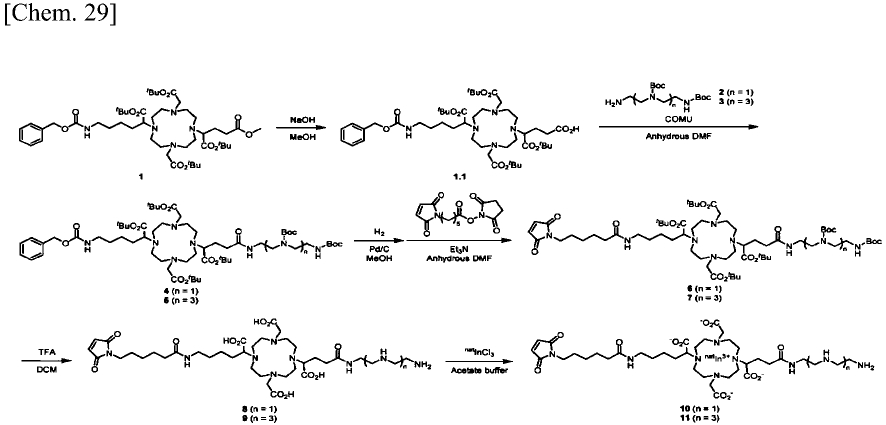

### Synthesis of Compound 8 and In Complex 10 thereof (Example 1-1) and Synthesis of Compound 9 and In Complex 11 thereof (Example 1-2)

### <Synthesis of Compound 1>

Compound 1 was synthesized according to a previous report (Org. Biomol. Chem., 2014, 12, 9389).

### <Synthesis of Compound 1.1>

Compound 1 (613 mg, 0.67 mmol) was dissolved in methanol (12 mL), a 0.2 normal aqueous NaOH solution (80 mg, 2.0 mmol, 10 mL) was added thereto, and then the mixture was stirred at room temperature for 20 hours. A 5% aqueous HCl solution was added to adjust the pH to 3, and then methanol was removed using a rotary evaporator. Ethyl acetate was added to the remaining aqueous solution to recover the organic layer, and the recovered organic layer was dried over sodium sulfate and then filtered. After distilling off the solvent in the filtrate, 376 mg of a residue was obtained as Compound 1.1 without purification (yield: 62%). HRMS (electrospray ionization method) of the obtained Compound 1.1 showed a measured m/z value of 906.5799 (theoretical m/z value: 906.5798 for C₄₇H₈₀N₅O₁₂⁺, [M+H]⁺)).

### <Synthesis of Compounds 2 and 3>

Compounds 2 and 3 were synthesized according to a previous report (J. Am. Chem. Soc., 2008, 130, 14, 4618-4627).

### <Synthesis of Compound 4>

Compound 1.1 (376 mg, 0.41 mmol) was dissolved in anhydrous DMF (1 mL), 1-((1-(cyano-2-ethoxy-2-oxo-ethylideneaminooxy)dimethylaminomorpholino))uronium hexafluorophosphate (COMU) (351 mg, 0.82 mmol) was added thereto at 0°C, and then the mixture was stirred at 0°C for 15 minutes. Then, Compound 2 (249 mg, 0.82 mmol) was added, and the mixture was stirred at room temperature for 13 hours. The solution was purified by the reverse phase HPLC using the COSMOSIL C₁₈ column (5C₁₈-AR-II, 10 mm I.D. × 250 mm) and a mobile phase (elution conditions: 0.1% trifluoroacetic acid aqueous solution/0.1% trifluoroacetic acid acetonitrile solution = 9/1 (0 min) to 1/9 (40 min), flow rate: 4 mL/min) to obtain 272 mg of Compound 4 (yield: 56%). HRMS (electrospray ionization method) of the obtained Compound 4 showed a measured m/z value of 596.3962 (theoretical m/z value: 596.3962 for C₆₁H₁₀₈N₈O₁₅²⁺, [M+2H]²⁺).

### <Synthesis of Compound 5>

Compound 1.1 (392 mg, 0.43 mmol) was dissolved in anhydrous DMF (800 µL), COMU (184 mg, 0.43 mmol) was added thereto at 0°C, and the mixture was stirred at 0°C for 15 minutes. Then, Compound 3 (635 mg, 0.43 mmol) was added, and the mixture was stirred at room temperature for 11 hours. The solution was purified by silica gel chromatography using a mobile phase (chloroform/methanol = 9/1) to obtain 293 mg of Compound 5 (yield: 46%). HRMS (electrospray ionization method) of the obtained Compound 5 showed a measured m/z value of 739.4905 (theoretical m/z value: 739.4908 for C₇₅H₁₃₄N₁₀O₁₉²⁺, [M+2H]²⁺).

### <Synthesis of Compound 6>

Compound 4 (262 mg, 0.22 mmol) was dissolved in methanol (20 mL), 10% palladium on carbon (100 mg) was added thereto, and the mixture was vigorously stirred at room temperature for 13 hours under a hydrogen gas atmosphere. Subsequently, filtration was performed using a Hyflo Super Cel, and the solvent from the filtrate was distilled off. The residue (159 mg, 0.15 mmol) was dissolved in anhydrous DMF (800 µL), 6-maleimidohexanoic acid N-hydroxysuccinimide ester (EMCS) (56 mg, 0.18 mmol) and triethylamine (23 mg, 0.23 mmol) were added thereto, and then the mixture was stirred at room temperature for 12 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 1/9 (40 min), flow rate: 4 mL/min) to obtain 21 mg of Compound 6 (yield: 11%). HRMS (electrospray ionization method) of the obtained Compound 6 showed a measured m/z value of 1250.8219 (theoretical m/z value: 1250.8222 for C₆₃H₁₁₂N₉O₁₆⁺, [M+H]⁺).

### <Synthesis of Compound 7>

Compound 5 (293 mg, 0.20 mmol) was dissolved in methanol (20 mL), 10% palladium on carbon (100 mg) was added thereto, and the mixture was vigorously stirred at room temperature for 16 hours under a hydrogen gas atmosphere. Subsequently, filtration was performed using a Hyflo Super Cel, and the solvent from the filtrate was distilled off. A part of the residue (50 mg, 37 µmol) was dissolved in anhydrous DMF (550 µL), 6-maleimidohexanoic acid N-hydroxysuccinimide ester (46 mg, 0.15 mmol) and triethylamine (15 mg, 0.15 mmol) were added thereto, and then the mixture was stirred at room temperature for 21 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 1/9 (40 min), flow rate: 4 mL/min) to obtain 24 mg of Compound 7 (yield: 42%). HRMS (electrospray ionization method) of the obtained Compound 7 showed a measured m/z value of 1537.0113 (theoretical m/z value: 1537.0115 for C₇₇H₁₃₈N₁₁O₂₀⁺, [M+H]⁺).

### <Synthesis of Maleimide-DOTAGL-PEI2 (MDPEI2) (Compound 8)>

Compound 6 (21 mg, 17 µmol) was dissolved in a mixed solution of trifluoroacetic acid (2 mL) and dichloromethane (1 mL), and the mixture was stirred at room temperature for 12 hours. Thereafter, the solvent was distilled off, and the residue was purified by the reverse phase HPLC using the COSMOSIL C₁₈ column (5C₁₈-AR-II, 4.6 mm I.D. × 150 mm) and a mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min) to obtain 1.9 mg of Compound 8 (yield: 14%). HRMS (electrospray ionization method) of the obtained Compound 8 showed a measured m/z value of 826.4669 (theoretical m/z value: 826.4669 for C₃₇H₆₄N₉O₁₂⁺, [M+H]⁺).

### <Synthesis of Maleimide-DOTAGL-PEI4 (MDPEI4) (Compound 9)>

Compound 7 (12 mg, 8.0 µmol) was dissolved in a mixed solution of trifluoroacetic acid (800 µL) and dichloromethane (200 µL), and the mixture was stirred at room temperature for 14 hours. Thereafter, the solvent was distilled off, and the residue was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (40 min), flow rate: 1 mL/min) to obtain 0.49 mg of Compound 9 (yield: 6.9%). HRMS (electrospray ionization method) of the obtained Compound 9 showed a measured m/z value of 912.5515 (theoretical m/z value: 912.5513 for C₄₁H₇₄N₁₁O₁₂⁺, [M+H]⁺).

### <Synthesis of Maleimide-[^{nat}In]In-DOTAGL-PEI2([^{nat}In]In-MDPEI2) (Compound 10)>

In the present synthesis, indium(III) chloride (^{nat}InCl₃) having a natural abundance of indium (III) (^{nat}In) was used. Compound 8 (1.5 mg, 1.8 µmol) was dissolved in acetate buffer solution (1.0 mol/L, pH 5.1, 200 µL), indium(III) chloride (3.9 mg, 18 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min), and further purified by the reverse phase HPLC using the COSMOSIL PBr column (3PBr, 4.6 mm I.D. × 150 mm) and the mobile phase (elution conditions: 0.01% trifluoroacetic acid aqueous solution/0.01% trifluoroacetic acid acetonitrile solution = 1010 (0 min) to 7/3 (30 min), flow rate: 1 mL/min) to obtain 0.45 mg of Compound 10 (yield: 27%). HRMS (electrospray ionization method) of the obtained Compound 10 showed a measured m/z value of 938.3473 (theoretical m/z value: 938.3473 for C₃₇H₆₁InN₉O₁₂⁺, [M+2H]⁺).

### <Synthesis of Maleimide-[^{nat}In]In-DOTAGL-PEI4([^{nat}In]In-MDPEI4) (Compound 11)>

Compound 9 (0.68 mg, 0.74 µmol) was dissolved in acetate buffer solution (1.0 mol/L, pH 5.1, 120 µL), indium(III) chloride (1.6 mg, 7.4 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and a mobile phase (elution conditions: 0.1% trifluoroacetic acid aqueous solution/0.1% trifluoroacetic acid acetonitrile solution = 9/1 (0 min) to 8/2 (20 min), flow rate: 1 mL/min) to obtain 0.07 mg of Compound 11 (yield: 9.2%). HRMS (electrospray ionization method) of the obtained Compound 11 showed a measured m/z value of 512.7197 (theoretical m/z value: 512.7195 for C₄₁H₇₂InN₁₁O₁₂²⁺, [M+3H]²⁺).

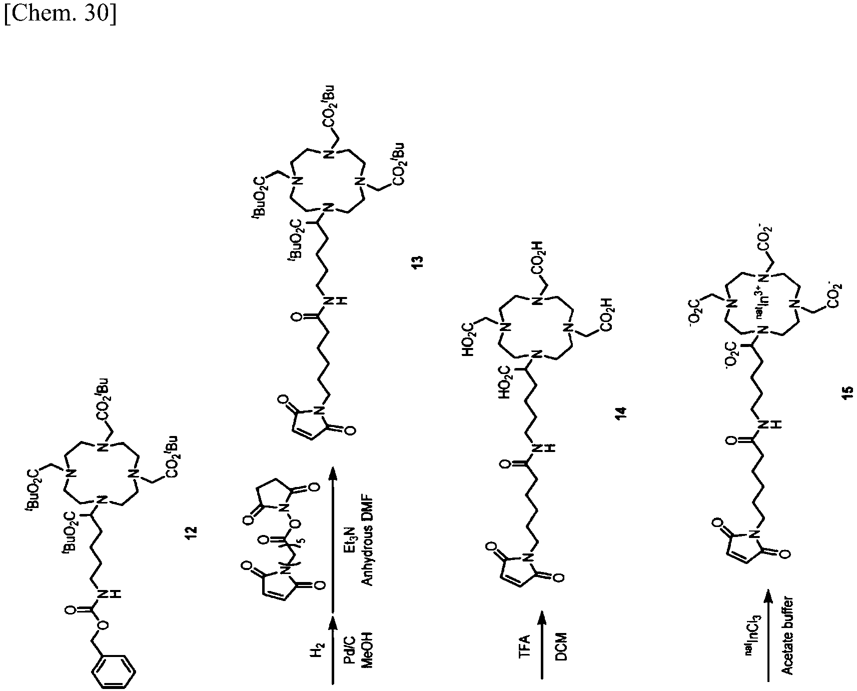

### · Synthesis of Compound 14 and In Complex 15 Thereof (Comparative Example 1-1)

### <Synthesis of Compound 12>

Compound 12 was synthesized according to a previous report (Angew. Chem. Int. Ed. Engl., 2017, 56, 9825-9828).

### <Synthesis of Compound 13>

Compound 12 (784 mg, 0.94 mmol) was dissolved in methanol (15 mL), 10% palladium on carbon (150 mg) was added thereto, and the mixture was vigorously stirred at room temperature for 19 hours under a hydrogen gas atmosphere. Subsequently, filtration was performed using a Hyflo Super Cel, and the solvent from the filtrate was distilled off. A part of the residue (100 mg, 0.14 mmol) was dissolved in anhydrous DMF (400 µL), 6-maleimidohexanoic acid N-hydroxysuccinimide ester (88 mg, 0.28 mmol) and triethylamine (58 mg, 0.57 mmol) were added thereto, and then the mixture was stirred at room temperature for 13 hours. The solution was purified by the reverse phase HPLC using the COSMOSIL C₁₈ column (5C₁₈-AR-II, 10 mm I.D. × 250 mm) and a mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min) to obtain 20 mg of Compound 13 (yield: 15%). HRMS (electrospray ionization method) of the obtained Compound 13 showed a measured m/z value of 893.5959 (theoretical m/z value: 893.5958 for C₄₆H₈₁N₆O₁₁⁺, [M+H]⁺). The identification data of the obtained Compound 13 by NMR is shown below. ¹HNMR(400MHz, CDCl₃)δ6.65(s, 2H), 3.85-2.57(m, 27H), 2.16(t, 2H), 1.57-1.25(m, 48H).

### <Synthesis of Maleimide-DOTAGA (MD) (Compound 14)>

Compound 13 (20 mg, 22 µmol) was dissolved in a mixed solution of trifluoroacetic acid (1.6 mL) and dichloromethane (0.4 mL), and the mixture was stirred at room temperature for 14 hours. Thereafter, the solvent was distilled off, and the residue was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min) to obtain 8.3 mg of Compound 14 (yield: 57%). HRMS (electrospray ionization method) of the obtained Compound 14 showed a measured m/z value of 669.3454 (theoretical m/z value: 669.3454 for C₃₀H₄₉N₆O₁₁⁺, [M+H]⁺).

### <Synthesis of Maleimide-[^{nat}In]In-DOTAGA([^{nat}In]In-MD) (Compound 15)>

Compound 14 (1.0 mg, 1.5 µmol) was dissolved in acetate buffer solution (1.0 mol/L, pH 5.1, 100 µL), indium(III) chloride (3.3 mg, 15 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min) to obtain 0.41 mg of Compound 15 (yield: 35%). HRMS (electrospray ionization method) of the obtained Compound 15 showed a measured m/z value of 781.2258 (theoretical m/z value: 781.2258 for C₃₀H₄₆InN₆O₁₁⁺, [M+2H]⁺).

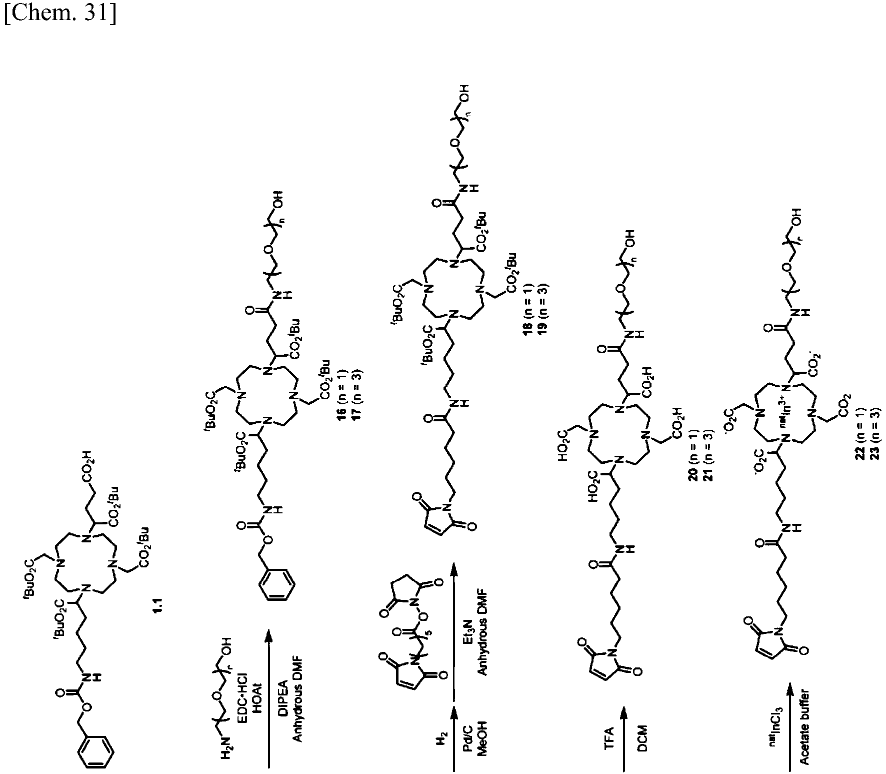

### · Synthesis of Compound 20 and In Complex 22 thereof (Comparative Example 1-2) and Synthesis of Compound 21 and In Complex 23 thereof (Comparative Example 1-3)

### <Synthesis of Compound 16>

Compound 1.1 (376 mg, 0.41 mmol) was dissolved in anhydrous DMF (700 µL), 1-(3-dimethylaminopropyl)-3-ethylcarbodiimide hydrochloride (EDC·HCl) (44 mg, 0.23 mmol), 1-hydroxy-7-azabenzotriazole (HOAt) (57 mg, 0.42 mmol), and N,N-diisopropylethylamine (DIPEA) (54 mg, 0.42 mmol) were added thereto at 0°C, and then the mixture was stirred at 0°C for 15 minutes. Thereafter, 2-(2-aminoethoxy)ethanol (33 mg, 0.31 mmol) was added thereto, and the mixture was stirred at room temperature for 11 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 1/9 (40 min), flow rate: 4 mL/min) to obtain 113 mg of Compound 16 (yield: 54%). HRMS (electrospray ionization method) of the obtained Compound 16 showed a measured m/z value of 993.6483 (theoretical m/z value: 993.6483 for C₅₁H₈₉N₆O₁₃⁺, [M+H]⁺).

### <Synthesis of Compound 17>

Compound 1.1 (76 mg, 84 µmol) was dissolved in anhydrous DMF (600 µL), EDC·HCl (44 mg, 0.23 mmol), HOAt (57 mg, 0.42 mmol), and DIPEA (54 mg, 0.42 mmol) were added thereto at 0°C, and the mixture was stirred at 0°C for 15 minutes. Thereafter, 2-(2-(2-(2-aminoethoxy)ethoxy)ethoxy)ethanol (48 mg, 0.25 mmol) was added thereto, and the mixture was stirred at room temperature for 7 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 1/9 (40 min), flow rate: 4 mL/min) to obtain 61 mg of Compound 17 (yield: 67%). HRMS (electrospray ionization method) of the obtained Compound 17 showed a measured m/z value of 1081.7009 (theoretical m/z value: 1081.7007 for C₅₅H₉₇N₆O₁₅⁺, [M+H]⁺).

### <Synthesis of Compound 18>

Compound 16 (113 mg, 0.11 mmol) was dissolved in methanol (15 mL), 10% palladium on carbon (100 mg) was added thereto, and the mixture was vigorously stirred at room temperature for 20 hours under a hydrogen gas atmosphere. Subsequently, filtration was performed using a Hyflo Super Cel, and the solvent from the filtrate was distilled off. A part of the residue (50 mg, 58 µmol) was dissolved in anhydrous DMF (500 µL), 6-maleimidohexanoic acid N-hydroxysuccinimide ester (18 mg, 58 µmol) and triethylamine (6.5 mg, 64 µmol) were added thereto, and then the mixture was stirred at room temperature for 17 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min) to obtain 24 mg of Compound 18 (yield: 39%). HRMS (electrospray ionization method) of the obtained Compound 18 showed a measured m/z value of 1052.6857 (theoretical m/z value: 1052.6854 for C₅₃H₉₄N₇O₁₄⁺, [M+H]⁺).

### <Synthesis of Compound 19>

Compound 17 (34 mg, 31 µmol) was dissolved in methanol (10 mL), 10% palladium on carbon (11 mg) was added thereto, and the mixture was vigorously stirred at room temperature for 13 hours under a hydrogen gas atmosphere. Subsequently, filtration was performed using a Hyflo Super Cel, and the solvent from the filtrate was distilled off. The residue was dissolved in anhydrous DMF (600 µL), 6-maleimidohexanoic acid N-hydroxysuccinimide ester (38 mg, 0.12 mmol) and triethylamine (13 mg, 0.12 mmol) were added thereto, and then the mixture was stirred at room temperature for 16 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 1/9 (40 min), flow rate: 4 mL/min) to obtain 14 mg of Compound 19 (yield: 40%). HRMS (electrospray ionization method) of the obtained Compound 19 showed a measured m/z value of 570.8724 (theoretical m/z value: 570.8724 for C₅₇H₁₀₃N₇O₁₆²⁺, [M+2H]²⁺).

### <Synthesis of Maleimide-DOTAGL-PEG2 (MDPEG2) (Compound 20)>

Compound 18 (24 mg, 23 µmol) was dissolved in a mixed solution of trifluoroacetic acid (1.6 mL) and dichloromethane (0.4 mL), and the mixture was stirred at room temperature for 14 hours. Thereafter, the solvent was distilled off, and the residue was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min) to obtain 9.4 mg of Compound 20 (yield: 50%). HRMS (electrospray ionization method) of the obtained Compound 20 showed a measured m/z value of 828.4350 (theoretical m/z value: 828.4350 for C₃₇H₆₂N₇O₁₄⁺, [M+H]⁺).

### <Synthesis of Maleimide-DOTAGL-PEG4 (MDPEG4) (Compound 21)>

Compound 19 (11 mg, 10 µmol) was dissolved in a mixed solution of trifluoroacetic acid (1280 µL) and dichloromethane (320 µL), and the mixture was stirred at room temperature for 14 hours. Thereafter, the solvent was distilled off, and the residue was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min) to obtain 2.6 mg of Compound 19 (yield: 28%). HRMS (electrospray ionization method) of the obtained Compound 21 showed a measured m/z value of 916.4874 (theoretical m/z value: 916.4874 for C₄₁H₇₀N₇O₁₆⁺, [M+H]⁺).

### <Synthesis of Maleimide-[^{nat}In]In-DOTAGL-PEG2([^{nat}In]In-MDPEG2) (Compound 22)>

Compound 20 (1.0 mg, 1.2 µmol) was dissolved in acetate buffer solution (1.0 mol/L, pH 5.1, 200 µL), indium(III) chloride (2.7 mg, 12 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min) to obtain 0.24 mg of Compound 22 (yield: 21%). HRMS (electrospray ionization method) of the obtained Compound 22 showed a measured m/z value of 940.3161 (theoretical m/z value: 940.3154 for C₃₇H₅₉InN₇O₁₄⁺, [M+2H]⁺).

### <Synthesis of Maleimide-[^{nat}In]In-DOTAGL-PEG4([^{nat}In]In-MDPEG4) (Compound 23)>

Compound 21 (2.3 mg, 2.5 µmol) was dissolved in acetate buffer solution (1.0 mol/L, pH 5.1, 100 µL), indium(III) chloride (5.6 mg, 25 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 7/3 (20 min), flow rate: 1 mL/min) to obtain 0.69 mg of Compound 23 (yield: 30%). HRMS (electrospray ionization method) of the obtained Compound 23 showed a measured m/z value of 514.6876 (theoretical m/z value: 514.6875 for C₄₁H₆₈InN₇O₁₆⁺, [M+3H]²⁺).

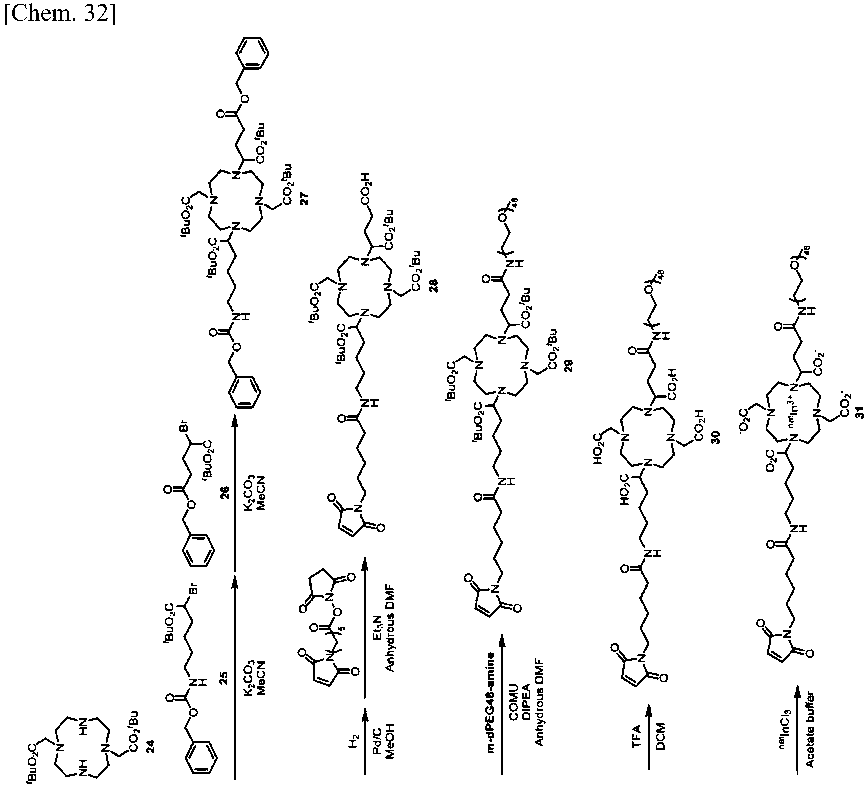

### · Synthesis of Compound 30 and In Complex 31 Thereof (Example 1-3)

### <Synthesis of Compound 24>

Compound 24 was synthesized according to a previous report (J. Chem. Soc., Chem. Commun., 1995, 185-186).

### <Synthesis of Compound 25>

Compound 25 was synthesized according to a previous report (Org. Biomol. Chem., 2013, 11, 1294-1305).

### <Synthesis of Compound 26>

Compound 26 was synthesized according to a previous report (Bioorg. Med. Chem. Lett., 2000, 10, 2133-2135).

### <Synthesis of Compound 27>

Compound 24 (1,400 mg, 3.5 mmol) was dissolved in acetonitrile (25 mL), and Compound 25 (1,678 mg, 4.2 mmol) and potassium carbonate (579 mg, 4.2 mmol) were added thereto, and then the mixture was stirred at 50°C for 24 hours. Thereafter, filtration was performed to recover the filtrate, and then the solvent was distilled off. The residue was dissolved in acetonitrile (30 mL), Compound 26 (2,496 mg, 7.0 mmol) and potassium carbonate (966 mg, 7.0 mmol) were added thereto, and then the mixture was stirred at 55°C for 15 hours. Thereafter, filtration was performed to recover the filtrate, and then the solvent was distilled off. The residue was purified by amine silica gel chromatography using a mobile phase (hexane/ethyl acetate = 1/1) to obtain 1,274 mg of Compound 27 (yield: 37%). MS (electrospray ionization method) of the obtained Compound 27 showed a measured m/z value of 996.5 (theoretical m/z value: 996.6268 for C₅₄H₈₆N₅O₁₂⁺, [M+H]⁺).

### <Synthesis of Compound 28>

Compound 27 (1,274 mg, 1.28 mmol) was dissolved in methanol (25 mL), 10% palladium on carbon (350 mg) was added thereto, and the mixture was vigorously stirred at room temperature for 48 hours under a hydrogen gas atmosphere. Subsequently, filtration was performed using a Hyflo Super Cel, and the solvent from the filtrate was distilled off. A part of the residue (100 mg, 0.13 mmol) was dissolved in anhydrous DMF (700 µL), 6-maleimidohexanoic acid N-hydroxysuccinimide ester (160 mg, 0.52 mmol) and triethylamine (52 mg, 0.52 mmol) were added thereto, and then the mixture was stirred at room temperature for 71 hours. The solution was purified by the reverse phase HPLC using the COSMOSIL C₁₈ column (5C₁₃-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min), and further purified by the reverse phase HPLC using the COSMOSIL C₁₈ column (5C₁₈-AR-II, 4.6 mm I.D. × 150 mm) and a mobile phase (elution conditions: 0.1% trifluoroacetic acid aqueous solution/0.1% trifluoroacetic acid acetonitrile solution = 7/3 (0 min) to 4/6 (30 min), flow rate: 1 mL/min) to obtain 19.6 mg of Compound 28 (yield: 16%). MS (electrospray ionization method) of the obtained Compound 28 showed a measured m/z value of 965.4 (theoretical m/z value: 965.6170 for C₄₉H₈₅N₆O₁₃⁺, [M+H]⁺).

### <Synthesis of Compound 29>

Compound 28 (17 mg, 18 µmol) was dissolved in anhydrous DMF (300 µL), COMU (7.7 mg, 36 µmol) and DIPEA (2.3 mg, 36 µmol) were added thereto at 0°C, and the mixture was stirred at 0°C for 15 minutes. Thereafter, m-dPEG48-amine (39 mg, 18 µmol) was added, and the mixture was stirred at room temperature for 136 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min) to obtain 10 mg of Compound 29 (yield: 18%). MS (electrospray ionization method) of the obtained Compound 29 showed a measured m/z value of 1546.7 (theoretical m/z value: 1546.9588 for C₁₄₆H₂₈₁N₇O₆₀²⁺, [M+2H]²⁺).

### <Synthesis of Maleimide-DOTAGL-PEG48 (MDPEG48) (Compound 30)>

Compound 29 (5.0 mg, 1.6 µmol) was dissolved in a mixed solution of trifluoroacetic acid (1.6 mL) and dichloromethane (0.4 mL), and the mixture was stirred at room temperature for 14 hours. Thereafter, the solvent was distilled off, and the residue was purified by the reverse phase HPLC using the COSMOSIL C₁₈ column (5C₁₈-AR-II, 4.6 mm I.D. × 150 mm) and a mobile phase (elution conditions: 0.1% trifluoroacetic acid aqueous solution/0.1% trifluoroacetic acid acetonitrile solution = 3/1 (0 min) to 11/9 (20 min), flow rate: 1 mL/min) to obtain 2.0 mg of Compound 30 (yield: 43%). HRMS (electrospray ionization method) of the obtained Compound 30 showed a measured m/z value of 1434.8340 (theoretical m/z value: 1434.8336 for C₁₃₀H₂₄₉N₇O₆₀²⁺, [M+2H]²⁺).

### <Synthesis of Maleimide-[^{nat}In]In-DOTAGL-PEG48([^{nat}In]In-MDPEG48) (Compound 31)>

Compound 30 (0.20 mg, 52 nmol) was dissolved in acetate buffer solution (1.0 mol/L, pH 5.1, 100 µL), indium(III) chloride (0.11 mg, 0.52 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 3/1 (0 min) to 11/9 (20 min), flow rate: 1 mL/min) to obtain 0.21 mg of Compound 31 (yield > 99%). HRMS (electrospray ionization method) of the obtained Compound 31 showed a measured m/z value of 1490.7724 (theoretical m/z value: 1490.7738 for C₁₃₀H₂₄₆InN₇O₆₀²⁺, [M+3H]²⁺).

### · Synthesis of Radiolabeled Antibody Drug (Examples 2-1 to 2-3 and Comparative Examples 2-1 to 2-3)

### <Introduction of Thiol Group to Trastuzumab>

Herceptin (3.0 mg) (manufactured by Roche) was dissolved in 0.1 mol/L borate buffer solution containing 2 mmol/L EDTA (pH 8.0, 4 mL), and purified and concentrated using Amicon Ultra-4 (recovered fraction: 50 kDa) manufactured by Millipore. 2-Iminothiolane hydrochloride was dissolved in 0.1 mol/L borate buffer solution containing 2 mmol/L EDTA (pH 8.0) to prepare a 7 mmol/L solution, and after adding it in an amount corresponding to 2 molar equivalents relative to trastuzumab in the concentrated solution, the mixture was incubated for 1 hour under light-shielded conditions. The reaction solution was purified using Zeba Spin Desalting Column (40K MWCO, 0.5 mL) to obtain trastuzumab into which thiol groups had been introduced (trastuzumab-SH). The purified trastuzumab-SH was promptly used for the labeling reaction.

The number of thiol groups introduced was determined by the Ellman method. 5,5'-Dithiobis(2-nitrobenzoic acid) (Ellman's reagent) (3.3 mg) was dissolved in 0.1 mol/L borate buffer solution containing 2 mmol/L EDTA (pH 8.0, 833 µL) to prepare an Ellman's reagent solution. In addition, a 0.1 mol/L borate buffer solution containing 2 mmol/L EDTA (pH8.0) was used as a control solution containing no antibody. Ellman's reagent solution (4 µL) was added to a trastuzumab-SH solution having a known antibody concentration (20 µL) or a control solution (20 µL), and the mixture was incubated at room temperature for 20 minutes. After incubation, the absorbance of the antibody solution at 412 nm was measured using the control solution as a reference. The antibody concentration at the time of absorbance measurement was calculated from the antibody concentration before the addition of Ellman's reagent and the solution volumes before and after the addition of Ellman's reagent. The thiol concentration was calculated using a molar extinction coefficient of 14.15 mmol/L⁻¹cm⁻¹ at 412 nm for 5-nitro-2-thiobenzoic acid, which is the product formed when Ellman's reagent reacts with thiols. The number of thiol groups introduced per antibody was calculated by dividing the thiol concentration by the antibody concentration. The number of thiol groups introduced per antibody was 1.32 ± 0.16.

### <Labeling Reaction>

### <First Labeling Step>

Compounds 8, 9, 14, 20, 21, and 30 (1.0 mg/mL DMSO solution, 3 to 8 µL) were each dissolved in 0.1 mol/L acetate buffer solution (pH 5.1, 350 µL), a [¹¹¹In]InCl₃ solution (500 to 600 µL, 35 to 37 MBq) was added thereto, and then the mixture was heated at 90°C for 6 minutes. The reaction solution was purified by the reverse phase HPLC using the COSMOSIL C₁₈ column (5C₁₈-AR-II, 4.6 mm I.D. × 150 mm) and a mobile phase (MeCN/H₂O/TFA [10/90/0.1 (0 min) to 30/70/0.1 (20 min)], flow rate: 1.0 mL/min), and the solvent of the fraction containing the target compound was distilled off under a stream of nitrogen gas.

In the present specification, radiolabeled compounds, in which the compounds 8, 9, 14, 20, 21, and 30 are coordinated to ¹¹¹In³⁺, are referred to as [¹¹¹In]In-MDPEI2, [¹¹¹In]In-MDPEI4, [¹¹¹In]In-MD, [¹¹¹In]In-MDPEG2, [¹¹¹In]In-MDPEG4, and [¹¹¹In]In-MDPEG48, respectively.

### <Second Labeling Step>

After a 0.1 mol/L phosphate buffer solution (pH 7, 500 µL) was added to the residue obtained by the first labeling step, trastuzumab-SH (0.6 mg) was added thereto, and the mixture was incubated for 4 to 6 hours at room temperature under light-shielded conditions. Thereafter, the reaction solution was purified and concentrated using Amicon Ultra-4 (recovered fraction: 50 kDa) to obtain a target radioactive metal-labeled antibody (hereinafter also referred to as "radiolabeled antibody drug"). The radiochemical purity (RCP) was measured as follows. Analysis was performed by size exclusion HPLC using a TSKgel G3000SW_{XL} column (5 µm, 7.8 mm I.D. × 300 mm) and a mobile phase (0.1 mol/L phosphate buffer solution (pH 6.8), flow rate: 0.5 mL/min), and the percentage of the area value of the radiolabeled compound relative to the area values of all detected peaks was defined as RCP (%).

In the present specification, a radiolabeled antibody drug (RIC) obtained by introducing [¹¹¹In]In-MDPEI2, [¹¹¹In]In-MDPEI4, [¹¹¹In]In-MD, [¹¹¹In]In-MDPEG2, [¹¹¹In]In-MDPEG4, and [¹¹¹In]In-MDPEG48 into Trastuzumab-SH is referred to as [¹¹¹In]In-TMDPEI2, [¹¹¹In]In-TMDPEI4, [¹¹¹In]In-TMD, [¹¹¹In]In-TMDPEG2, [¹¹¹In]In-TMDPEG4, and [¹¹¹In]In-TMDPEG48, respectively. The radiochemical yield (RCY) of each labeling step in the two-step labeling process was calculated as the percentage of radioactivity of the purified ¹¹¹In-labeled product relative to the amount of radioactivity added at the beginning of each step. In addition, the overall radiochemical yield was calculated as the percentage of radioactivity of the obtained ¹¹¹In-labeled antibody drug relative to the amount of radioactivity of the [¹¹¹In]InCl₃ solution added during the first labeling step.

In Example 2-1 ([¹¹¹In]In-TMDPEI2), the RCY of the first step was 59.1%, the RCY of the second step was 73.5%, and the overall radiochemical yield was 37.8%.

In Example 2-2 ([¹¹¹In]In-TMDPEI4), the RCY of the first step was 65.1%, the RCY of the second step was 49.3%, and the overall radiochemical yield was 26.7%.

In Example 2-3 ([¹¹¹In]In-TMDPEG48), the RCY of the first step was 76.1%, the RCY of the second step was 65.7%, and the overall radiochemical yield was 46.7%.

In Comparative Example 2-1 ([¹¹¹In]In-TMD), the RCY of the first step was 80.4%, the RCY of the second step was 25.8%, and the overall radiochemical yield was 20%.

In Comparative Example 2-2 ([¹¹¹In]In-TMDPEG2), the RCY of the first step was 75.8%, the RCY of the second step was 31.4%, and the overall radiochemical yield was 23.8%.

In Comparative Example 2-3 ([¹¹¹In]In-TMDPEG4), the RCY of the first step was 69.6%, the RCY of the second step was 40.9%, and the overall radiochemical yield was 28.5%.

Each antibody drug was synthesized via a two-step labeling reaction. The radiochemical purity of all ¹¹¹In-labeled antibody drugs was greater than 95%.

All animal experiments were conducted in accordance with the guidelines of the Animal Experimentation Committee of Kyoto University. Female BALB/c-nu/nu mice (5 weeks old) were purchased from Shimizu Laboratory Supplies Co., Ltd. The animals were housed under a 12-hour light/12-hour dark cycle, with free access to food and water.

HER2-positive ovarian cancer cells, i.e. SK-OV-3 cells were purchased from American Type Culture Collection (ATCC). The cells were cultured in RPMI-1640 medium (manufactured by Nacalai Tesque, Inc.) containing antibiotics (penicillin and streptomycin) (100 U/mL) and 10% by mass heat-inactivated fetal bovine serum (FBS), at 37°C under a 5% by volume CO₂ atmosphere.

### <Evaluation of Immunoreactivity> (Examples 3-1 to 3-3 and Comparative Examples 3-1 to 3-3)

SK-OV-3 cells (0.63, 1.25, 2.5, 5.0 × 10⁶ cells) were suspended in a medium (300 µL) in Protein LoBind tubes manufactured by Eppendorf. A ¹¹¹In-labeled antibody drug (0.74 kBq) was added to the cell suspension, and the mixture was incubated for 1 hour while being inverted and mixed. Non-specific binding was evaluated by adding an excess amount of trastuzumab (50 µg). After incubation, the cell suspension was centrifuged at 860 × g for 3 min and the supernatant was removed. The cell mass was resuspended in phosphate buffered saline (PBS) containing 1% by mass BSA, centrifuged again at 860 × g for 3 minutes, and the supernatant was removed. The washing using PBS was repeated again, and then the radioactivity of the cell mass was measured using Gamma Counter 2480 WIZARD2 manufactured by PerkinElmer Inc.

The immunoreactive fraction of each radiolabeled antibody drug was calculated according to a previous report (J. Immunol. Methods, 1984, 72, 77-89). Specifically, for each antibody drug, a scatter plot was created with the reciprocal of cell concentration (mL/million) on the horizontal axis and the reciprocal of the specific binding ratio (total applied/specific binding) on the vertical axis. The immunoreactive fraction was determined as the reciprocal of the y-intercept of the approximated linear regression line. The results are as follows.

The result of Example 3-1 ([¹¹¹In]In-TMDPEI2) was 0.99 ± 0.02, the result of Example 3-2 ([¹¹¹In]In-TMDPEI4) was 0.96 ± 0.03, and the result of Example 3-3 ([¹¹¹In]In-TMDPEG48) was 0.98 ± 0.04.

In addition, the result of Comparative Example 3-1 ([¹¹¹In]In-TMD) was 0.81 ± 0.06, the result of Comparative Example 3-2 ([¹¹¹In]In-TMDPEG2) was 0.91 ± 0.04, and the result of Comparative Example 3-3 ([¹¹¹In]In-TMDPEG4) was 0.84±0.01.

Each drug showed good HER2 binding ability, and no decrease in target binding ability of the antibody was observed through the two-step labeling reaction.

### <Cell Internalization Experiment> (Examples 4-1 to 4-3 and Comparative Examples 4-1 to 4-3)

The ¹¹¹In-labeled antibody drug (148 kBq/10⁶ cells) was added to the SK-OV-3 cell suspension (1 mL) and incubated at 4°C for 1 hour. After incubation, the cells were washed with PBS containing 1% by mass of BSA according to the method described in <Evaluation of Immunoreactivity>. The cell mass was resuspended in a new medium, dispensed by adjusting to 1.0 × 10⁶ cells per 500 µL, and incubated at 37°C under a 5% by volume CO₂ atmosphere for 0, 1, 2, 4, or 24 hours. Thereafter, centrifugation was performed at 860 × g for 3 minutes, and the supernatant was recovered as a medium fraction. Similarly, the cell mass was washed twice with PBS containing 1% by mass BSA, and all the supernatants were collected as a medium fraction. For the purpose of separating the radioactive component bound to the cell surface and the radioactive component present in the cell, the cell mass was resuspended in 0.2 mol/L sodium citrate buffer solution (300 µL) at pH 2. After incubation at 4°C for 3 minutes, centrifugation was performed at 860 × g for 3 minutes, and the supernatant was recovered as a cell surface fraction. Similarly, washing with a sodium citrate buffer solution was performed again, and the supernatants were collected as a cell surface fraction, and the remaining cell mass was recovered as a cell internalized fraction. For each fraction, radioactivity was measured using a gamma counter.

Regarding the medium fraction after 24 hours of incubation, separation based on molecular size was performed using Amicon Ultra-4 (recovered fraction: 50 kDa), and the radioactive component derived from the radiolabeled antibody drug dissociated from the cell surface and the radioactive component derived from the cell metabolites were separated. The concentrated solution was recovered as a dissociated fraction, and radioactivity was measured with a gamma counter. The radioactivity remaining in the Amicon was defined as the radioactivity of the degraded fraction. Significance testing in cell internalization experiments was performed by combining one-way analysis of variance and Bonferroni post-hoc test with a 95% confidence interval.

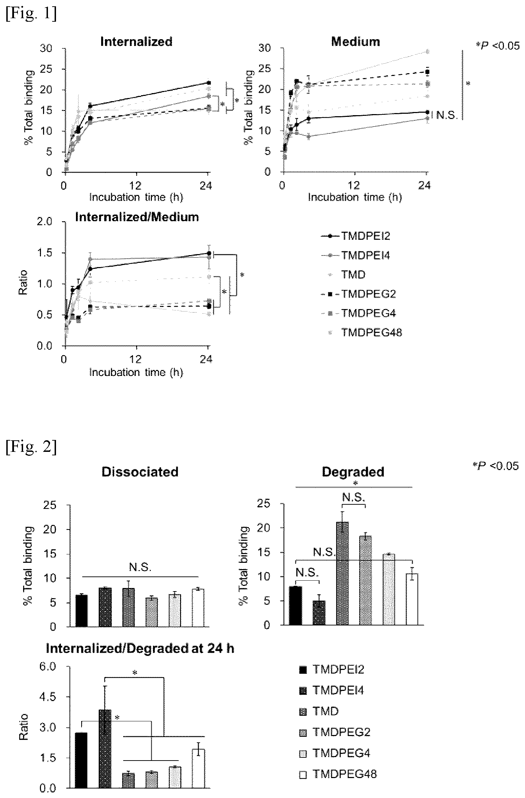

The percentage of radioactivity of each fraction is shown in Tables 6 to 11. In Tables 6 to 11, "N.D." indicates that no data is acquired. Regarding the percentages of radioactivity of the medium fraction (medium) and the cell internalized fraction (internalized), the results are collectively shown in Fig. 1. For each of the radiolabeled antibody drugs, the ratio of the radioactivity of the recovered fraction to the total radioactivity bound to the cells was defined as the percentage of radioactivity. For the cell internalized fraction, TMDPEI2/4 and TMDPEG48 after 24 hours of incubation showed higher values compared to TMD and TMDPEG2/4, indicating high intracellular retention. Regarding the medium fraction, TMDPEI2/4 showed approximately half the value compared to TMD and TMDPEG2/4, and TMDPEG48 showed a slightly lower value. In addition, the ratio obtained by dividing the amount of radioactivity of the cell internalized fraction by the amount of radioactivity of the medium fraction was, after 24 hours of incubation, in the order of TMDPEI2/4, TMDPEG48, TMDPEG2/4, and TMD from the highest drug.

The results of the percentages of radioactivity of the dissociated fraction (dissociated) and the degraded fraction (degraded) are collectively shown in Fig. 2. There was no significant difference in the amount of the radiolabeled antibody drug dissociated from the cells between the antibody drugs. Regarding the amount of radioactivity derived from the metabolized radiolabeled antibody drug, TMDPEI2/4 and TMDPEG48 showed lower values compared to TMD and TMDPEG2/4. In addition, the ratio between the internalization rate and the proportion of metabolites that flowed out of cells (internalized/degraded) at 24 hours after incubation was in the order of TMDPEI2/4, TMDPEG48, TMDPEG2/4, and TMD from the highest drug.

**[Table 6]**

| **[¹¹¹In]In-TMDPEI2** | | Incubation time (h) | | | | |
|---|---|---|---|---|---|---|
| | | **0** | **1** | **2** | **4** | **24** |
| Percentage of radioactivity (%) | Medium fraction | 6.3 ± 0.7 | 10.4 ± 1.0 | 11.5 ± 1.5 | 13.0 ± 1.1 | 14.5 ± 0.3 |
| | Cell surface fraction | 90.8 ± 1.4 | 80.4 ± 1.3 | 77.8 ± 1.6 | 71.0 ± 1.0 | 63.7 ± 0.6 |
| | Cell internalized fraction | 2.9 ± 1.6 | 9.3 ± 0.3 | 10.1 ± 0.6 | 16.0 ± 0.7 | 21.7 ± 0.3 |
| | Dissociated fraction | N.D. | N.D. | N.D. | N.D. | 6.6 ± 0.3 |
| | Degraded fraction | N.D. | N.D. | N.D. | N.D. | 8.0 ± 0.0 |
| Ratio | Cell internalized fraction/Medium fraction | 0.46±0.29 | 0.90±0.06 | 0.94±0.13 | 1.24±0.13 | 1.49±0.01 |
| | Cell internalized fraction/ Degraded fraction | N.D. | N.D. | N.D. | N.D. | 2.72 ± 0.03 |

**[Table 7]**

| **[¹¹¹In]In-TMDPEI4** | | Incubation time (h) | | | | |
|---|---|---|---|---|---|---|
| | | **0** | **1** | **2** | **4** | **24** |
| Percentage of radioactivity (%) | Medium fraction | 5.3 ± 0.5 | 9.5 ± 1.2 | 9.4 ± 0.3 | 8.6 ± 0.8 | 13.0 ± 1.2 |
| | Cell surface fraction | 93.4 ± 0.7 | 85.0 ± 0.8 | 82.6 ± 0.6 | 79.5 ± 1.2 | 68.5 ± 0.7 |
| | Cell internalized fraction | 1.3 ± 0.2 | 5.4 ± 0.4 | 8.0 ± 0.9 | 11.9 ± 0.4 | 18.5 ± 0.7 |
| | Dissociated fraction | N.D. | N.D. | N.D. | N.D. | 8.0 ± 0.2 |
| | Degraded fraction | N.D. | N.D. | N.D. | N.D. | 5.0 ± 1.2 |
| Ratio | Cell internalized fraction/Medium fraction | 0.25 ± 0.02 | 0.58 ± 0.11 | 0.86 ± 0.12 | 1.40 ± 0.11 | 1.43 ± 0.19 |
| | Cell internalized fraction/ Degraded fraction | N.D. | N.D. | N.D. | N.D. | 3.87 ± 1.17 |

**[Table 8]**

| **[¹¹¹In]In-TMD** | | Incubation time (h) I | | | | |
|---|---|---|---|---|---|---|
| | | **0** | **1** | **2** | **4** | **24** |
| Percentage of radioactivity (%) | Medium fraction | 8.5 ± 1.0 | 16.1 ± 1.8 | 18.6 ± 1.6 | 21.0 ± 1.9 | 29.1 ± 0.6 |
| | Cell surface fraction | 88.1 ± 2.1 | 73.8 ± 1.4 | 66.7 ± 4.6 | 63.9 ± 1.8 | 56.1 ± 0.4 |
| | Cell internalized fraction | 3.3 ± 1.2 | 10.1 ± 1.0 | 14.7 ± 4.1 | 15.0 ± 0.3 | 14.8 ± 0.8 |
| | Dissociated fraction | N.D. | N.D. | N.D. | N.D. | 7.9 ±1.6 |
| | Degraded fraction | N.D. | N.D. | N.D. | N.D. | 21.2 ± 2.1 |
| Ratio | Cell internalized fraction/Medium fraction | 0.39 ± 0.10 | 0.64 ± 0.13 | 0.79 ± 0.22 | 0.72 ± 0.08 | 0.51 ± 0.04 |
| | Cell internalized fraction/ Degraded fraction | N.D. | N.D. | N.D. | N.D. | 0.70 ± 0.11 |

**[Table 9]**

| **[¹¹¹In]In-TMDPEG2** | | Incubation time (h) | | | | |
|---|---|---|---|---|---|---|
| | | **0** | **1** | **2** | **4** | **24** |
| Percentage of radioactivity (%) | Medium fraction | 5.6 ± 0.2 | 19.0 ± 0.7 | 22.0 ± 0.2 | 21.2 ± 2.1 | 24.3 ± 1.0 |
| | Cell surface fraction | 92.9 ± 0.7 | 71.8 ± 1.5 | 68.0 ± 0.3 | 65.7 ± 1.0 | 60.1 ± 1.1 |
| | Cell internalized fraction | 1.4 ± 0.5 | 9.2 ± 0.9 | 10.0 ± 0.5 | 13.1 ± 1.2 | 15.6 ± 0.7 |
| | Dissociated fraction | N.D. | N.D. | N.D. | N.D. | 5.9 ± 0.5 |
| | Degraded fraction | N.D. | N.D. | N.D. | N.D. | 18.3 ± 0.7 |
| Ratio | Cell internalized fraction/Medium fraction | 0.25 ± 0.07 | 0.48 ± 0.03 | 0.45 ± 0.03 | 0.63 ± 0.11 | 0.64 ± 0.04 |
| | Cell internalized fraction/ Degraded fraction | N.D. | N.D. | N.D. | N.D. | 0.85 ± 0.04 |

**[Table 10]**

| **[¹¹¹In]ln-TMDPEG4** | | Incubation time (h) | | | | |
|---|---|---|---|---|---|---|
| | | **0** | **1** | **2** | **4** | **24** |
| Percentage of radioactivity (%) | Medium fraction | 3.6 ± 0.6 | 15.1 ± 0.4 | 20.6 ± 0.8 | 20.8 ± 0.4 | 21.3 ± 0.8 |
| | Cell surface fraction | 95.5 ± 0.6 | 78.0 ± 0.7 | 71.2 ± 0.7 | 67.1 ± 0.7 | 63.2 ± 1.4 |
| | Cell internalized fraction | 0.9 ± 0.4 | 6.9 ± 0.3 | 8.3 ± 0.4 | 12.1 ± 0.4 | 15.5 ± 0.7 |
| | Dissociated fraction | N.D. | N.D. | N.D. | N.D. | 6.7 ± 0.6 |
| | Degraded fraction | N.D. | N.D. | N.D. | N.D. | 14.6 ± 0.2 |
| Ratio | Cell internalized fraction/Medium fraction | 0.25 ± 0.12 | 0.46 ± 0.01 | 0.40 ± 0.03 | 0.58 ± 0.01 | 0.73 ± 0.02 |
| | Cell internalized fraction/ Degraded fraction | N.D. | N.D. | N.D. | N.D. | 1.06 ± 0.04 |

**[Table 11]**

| [**¹¹¹In]In-TMDPEG48** | | Incubation time (h) | | | | |
|---|---|---|---|---|---|---|
| | | **0** | **1** | **2** | **4** | **24** |
| Percentage radioactivity (%) | Medium fraction | 7.5 ± 1.4 | 14.8 ± 1.6 | 15.5 ± 1.8 | 14.5 ± 1.7 | 18.3 ± 1.2 |
| | Cell surface fraction of | 90.7 ± 1.9 | 75.5 ± 3.3 | 71.0 ± 4.1 | 71.1 ± 1.1 | 61.4 ± 2.5 |
| | Cell internalized fraction | 1.7 ± 0.6 | 9.7 ± 1.7 | 13.5 ± 2.3 | 14.4 ± 2.1 | 20.2 ± 2.4 |
| | Dissociated fraction | N.D. | N.D. | N.D. | N.D. | 7.8 ± 0.3 |
| | Degraded fraction | N.D. | N.D. | N.D. | N.D. | 10.5 ± 1.3 |
| Ratio | Cell internalized fraction/Medium fraction | 0.23 ± 0.05 | 0.65 ± 0.05 | 0.86 ± 0.05 | 1.02 ± 0.25 | 1.11 ± 0.16 |
| | Cell internalized fraction/Degraded fraction | N.D. | N.D. | N.D. | N.D. | 1.94 ± 0.32 |

### <Preparation of Tumor Transplantation Model Mice>

SK-OV-3 cells (1.0 × 10⁶ or 4.0 × 10⁶ cells/mouse) were suspended in a mixed solution of RPMI-1640 and Matrigel manufactured by Corning Life Sciences (1:1, 100 µL), and subcutaneously transplanted to the right waist of a female BALB/c-nu/nu mice (5 weeks old) under isoflurane anesthesia. The mice were housed for 3 to 5 weeks, and those with a tumor diameter reaching 0.5 cm were used for the in vivo radioactivity distribution experiment, while those with a tumor diameter reaching 0.8 cm were used for the SPECT/CT imaging described later.

### <In Vivo Radioactivity Distribution Experiment Using Tumor Transplantation Model Mice> (Examples 5-1 to 5-3 and Comparative Examples 5-1 to 5-3)

A physiological saline solution (100 µL) containing ¹¹¹In-labeled antibody drug (315 to 333 kBq, 18 µg) was administered via the tail vein of SK-OV-3 tumor transplantation model mice, and the mice were euthanized at 4, 24, 48, 72, 96, or 192 hours after administration (each n = 4). Blood and each organ were collected, and the weight and radioactivity of the organ were measured. The value obtained by dividing the percentage of the amount of radioactivity relative to the administered radioactivity (%ID) by the blood weight or organ weight (g) (%ID/g) was determined as an index indicating the in vivo radioactivity distribution. These results are shown in Tables 12 to 17, categorized by the radiolabeled antibody drug administered to the mice.

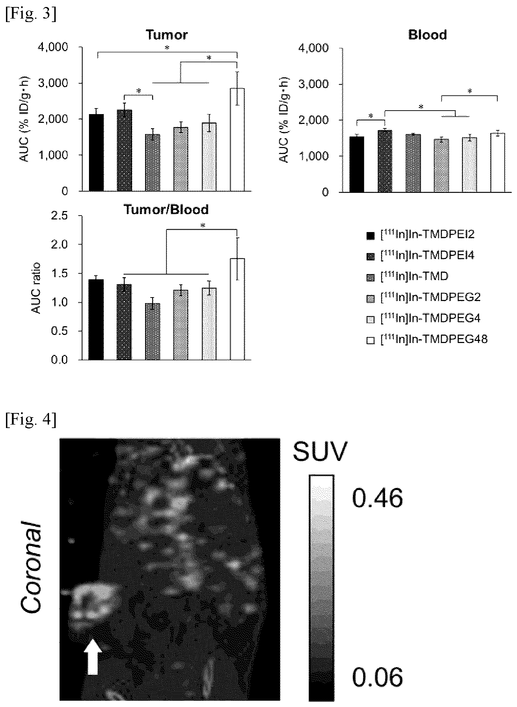

In addition, the area under the curve (AUC) of blood and tumor was calculated using GraphPad Prism software based on the results of the in vivo radioactivity distribution experiment without decay correction. Significance testing in in vivo radioactivity distribution experiments was performed by combining one-way analysis of variance and Bonferroni post-hoc test with a 95% confidence interval. The results are shown in Fig. 3.

**[Table 12]**

| **[¹¹¹In]In-TMDPEI2** | Tissue | Time after administration (h) | | | | | |
|---|---|---|---|---|---|---|---|
| | | **4** | **24** | **48** | **72** | **96** | **192** |
| | Blood | 34.51 (4.07) | 19.43 (2.43) | 16.62 (2.18) | 14.63 (3.82) | 11.26 (1.76) | 6.32 (1.66) |
| | Spleen | 8.78 (1.55) | 6.83 (1.42) | 7.13 (1.46) | 7.55 (0.77) | 5.90 (0.61) | 5.17 (0.74) |
| | Pancreas | 1.88 (0.35) | 2.49 (0.64) | 2.04 (0.36) | 2.68 (0.48) | 2.54 (0.80) | 1.91 (0.80) |
| | Stomach (%ID) | 0.48 (0.16) | 0.37 (0.05) | 0.34 (0.05) | 0.33 (0.05) | 0.35 (0.07) | 0.24 (0.05) |
| | Intestine | 2.66 (0.28) | 2.05 (0.35) | 1.93 (0.21) | 1.95 (0.28) | 1.73 (0.31) | 0.83 (0.12) |
| % ID/g | Kidney | 8.45 (1.44) | 7.31 (1.24) | 7.39 (0.89) | 7.21 (1.03) | 6.58 (0.88) | 3.84 (0.19) |
| | Liver | 9.71 (1.40) | 7.46 (1.79) | 7.40 (0.79) | 8.48 (1.30) | 6.58 (0.97) | 5.11 (1.03) |
| | Heart | 7.97 (1.21) | 4.76 (0.77) | 3.95 (0.36) | 3.06 (0.42) | 2.67 (0.12) | 1.64 (0.30) |
| | Lung | 18.12 (3.14) | 11.10 (1.43) | 8.85 (0.78) | 8.75 (1.74) | 6.84 (1.10) | 4.18 (1.07) |
| | Brain | 0.70 (0.29) | 0.59 (0.16) | 0.43 (0.06) | 0.45 (0.10) | 0.29 (0.04) | 0.19 (0.05) |
| | Tumor | 2.47 (0.84) | 21.72 (6.86) | 31.16 (3.10) | 33.47 (11.26) | 32.58 (8.66) | 24.80 (6.21) |
| | Muscle | 0.96 (0.41) | 1.62 (0.44) | 1.56 (0.22) | 1.50 (0.31) | 1.18 (0.12) | 0.68 (0.15) |
| Ratio | Tumor/Blood | 0.07 (0.02) | 1.10 (0.27) | 1.90 (0.33) | 2.25 (0.28) | 2.87 (0.52) | 3.97 (0.64) |
| | Tumor/Muscle | 2.63 (0.43) | 13.37 (1.93) | 20.42 (4.45) | 23.26 (9.67) | 28.07 (9,59) | 37.22 (9.18) |

**[Table 13]**

| **[¹¹¹In]In-TMDPEI4** | Tissue | Time after administration (h) | | | | | |
|---|---|---|---|---|---|---|---|
| | | **4** | **24** | **48** | **72** | **96** | **192** |
| | Blood | 34.73 (2.31) | 24.24 (2.06) | 19.56 (2.52) | 16.16 (1.24) | 12.61 (2.72) | 8.60 (1.54) |
| | Spleen | 7.65 (1.71) | 7.38 (1.32) | 7.51 (1.35) | 6.47 (1.04) | 6.50 (1.11) | 5.66 (1.50) |
| | Pancreas | 1.31 (0.26) | 2.12(0.11) | 1.75 (0.38) | 1.59 (0.26) | 1.39 (0.35) | 1.43 (0.32) |
| | Stomach (%ID) | 0.41 (0.09) | 0.36 (0.04) | 0.31 (0.03) | 0.33 (0.04) | 0.31 (0.06) | 0.24 (0.07) |
| | Intestine | 2.20 (0.56) | 2.15 (0.02) | 1.66 (0.19) | 1.69 (0.30) | 1.54 (0.17) | 1.15 (0.17) |
| % ID/g | Kidney | 7.64 (0.88) | 8.02 (0.48) | 7.88 (0.99) | 7.76 (0.48) | 7.22 (0.99) | 5.31 (0.92) |
| | Liver | 12.03 (1.76) | 9.73 (1.32) | 9.39 (1.52) | 8.57 (1.67) | 9.36 (2.14) | 5.75 (0.49) |
| | Heart | 8.56 (0.75) | 5.12 (0.42) | 4.59 (0.55) | 3.17 (0.30) | 3.20 (0.63) | 2.23 (0.39) |
| | Lung | 18.93 (2.11) | 14.01 (2.87) | 11.13 (2.75) | 9.71 (1.04) | 8.07 (1.10) | 5.09 (1.29) |
| | Brain | 0.56 (0.06) | 0.52 (0.10) | 0.37 (0.08) | 0.35 (0.02) | 0.29 (0.03) | 0.22 (0.05) |
| | Tumor | 4.00 (3.03) | 23.35 (4.54) | 32.32 (1.29) | 41.31 (13.06) | 29.72 (3.61) | 30.52 (13.24) |
| | Muscle | 0.69 (0.04) | 1.62 (0.18) | 1.62 (0.26) | 1.58 (0.23) | 1.12 (0.23) | 0.95 (0.20) |
| Ratio | Tumor/Blood | 0.12 (0.10) | 0.97 (0.21) | 1.67 (0.16) | 2.60 (0.95) | 2.40 (0.29) | 3.53 (1.18) |
| | Tumor/Muscle | 5.71 (4.05) | 14.54 (3.41) | 20.24 (2.46) | 26.82 (11.08) | 27.07 (3.59) | 33.04 (14.43) |

**[Table 14]**

| [¹¹¹**In]In-TMD** | Tissue | Time after administration (h) | | | | | |
|---|---|---|---|---|---|---|---|
| | | **4** | **24** | **48** | **72** | **96** | **192** |
| | Blood | 33.23 (2.02) | 20.58 (1.27) | 17.94 (1.94) | 14.65 (0.52) | 13.18 (0.93) | 7.79 (1.88) |
| | Spleen | 7.87 (4.29) | 4.82 (1.12) | 4.58 (0.83) | 4.22 (0.30) | 5.14 (1.55) | 3.76 (0.82) |
| | Pancreas | 1.42 (0.01) | 1.66 (0.14) | 1.55 (0.08) | 1.50 (0.10) | 1.31 (0.10) | 0.96 (0.09) |
| | Stomach (%ID) | 0.36 (0.06) | 0.35 (0.03) | 0.34 (0.05) | 0.28 (0.03) | 0.26 (0.03) | 0.20 (0.03) |
| | Intestine | 2.18 (0.25) | 1.88 (0.28) | 1.66 (0.30) | 1.39 (0.06) | 1.29 (0.29) | 0.78 (0.14) |
| % ID/g | Kidney | 8.48 (0.89) | 8.00 (0.50) | 7.35 (0.57) | 6.79 (0.48) | 6.39 (052) | 4.65 (0.90) |
| | Liver | 8.34 (1.16) | 6.43 (0.45) | 6.44 (1.35) | 6.08 (0.60) | 5.51 (0.90) | 5.05 (0.66) |
| | Heart | 6.77 (1.28) | 3.79 (0.49) | 3.92 (0.48) | 2.97 (0.27) | 2.65 (0.14) | 1.58 (0.19) |
| | Lung | 15.97 (3.08) | 10.41 (1.97) | 9.54 (1.10) | 7.35 (0.48) | 7.24 (1.24) | 4.89 (0.89) |
| | Brain | 0.58 (0.10) | 0.40 (0.04) | 0.33 (0.03) | 0.32 (0.04) | 0.29 (0.04) | 0.18 (0.06) |
| | Tumor | 5.64 (2.13) | 12.29 (2.18) | 21.19 (3.99) | 24.72 (4.19) | 25.32 (6.99) | 22.30 (4.29) |
| | Muscle | 0.83 (0.11) | 1.32 (0.21) | 1.17 (0.05) | 1.12 (0.13) | 1.07 (0.01) | 0.62 (0.13) |
| Ratio | Tumor/Blood | 0.17 (0.06) | 0.60 (0.12) | 1.21 (0.34) | 1.68 (0.22) | 1.92 (0.51) | 3.07 (1.27) |
| | Tumor/Muscle | 6.69 (1.75) | 9.38 (1.07) | 18.25 (3.82) | 22.41 (5.27) | 23.76 (6.52) | 38.12 (16.44) |

**[Table 15]**

| **[¹¹¹In]In-TMDPEG2** | Tissue | Time after administration (h) | | | | | |
|---|---|---|---|---|---|---|---|
| | | **4** | **24** | **48** | **72** | **96** | **192** |
| | Blood | 31.90 (1.38) | 18.88 (1.04) | 15.57 (1.42) | 14.12 (2.43) | 10.76 (1.39) | 6.97 (1.49) |
| | Spleen | 8.11 (1.99) | 5.77 (1.26) | 4.01 (0.56) | 4.61 (0.60) | 4.91 (1.48) | 4.77 (1.61) |
| | Pancreas | 1.35 (0.09) | 1.47 (0.14) | 1.49 (0.12) | 1.50 (0.13) | 1.29 (0.06) | 0.99 (0.08) |
| | Stomach (%ID) | 0.35 (0.04) | 0.33 (0.04) | 0.31 (0.08) | 0.29 (0.04) | 0.28 (0.03) | 0.20 (0.03) |
| | Intestine | 2.43 (0.45) | 1.45 (0.08) | 1.26 (0.12) | 1.21 (0.12) | 1.24 (0.08) | 0.89 (0.12) |
| % ID/g | Kidney | 8.27 (0.73) | 6.69 (0.20) | 6.32 (0.88) | 6.10 (0.29) | 5.48 (0.13) | 4.30 (0.49) |
| | Liver | 8.56 (1.06) | 5.20 (1.70) | 4.14 (0.81) | 4.18 (0.46) | 4.35 (0.38) | 3.63 (0.64) |
| | Heart | 6.87 (0.23) | 3.86 (0.66) | 3.41 (0.28) | 3.25 (0.15) | 2.76 (0.39) | 1.73 (0.29) |
| | Lung | 16.84 (2.06) | 10.01 (1.26) | 7.06 (1.23) | 6.64 (1.15) | 6.11 (0.47) | 4.24 (0.89) |
| | Brain | 0.65 (0.07) | 0.39 (0.04) | 0.43 (0.14) | 0.40 (0.03) | 0.32 (0.05) | 0.19 (0.02) |
| | Tumor | 7.48 (5.24) | 16.88 (2.86) | 22.35 (3.03) | 25.99 (4.61) | 26.02 (1.79) | 29.25 (5.89) |
| | Muscle | 0.97 (0.19) | 1.43 (0.17) | 1.13 (0.05) | 1.23 (0.31) | 1.08 (0.19) | 0.63 (0.13) |
| Ratio | Tumor/Blood | 0.23 (0.16) | 0.89 (0.14) | 1.45 (0.30) | 1,86 (0.30) | 2.46 (0,43) | 4.26 (0.71) |
| | Tumor/Muscle | 8.35 (6.18) | 11.91 (2.12) | 19.86 (3.10) | 21.59 (3.55) | 24.83 (5.58) | 47.43 (10.46) |

**[Table 16]**

| [**¹¹¹In]In]-TMDPFC4** | Tissue | Time after administration (h) | | | | | |
|---|---|---|---|---|---|---|---|
| | | **4** | **24** | **48** | **72** | **96** | **192** |
| | Blood | (2,72) | 19.25 (2.50) | 16.62 (2.31) | 14.76 (1.58) | 12.38 (1.91) | 5.43 (0.68) |
| | Spleen | 7.61 (2.39) | 5.21 (0.79) | 4.97 (1.44) | 5.28 (1.20) | 4.16 (0.46) | 3.11 (0.27) |
| | Pancreas | 1.50 (0.19) | 1.64 (0.20) | 1.68 | 1.59 (0.19) | 1.35 (0.10) | 0.84 (0.09) |
| | Stomach (%ID) | 0.32 (0.04) | 0.28 (0.03) | 0.24 (0.04) | 0.22 (0.03) | 0.23 (0.02) | 0.17 (0.06) |
| | Intestine | 31.74 2.85 | 1.33 (0.17) | 1.43 (0.21) | 1.42 (0.22) | 1.26 (0.19) | 0.67 (0.13) |
| % ID/g | Kidney | (0.46) 7.84 (0.81) | 6.30 (0.95) | 6.81 (1.03) | 7.12 (0.46) | 6.23 (0.71) | 3.64 (0.65) |
| | Liver | 8.23 (1.32) | 4.68 (0.88) | (0.21) 5.16 (0.82) | 4.89 (0.43) | ) 4.46 (0.71) 28.98 | 4.83 (0.95) |
| | Heart | 6.62 (1.23) | 4.16 (0.77) | 3.60 (0.44) | 3.36 (0.11) | 2.66 (0.30) | 1.41 (0.29) |
| | Lung | 14.86 (2,77) | 9.13 (1.71) | 8.75 (1.88) | 8.51 (1.54) | 7.26 (1.32) | 3.38 (0.40) |
| | Brain | 0.73 (0.11) | 0.36 (0.05) | 0.39 (0.04) | 0.34 (0.03) | 0.35 (0.05) | 0.16 (0.04) |
| | Tumor | 8,61 (4.84) | 20.21 (2.24) | 27.50 (9.29) | 23.87 (2.73) | (9.52) | 21.81 (3.97) |
| | Muscle | 0.85 (0.07) | 1.40 (0.13) | 1.36 (0.28) | 1.25 (0.14) | 1.00 (0.12) | 0.63 (0.19) |
| Ratio | Tumor/Blood | 0.27 (0.15) | 1.07 (0.25) | 1.63 (0.33) | 1.63 (0.20) | 2.35 (0.77) | 4.02 (0.52) |
| | Tumor/Muscle | 9.94 (5.41) | 14.61 (2.57) | 19.86 (2.77) | 19.34 (3.35) | 28.76 (8.32) | 35.64 (5.81) |

**[Table 17]**

| **[¹¹¹In]In-TMDPEG48** | Tissue | Time after administration (h) | | | | | |
|---|---|---|---|---|---|---|---|
| | | **4** | **24** | **48** | **72** | **96** | **192** |
| | Blood | 30.70 (1.39) | 21.69 (1.36) | 19.36 (2.47) | 15.58 (0.70) | 13.22 (2.87) | 8.65 (1.11) |
| | Spleen | 5.58 (1.27) | 5.16 (0.30) | 5.35 (0.99) | 5.10 (0.45) | 4.19 (0.80) | 4.33 (1.18) |
| | Pancreas | 1.60 (0.33) | 2.03 (0.35) | 1.64 (0.20) | 1.68 (0.37) | 1.37 (0.20) | 1.19 (0.11) |
| | Stomach (%ID) | 0.36 (0.07) | 0.32 (0.05) | 0.39(0.14) | 0.26 (0.05) | 0.24 (0.07) | 0.20 (0.01) |
| | Intestine | 2.10 (0.47) | 1.43 (0.22) | 1.52 (0.38) | 1.37 (0.13) | 1.10 (0.14) | 0.94 (0.11) |
| % ID/g | Kidney | 6.58 (0.69) | 5.82 (0.67) | 5.63 (0.77) | 4.89 (0.47) | 4.57 (0.68) | 3.09 (0.32) |
| | Liver | 6.28 (0.81) | 4.99 (0.22) | 5.63 (1.17) | 5.86 (1.22) | 5.06 (0.99) | 3.45 (0.77) |
| | Heart | 7.02 (0.68) | 4.81 (0.60) | 4.22 (0.58) | 3.68 (0.61) | 3.13 (0.22) | 2.00 (0.32) |
| | Lung | 15.77 (1.94) | 11.68 (0.49) | 11.53 (2.53) | 8.44 (1.21) | 7.80 (0.17) | 5.43 (0.81) |
| | Brain | 0.56 (0.07) | 0.43 (0.04) | 0.36 (0.09) | 0.34 (0.04) | 0.27 (0.03) | 0.23 (0.02) |
| | Tumor | 4.01 (1.64) | 20.41 (3.04) | 39.64 (11.78) | 62.93 (20.56) | 41.13 (2.65) | 35.50 (11.13) |
| | Muscle | 0.79 (0.11) | 1.46 (0.11) | 1.31 (0.20) | 1.18 (0.24) | 1.09 (0.23) | 0.65 (0.13) |
| Ratio | Tumor/Blood | 11.13 (0.05) | 0.95 (0.19) | 2.05 (0.60) | 4.01 (1.15) | 3.27 (1.01) | 4.17(1.45) |
| | Tumor/Muscle | 5.10 (2.02) | 14.11 (3.04) | 30.64 (10.13) | 53.55 (12.90) | 39.08 (9.73) | 56.46 (19.33) |

As shown in Tables 12 to 17, regarding radioactivity uptake of the tumor, all the ¹¹¹In-labeled antibodies reached the maximum values at approximately 72 hours after administration. At 72 hours after administration, the tumor accumulation of [¹¹¹In]In-TMDPEI2, [¹¹¹In]In-TMDPEI4, and [¹¹¹In]In-TMDPEG48 was 33.5 ± 11.3, 41.3 ± 13.1, and 62.9 ± 20.6 %ID/g, respectively, which were higher compared to [¹¹¹In]In-TMDPEG2, [¹¹¹In]In-TMDPEG4, and [¹¹¹In]In-TMD (26.0 ± 4.6, 23.9 ± 2.7, and 24.7 ± 4.2 %ID/g, respectively).

As shown in Fig. 3, regarding the AUC in the tumor, [¹¹¹In]In-TMDPEG48 showed the highest value, and [¹¹¹In]In-TMDPEI2/4 also showed a higher value compared to [¹¹¹In]In-TMD and [¹¹¹In]In-TMDPEG2/4.

### <In Vivo Blocking Experiment Using Tumor Transplantation Model Mice>

### (Example 6-1)

A physiological saline solution (100 µL) containing [¹¹¹In]In-TMDPEI2 (148 kBq) and trastuzumab (2.0 mg/mouse) was administered via the tail vein of the SK-OV-3 tumor transplantation model mice, and the mice were euthanized at 72 hours after the administration (n = 4). Blood and each organ were collected, and the weight and radioactivity of the organ were measured. Significance testing in in vivo blocking experiments was performed using Student's t-test with a 95% confidence interval. The results are shown in Table 18.

As shown in Table 18, co-administration of trastuzumab significantly reduced tumor accumulation of [¹¹¹In]In-TMDPEI2 (14.8 ± 1.0%ID/g).

**[Table 18]**

| **[¹¹¹In]In-TMDPEI2 + Blocking** | Tissue | Time after administration (h) |
|---|---|---|
| | | **72** |
| | Blood | 9.82 (1.76) |
| | Spleen | 5.00 (0.45) |
| | Pancreas | 1.18 (0.06) |
| | Stomach (%ID) | 0.19 (0.03) |
| | Intestine | 1.13 (0.06) |
| *%* ID/g | Kidney | 5.97 (0.51) |
| | Liver | 5.51 (0.78) |
| | Heart | 3.12 (0.05) |
| | Lung | 6.40 (1.05) |
| | Brain | 0.21 (0.02) |
| | Tumor | 14.78 (0.96) |
| | Muscle | 0.91 (0.11) |
| Ratio | Tumor/Blood | 1.54 (0.29) |
| | Tumor/Muscle | 16.51 (2.98) |

### <SPECT/CT Imaging> (Example 7-1)

A physiological saline solution (100 µL) containing [¹¹¹In]In-TMDPEI2 (0.7 MBq, 50 µg) was administered via the tail vein of the SK-OV-3 tumor transplantation model mice. SPECT/CT was performed at 72 hours after administration using the FX3300 pre-clinical imaging system manufactured by Gamma Medica-Ideas. The SPECT images were captured using a pinhole collimator with a diameter of 1.0 mm and a focal length of 75 mm at a rotation radius of 35 mm, a projection time of 100 seconds, and the number of projections of 32 times under isoflurane anesthesia. After SPECT, CT (tube voltage: 60 kV, tube current: 270 µA) was performed. Image reconstruction by a three-dimensional ordered subset expectation maximization (3D-OSEM) method was performed for the projection data of SPECT. The obtained SPECT/CT images were analyzed with PMOD software (version 3.6, PMOD Technologies).

SPECT/CT imaging results are shown in Fig. 4. Although [¹¹¹In]In-TMDPEI2 partially showed background accumulation derived from blood, the tumor could be clearly visualized.

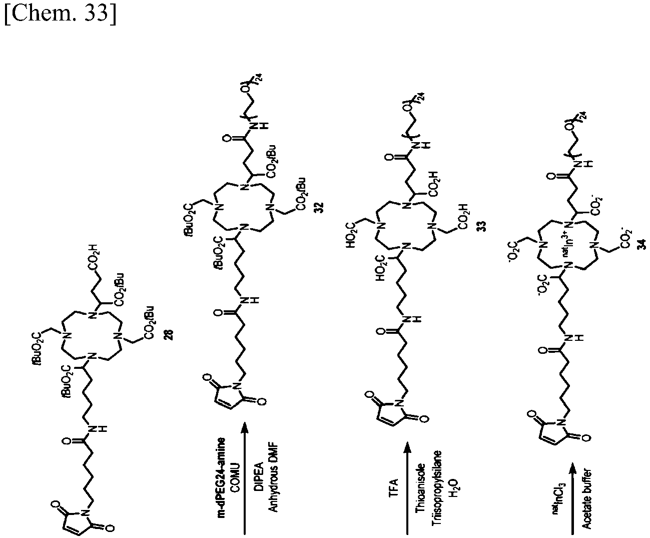

### · Synthesis of Compound 33 and In Complex 34 Thereof (Example 1-4)

### <Synthesis of Compound 32>

Compound 28 (11 mg, 11 µmol) was dissolved in anhydrous DMF (200 µL), COMU (20 mg, 46 µmol) and DIPEA (5.9 mg, 46 µmol) were added thereto at 0°C, and the mixture was stirred at 0°C for 15 minutes. Thereafter, m-dPEG24-amine (25 mg, 23 µmol) was added, and the mixture was stirred at room temperature for 14 hours. The solution was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 1 mL/min) to obtain 12 mg of Compound 32 (yield: 50%). MS (electrospray ionization method) of the obtained Compound 32 showed a measured m/z value of 1018.6 (theoretical m/z value: 1018.6442 for C₉₈H₁₈₅N₇O₃₆²⁺, [M+2H]²⁺).

### <Synthesis of Maleimide-DOTAGL-PEG24 (MDPEG24) (Compound 33)>

Compound 32 (5.8 mg, 2.9 µmol) was dissolved in a mixed solution of trifluoroacetic acid (1.9 mL), thioanisole (60 µL), triisopropylsilane (20 µL), and water (20 µL), and the mixture was stirred at room temperature for 56 hours. Thereafter, the solvent was distilled off, and the residue was purified by the reverse phase HPLC using the COSMOSIL PBr column (3PBr, 4.6 mm I.D. × 150 mm) and a mobile phase (elution conditions: 0.01% trifluoroacetic acid aqueous solution/0.01% trifluoroacetic acid acetonitrile solution = 9/1 (0 min) to 3/2 (30 min), flow rate: 1 mL/min) to obtain 0.47 mg of Compound 33 (yield: 9.1%). MS (electrospray ionization method) of the obtained Compound 33 showed a measured m/z value of 906.5 (theoretical m/z value: 906.0173 for C₈₂H₁₅₃N₇O₃₆²⁺, [M+2H]²⁺).

### <Synthesis of Maleimide-[^{nat}In]In-DOTAGL-PEG24([^{nat}In]In-MDPEG24) (Compound 34)>

Compound 33 (0.47 mg, 0.26 µmol) was dissolved in acetate buffer solution (1.0 mol/L, pH 5.1, 100 µL), indium(III) chloride (0.57 mg, 2.6 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and a mobile phase (elution conditions: 0.1% trifluoroacetic acid aqueous solution/0.1% trifluoroacetic acid acetonitrile solution = 4/1 (0 min) to 1/1 (30 min), flow rate: 1 mL/min), and then purified by the reverse phase HPLC using the COSMOSIL PBr column (3PBr, 4.6 mm I.D. × 150 mm) and the mobile phase (elution conditions: 0.01% trifluoroacetic acid aqueous solution/0.01% trifluoroacetic acid acetonitrile solution = 9/1 (0 min) to 3/2 (30 min), flow rate: 1 mL/min) to obtain 0.13 mg of Compound 34 (yield: 26%). MS (electrospray ionization method) of the obtained Compound 34 showed a measured m/z value of 647.6 (theoretical m/z value: 647.3163 for C₈₂H₁₅₄InN₈O₃₆²⁺, [M+3H+NH₄]³⁺).

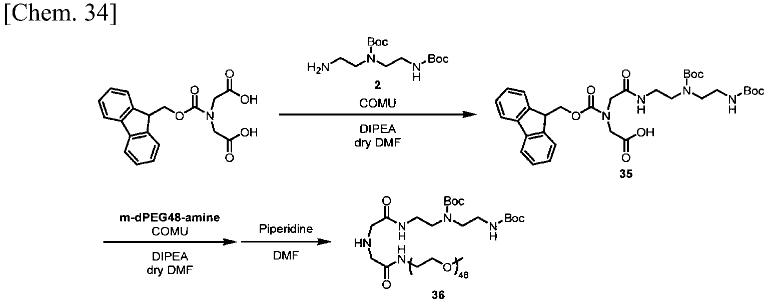

### · Synthesis of Compound 38 and In Complex 39 Thereof (Example 1-5)

### <Synthesis of Compound 35>

2-(carboxymethylamino)-3-(9H-fluoren-9-ylmethoxy)-3-oxopropanoic acid (300 mg, 0.84 mmol) was dissolved in anhydrous DMF (1,990 µL), COMU (434 mg, 1.01 mmol) and DIPEA (164 mg, 1.27 mmol) were added thereto at 0°C, and the mixture was stirred at 0°C for 15 minutes. Then, Compound 2 (306 mg, 1.01 mmol) was added, and the mixture was stirred at room temperature for 12 hours. Water and ethyl acetate were added to the reaction solution to perform a liquid separation operation, then the organic layer was washed with a saturated saline aqueous solution, and the recovered organic layer was dried over sodium sulfate and then filtered. The filtrate was distilled off under reduced pressure, and then the residue was purified by silica gel chromatography using a mobile phase (chloroform/methanol = 7/3) to obtain 188 mg of Compound 35 (yield: 35%). MS (electrospray ionization method) of the obtained Compound 35 showed a measured m/z value of 641.2 (theoretical m/z value: 641.3182 for C₃₃H₄₅N₄O₉⁺, [M+H]⁺).

### <Synthesis of Compound 36>

Compound 35 (9.0 mg, 14 µmol) was dissolved in anhydrous DMF (240 µL), COMU (12 mg, 28 µmol) and DIPEA (3.6 mg, 28 µmol) were added thereto at 0°C, and the mixture was stirred at 0°C for 15 minutes. Thereafter, m-dPEG48-amine (30 mg, 14 µmol) was added, and the mixture was stirred at room temperature for 11 hours. Piperidine (60 µL) was added to the reaction solution, the mixture was stirred at room temperature for 4 hours, and then the solvent was distilled off under reduced pressure. The residue was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min) to obtain 20 mg of Compound 36 (yield: 55%). MS (electrospray ionization method) of the obtained Compound 36 showed a measured m/z value of 849.2 (theoretical m/z value: 849.5193 for C₁₁₅H₂₃₂N₅O₅₄³⁺, [M+3H]³⁺).

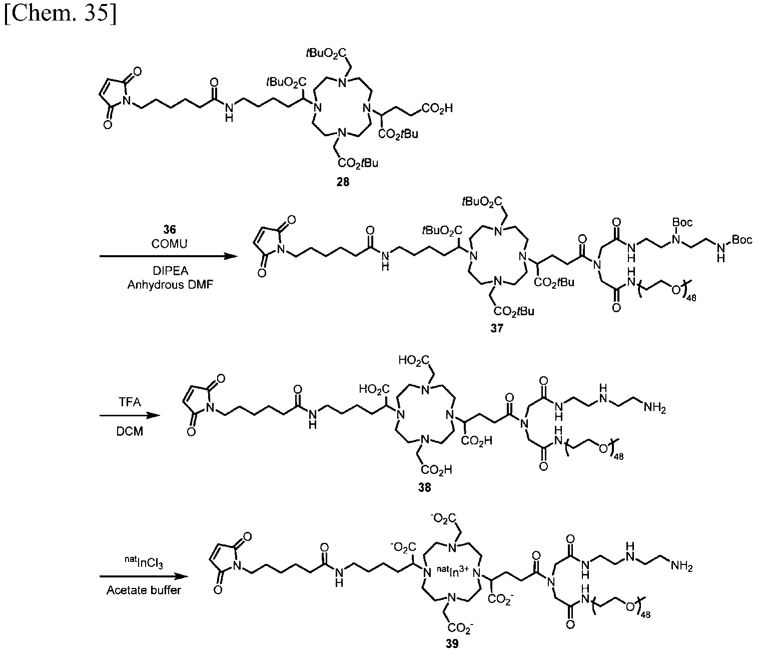

### <Synthesis of Compound 37>

Compound 28 (15 mg, 15 µmol) was dissolved in anhydrous DMF (220 µL), COMU (33 mg, 78 µmol) and DIPEA (10 mg, 78 µmol) were added thereto at 0°C, and the mixture was stirred at 0°C for 15 minutes. Then, Compound 36 (20 mg, 7.8 µmol) was added, and the mixture was stirred at room temperature for 17 hours. The solution was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 1 mL/min) to obtain 11 mg of Compound 37 (yield: 41%). MS (electrospray ionization method) of the obtained Compound 37 showed a measured m/z value of 874.0 (theoretical m/z value: 874.0411 for C₁₆₄H₃₁₅N₁₁O₆₆⁴⁺, [M+4H]⁴⁺).

### <Synthesis of Maleimide-DOTAGL-PEI2-PEG48 (MDPEI2-PEG48) (Compound 38)>

Compound 37 (11 mg, 3.2 µmol) was dissolved in a mixed solution of trifluoroacetic acid (1.6 mL) and dichloromethane (0.4 mL), and the mixture was stirred at room temperature for 14 hours. Thereafter, the solvent was distilled off, and the residue was purified by the reverse phase HPLC using the COSMOSIL C₁₈ column (5C₁₈-AR-II, 4.6 mm I.D. × 150 mm) and a mobile phase (elution conditions: 0.1% trifluoroacetic acid aqueous solution/0.1% trifluoroacetic acid acetonitrile solution = 4/1 (0 min) to 3/2 (20 min), flow rate: 1 mL/min), and then purified using the same column again under the elution conditions: 0.1% trifluoroacetic acid aqueous solution/0.1% trifluoroacetic acid acetonitrile solution = 3/1 (0 min) to 13/7 (20 min) and the flow rate is 1 mL/min to obtain 2.3 mg of Compound 38 (yield: 23%). MS (electrospray ionization method) of the obtained Compound 38 showed a measured m/z value of 776.4 (theoretical m/z value: 776.4655 for C₁₃₈H₂₇₃N₁₃O₆₂⁴⁺, [M+2H+2NH₄]⁴⁺).

### <Synthesis of Maleimide-[^{nat}In]In-DOTAGL-PEI2-PEG48([^{nat}In]In-MDPEI2-PEG48) (Compound 39)>

Compound 38 (0.5 mg, 0.16 µmol) was dissolved in acetate buffer solution (1.0 mol/L, pH 5.1, 100 µL), indium(III) chloride (3.6 mg, 16 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 4/1 (0 min) to 1/1 (30 min), flow rate: 1 mL/min) to obtain 0.14 mg of Compound 39 (yield: 27%). MS (electrospray ionization method) of the obtained Compound 39 showed a measured m/z value of 804.3 (theoretical m/z value: 804.4356 for C₁₃₈H₂₇₀InN₁₃O₆₂⁴⁺, [M+3H+2NH₄]⁴⁺).

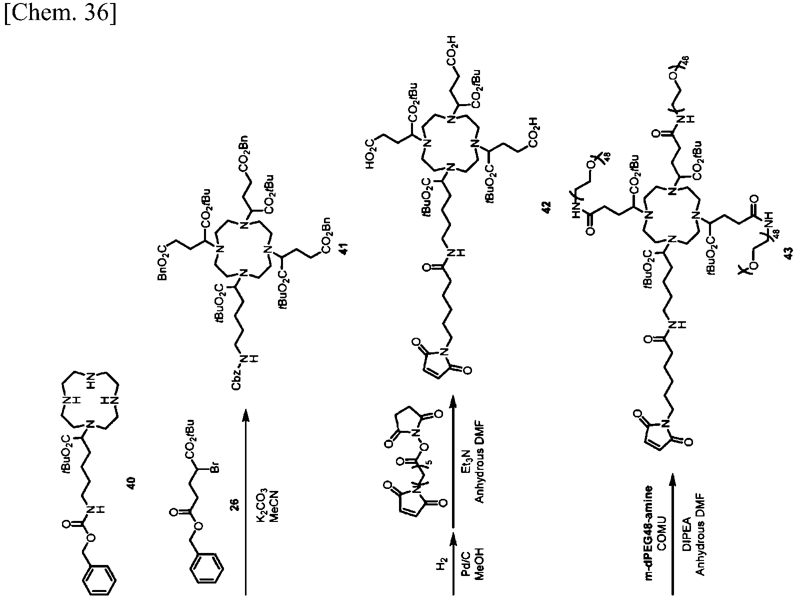

### · Synthesis of Compound 44 and In Complex 45 Thereof (Example 1-6)

### <Synthesis of Compound 40>

Compound 40 was synthesized according to a previous report (Angew. Chem. Int. Ed. Engl., 2017, 56, 9825-9828).

### <Synthesis of Compound 41>

Compound 40 (285 mg, 0.58 mmol) was dissolved in acetonitrile (20 mL), and Compound 26 (1,858 mg, 5.2 mmol) and potassium carbonate (722 mg, 5.2 mmol) were added thereto, and then the mixture was stirred at 50°C for 33 hours. Then, Compound 26 (600 mg, 1.7 mmol) and potassium carbonate (240 mg, 1.7 mmol) were added to the reaction solution, and the mixture was further stirred at 50°C for 10 hours. After the reaction solution was filtered to recover the filtrate, the solvent was distilled off under reduced pressure. The residue was purified by amine silica gel chromatography using a mobile phase (hexane/ethyl acetate = 3/7) to obtain 351 mg of Compound 41 (yield: 46%). MS (electrospray ionization method) of the obtained Compound 41 showed a measured m/z value of 1320.8 (theoretical m/z value: 1320.7630 for C₇₄H₁₀₆N₅O₁₆⁺, [M+H]⁺).

### <Synthesis of Compound 42>

Compound 41 (350 mg, 0.27 mmol) was dissolved in methanol (30 mL), 10% palladium on carbon (140 mg) was added thereto, and the mixture was vigorously stirred at room temperature for 43 hours under a hydrogen gas atmosphere. Subsequently, filtration was performed using a Hyflo Super Cel, and the solvent from the filtrate was distilled off. The residue was dissolved in anhydrous DMF (1.0 mL), 6-maleimidohexanoic acid N-hydroxysuccinimide ester (EMCS) (323 mg, 1.1 mmol) and triethylamine (106 mg, 1.1 mmol) were added thereto, and then the mixture was stirred at room temperature for 9 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min) to obtain 114 mg of Compound 42 (yield: 39%). MS (electrospray ionization method) of the obtained Compound 42 showed a measured m/z value of 1109.6 (theoretical m/z value: 1109.6592 for C₅₅H₉₃N₆O₁₇⁺, [M+H]⁺).

### <Synthesis of Compound 43>

Compound 42 (3.1 mg, 2.8 µmol) was dissolved in anhydrous DMF (200 µL), COMU (12 mg, 28 µmol) and DIPEA (3.6 mg, 28 µmol) were added thereto at 0°C, and the mixture was stirred at 0°C for 15 minutes. Thereafter, m-dPEG48-amine (30 mg, 14 µmol) was added, and the mixture was stirred at room temperature for 24 hours. The solution was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min) to obtain 0.59 mg of Compound 43 (yield: 2.8%). MS (electrospray ionization method) of the obtained Compound 43 showed a measured m/z value of 833.2 (theoretical m/z value: 833.2886 for C₃₄₆H₆₈₆N₉O₁₅₈⁹⁺, [M+9H]⁹⁺).

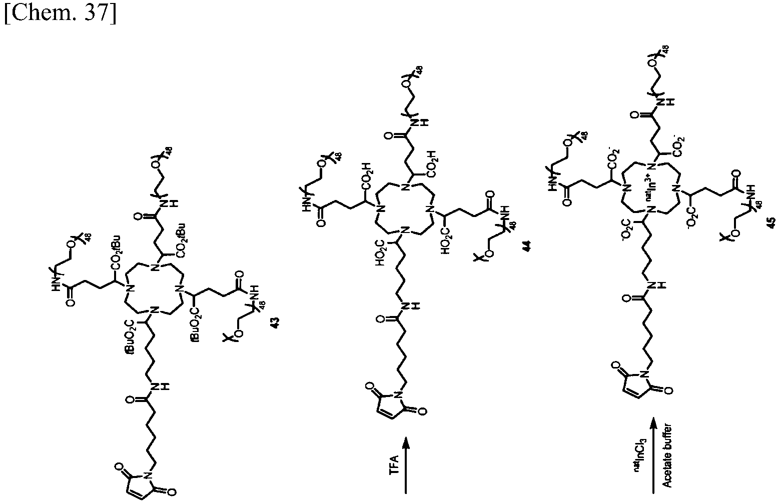

### <Synthesis of Maleimide-DOTA-PEG48-3 (MDPEG48-3) (Compound 44)>

Compound 43 (0.59 mg, 79 nmol) was dissolved in trifluoroacetic acid (2.0 mL), and the mixture was stirred at room temperature for 84 hours. Thereafter, the solvent was distilled off, and the residue was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 4/1 (0 min) to 1/1 (30 min), flow rate: 1 mL/min) to obtain 0.38 mg of Compound 44 (yield: 66%). MS (electrospray ionization method) of the obtained Compound 44 showed a measured m/z value of 808.3 (theoretical m/z value: 808.3719 for C₃₃₀H₆₅₄N₉O₁₅₈⁹⁺, [M+9H]⁹⁺).

### <Synthesis of Maleimide-[^{nat}In]In-DOTA-PEG48-3([^{nat}In]In-MDPEG48-3) (Compound 45)>

Compound 44 (0.46 mg, 63 nmol) was dissolved in acetate buffer solution (1.0 mol/L, pH 5.1, 100 µL), indium(III) chloride (1.4 mg, 6.3 µmol) was added thereto, and then the mixture was heated at 90°C for 30 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 4/1 (0 min) to 1/1 (30 min), flow rate: 1 mL/min) to obtain 0.05 mg of Compound 45 (yield: 11%). MS (electrospray ionization method) of the obtained Compound 45 showed a measured m/z value of 671.7 (theoretical m/z value: 671.7493 for C₃₃₀H₆₅₃InN₉O₁₅₈¹¹⁺, [M+12H]¹¹⁺).

### · Synthesis of Radiolabeled Antibody Drugs (Examples 2-4 to 2-6)

### <Labeling Reaction>

### <First Labeling Step>

Compound 33 (1.0 mg/mL DMSO solution, 4 µL) was dissolved in 0.1 mol/L acetate buffer solution (pH 5.8, 450 µL), the [¹¹¹In]InCl₃ solution (400 µL, 32.3 MBq) was added thereto, and then the mixture was heated at 90°C for 5 minutes. The reaction solution was purified by the reverse phase HPLC using the COSMOSIL C₁₈ column (5C₁₈-AR-II, 4.6 mm I.D. × 150 mm) and a mobile phase (H₂O/MeCN/TFA [80/20/0.1 (0 min) to 50/50/0.1 (30 min)], flow rate: 1.0 mL/min), and the solvent of the fraction containing the target compound was distilled off under an argon gas stream.

Compound 38 (1.0 mg/mL DMSO solution, 15 µL) was dissolved in 0.1 mol/L acetate buffer solution (pH 5.5, 500 µL), the [¹¹¹In]InCl₃ solution (450 µL, 31.5 MBq) was added thereto, and then the mixture was heated at 90°C for 6 minutes. The reaction solution was purified by the reverse phase HPLC using the COSMOSIL C₁₈ column (5C₁₈-AR-II, 4.6 mm I.D. × 150 mm) and a mobile phase (H₂O/MeCN/TFA [75/25/0.1 (0 min) to 55/45/0.1 (20 min)], flow rate: 1.0 mL/min), and the solvent of the fraction containing the target compound was distilled off under an argon gas stream.

Compound 44 (2.0 mg/mL DMSO solution, 55 µL) was dissolved in 0.1 mol/L acetate buffer solution (pH 5.8, 300 µL), the [¹¹¹In]InCl₃ solution (400 µL, 32.4 MBq) was added thereto, and then the mixture was heated at 90°C for 7 minutes. The reaction solution was purified by the reverse phase HPLC using the COSMOSIL C₁₈ column (5C₁₈-AR-II, 4.6 mm I.D. × 150 mm) and a mobile phase (H₂O/MeCN/TFA [80/20/0.1 (0 min) to 50/50/0.1 (30 min)], flow rate: 1.0 mL/min), and the solvent of the fraction containing the target compound was distilled off under an argon gas stream.

In the present specification, radiolabeled compounds, in which Compounds 33, 38, and 44 are coordinated to ¹¹¹In³⁺, are referred to as [¹¹¹In]In-MDPEG24, [¹¹¹In]In-MDPEI2-PEG48, and [¹¹¹In]In-MDPEG48-3, respectively.

### <Second Labeling Step>

After the 0.1 mol/L phosphate buffer solution (pH 7, 500 µL) was added to the residues of [¹¹¹In]In-MDPEG24 and [¹¹¹In]In-MDPEI2-PEG48 obtained by the first labeling step, trastuzumab-SH (0.6 mg) was added thereto, and the mixture was incubated for 9 to 10 hours at room temperature under light-shielded conditions. Thereafter, the reaction solution was purified and concentrated using Amicon Ultra-4 (recovered fraction: 50 kDa) to obtain a target radiolabeled antibody drug. In addition, after the 0.1 mol/L phosphate buffer solution (pH 7, 500 µL) was added to the residue of [¹¹¹In]In-MDPEG48-3 obtained by the first labeling step, trastuzumab-SH (0.4 mg) was added thereto, and the mixture was incubated for 9 hours at room temperature under light-shielded conditions. Thereafter, the reaction solution was concentrated using Amicon Ultra-4 (recovered fraction: 50 kDa), and purified by the size exclusion HPLC using the TSKgel G3000SW_{XL} column (5 µm, 7.8 mm I.D. × 300 mm) and the mobile phase (0.1 mol/L phosphate buffer solution (pH 6.8), flow rate: 0.5 mL/min). The solution of the fraction containing the target compound was concentrated using Amicon Ultra-4 (recovered fraction: 50 kDa) to obtain the target radiolabeled antibody drug. The radiochemical purity (RCP) was measured as follows. Analysis was performed by the size exclusion HPLC using the TSKgel G3000SW_{XL} column (5 µm, 7.8 mm I.D. × 300 mm) and the mobile phase (0.1 mol/L phosphate buffer solution (pH 6.8), flow rate: 0.5 mL/min), and the percentage of the area value of the radiolabeled compound relative to the area values of all detected peaks was defined as RCP (%).

In the present specification, radiolabeled antibody drugs (RIC) obtained by introducing [¹¹¹In]In-MDPEG24, [¹¹¹In]In-MDPEI2-PEG48, and [¹¹¹In]In-MDPEG48-3 into trastuzumab-SH are referred to as [¹¹¹In]In-TNMPEG24, [¹¹¹In]In-TMDPEI2-PEG48, and [¹¹¹In]In-TMDPEG48-3, respectively. The radiochemical yield (RCY) of each labeling step in the two-step labeling process was calculated as the percentage of radioactivity of the purified ¹¹¹In-labeled product relative to the amount of radioactivity added at the beginning of each step. In addition, the overall radiochemical yield (total RCY) was calculated as the percentage of radioactivity of the obtained ¹¹¹In-labeled antibody drug relative to the amount of radioactivity of the [¹¹¹In]InCl₃ solution added during the first labeling step.

In Example 2-4 ([¹¹¹In]In-TMDPEG24), the RCY of the first step was 61.4%, the RCY of the second step was 67.4%, and the overall radiochemical yield was 23.6%.

In Example 2-5 ([¹¹¹In]In-TMDPEI2-PEG48), the RCY of the first step was 24.6%, the RCY of the second step was 33.6%, and the overall radiochemical yield was 5.6%.

In Example 2-6 ([¹¹¹In]In-TMDPEG48-3), the RCY of the first step was 44.4%, the RCY of the second step was 21.5%, the overall radiochemical yield was 8.3%, and each antibody drug was synthesized via a two-step labeling reaction. The radiochemical purity of all ¹¹¹In-labeled antibody drugs was greater than 95%.

### <Evaluation of Immunoreactivity> (Examples 3-4 to 3-6)

The immunoreactive fraction of each radiolabeled antibody drug was calculated in the same manner as in Examples 3-1 to 3-3. The results are as follows.

The result of Example 3-4 ([¹¹¹In]In-TMDPEG24) was 0.98 ± 0.09, the result of Example 3-5 ([¹¹¹In]In-TMDPEI2-PEG48) was 0.84 ± 0.01, and the result of Example 3-6 ([¹¹¹In]In-TMDPEG48-3) was 0.86 ± 0.03. Each drug showed good HER2 binding ability, and no decrease in target binding ability of the antibody was observed through the two-step labeling reaction.

### <Cell Internalization Experiment> (Examples 4-4 to 4-6)

A cell internalization experiment was performed in the same manner as in Examples 4-1 to 4-3.

The radioactivity of the dissociated fraction and the degraded fraction after 24 hours was measured in the same manner as in Example 4, and the results of the percentage of radioactivity of the medium fraction and the internalized fraction are shown in Tables 19 to 21 for each radiolabeled antibody drug. For each of the radiolabeled antibody drugs, the ratio of the radioactivity of the recovered fraction to the total radioactivity bound to the cells was defined as the percentage of radioactivity. For the internalized fraction, all of TMDPEG24, TMDPEI2-PEG48, and TMDPEG48-3 after 24 hour incubation showed high values, indicating high intracellular retention. For the medium fraction, each radiolabeled antibody drug showed comparable values. In addition, regarding the ratio obtained by dividing the amount of radioactivity of the internalized fraction by the amount of radioactivity of the medium fraction, no significant difference was observed between the respective radiolabeled antibody drugs, and it was shown that all the radiolabeled antibody drugs have a strong cell retention tendency.

Regarding the percentage of radioactivity of the dissociated fraction and the degraded fraction, a tendency was observed for the amount of radiolabeled antibody drug dissociated from the cells to increase with an increase in molecular weight. Meanwhile, a tendency was observed for the amount of radioactivity derived from the metabolized radiolabeled antibody drug to decrease with an increase in molecular weight. In addition, a tendency was observed for the ratio of the internalization rate at 24 hours after incubation to the proportion of metabolites that flowed out of cells to be higher for radiolabeled antibody drugs with larger molecular weights. The above results suggest that the increase in molecular weight promotes dissociation of the radiolabeled antibody drug from the cell surface while enhancing radioactivity retention, and that a combination of a functional unit having a positive charge and a functional unit having a high molecular weight or a combination of a plurality of high molecular weight functional units does not exhibit a synergistic effect.

**[Table 19]**

| **[¹¹¹In]In-TMDPEG24** | | Incubation time (h) | | | | |
|---|---|---|---|---|---|---|
| | | **0** | **1** | **2** | **4** | **24** |
| % of total bound radioactivity | Medium fraction | 5.5 ± 0.6 | 8.3 ± 0.2 | 11.6 ± 2.0 | 11.4 ± 1.1 | 19.6 ± 2.1 |
| | Cell surface fraction | 91.7 ± 0.8 | 85.4 ± 1.2 | 76.2 ± 6.2 | 73.6 ± 1.2 | 53.1 ± 3.7 |
| | Cell internalized fraction | 2.8 ± 0.4 | 6.3 ± 1.4 | 12.1 ± 4.2 | 15.0 ± 1.0 | 27.3 ± 1.7 |
| | Dissociated fraction | N.D. | N.D. | N.D. | N.D. | 7.2 ± 0.7 |
| | Degraded fraction | N.D. | N.D. | N.D. | N.D. | 12.5 ± 1.8 |
| Ratio | Cell internalized fraction/Medium fraction | 0.51 ± 0.08 | 0.76 ± 0.17 | 1.02 ± 0.17 | 1.32 ± 0.18 | 1.39 ± 0.06 |
| | Cell internalized fraction/ Degraded fraction | N.D. | N.D. | N.D. | N.D. | 2.20 ± 0.22 |

**[Table 20]**

| [**¹¹¹In]In-TMDPEI2-PEG48** | | Incubation time (h) | | | | |
|---|---|---|---|---|---|---|
| | | **0** | **1** | **2** | **4** | **24** |
| % of total bound radioactivity | Medium fraction | 7.1 ± 0.8 | 12.3 ± 1.1 | 11.6 ± 1.3 | 11.7 ± 1.7 | 16.8 ± 0.1 |
| | Cell surface fraction | 87.9 ± 0.8 | 75.2 ± 8.3 | 79.1 ± 1.2 | 78.2 ± 1.4 | 61.0 ± 2.8 |
| | Cell internalized fraction | 5.0 ± 0.8 | 12.5 ± 7.6 | 9.3 ± 2.3 | 10.1 ± 0.5 | 22.2 ± 2.7 |
| | Dissociated fraction | N.D. | N.D. | N.D. | N.D. | 9.4 ± 0.6 |
| | Degraded fraction | N.D. | N.D. | N.D. | N.D. | 7.2 ± 0.4 |
| Ratio | Cell internalized fraction/Medium fraction | 0.72 ± 0.17 | 1.00 ± 0.55 | 0.81 ± 0.28 | 0.88 ± 0.16 | 1.32 ± 0.16 |
| | Cell internalized fraction/ Degraded fraction | N.D. | N.D. | N.D. | N.D. | 3.00 ± 0.47 |

**[Table 21]**

| **[¹¹¹In]In-TMDPEG48-3** | | Incubation time (h) | | | | |
|---|---|---|---|---|---|---|
| | | **0** | **1** | **2** | **4** | **24** |
| % of total bound radioactivity | Medium fraction | 5.4 ± 0.3 | 9.2 ± 0.7 | 9.8 ± 0.5 | 9.5 ± 1.8 | 17.5 ± 0.5 |
| | Cell surface fraction | 93.7 ± 0.1 | 81.4 ± 1.9 | 76.8 ± 0.5 | 73.3 ± 3.2 | 50.9 ± 2.0 |
| | Cell internalized fraction | 0.9 ± 0.2 | 9.4± 1.2 | 13.4 ± 0.4 | 17.2 ± 1.7 | 31.6 ± 1.6 |
| | Dissociated fraction | N.D. | N.D. | N.D. | N.D. | 11.7 ± 0.6 |
| | Degraded fraction | N.D. | N.D. | N.D. | N.D. | 5.8 ± 0.1 |
| Ratio | Cell internalized fraction/Medium fraction | 0.18 ± 0.06 | 1.02 ± 0.05 | 1.37 ± 0.09 | 1.84 ± 0.31 | 1.80 ± 0.05 |
| | Cell internalized fraction/ Degraded fraction | N.D. | N.D. | N.D. | N.D. | 5.41 ± 0.32 |

### <In Vivo Radioactivity Distribution Experiment Using Tumor Transplantation Model Mice> (Examples 5-4 to 5-6)

Model mice were prepared by the same method as in Examples 5-1 to 5-3, and an in vivo radioactivity distribution experiment was conducted. The administered radioactivity was [¹¹¹In]In-TMDPEG24 (148 kBq, 18 µg), [¹¹¹In]In-MDPEI2-PEG48(111kBq, 18µg), and [¹¹¹In]In-TMDPEG48-3(148kBq, 18µg), respectively. These results are shown in Tables 22 to 24, categorized by the radiolabeled antibody drug administered to the mice.

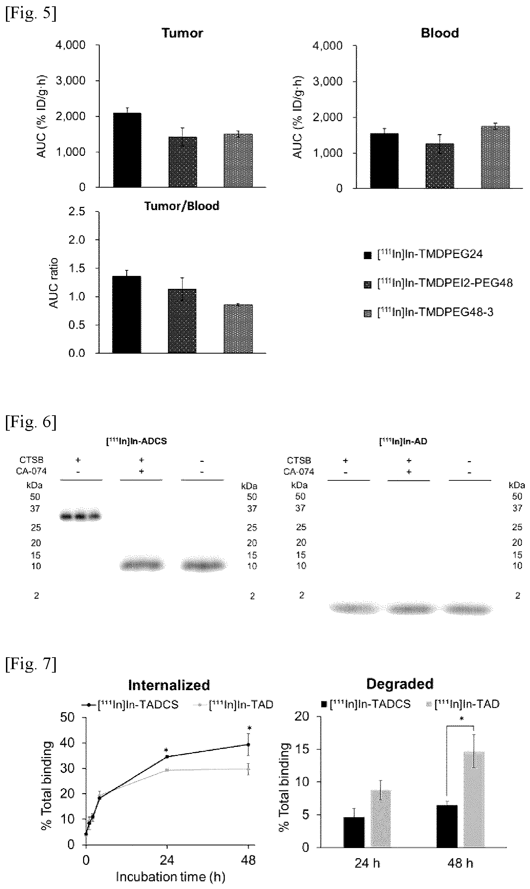

In addition, the area under the curve (AUC) of blood and tumor was calculated using GraphPad Prism software based on the results of the in vivo radioactivity distribution experiment without decay correction. The results are shown in Fig. 5.

**[Table 22]**

| **[¹¹¹In]In-TMDPEG24** | Tissue | Time after administration (h) | | | | | |
|---|---|---|---|---|---|---|---|
| | | **4** | **24** | **48** | **72** | **96** | **192** |
| | Blood | 31.49 (1,29) | 21.07 (0.33) | 16.34 (3.32) | 14.73 (1.37) | 11.54 (2.85) | 7.60 (1.79) |
| | Spleen | 9.11 (3.38) | 6.60 (1.54) | 6.15 (1.96) | 5.47 (0.62) | 5.85 (0.66) | 4.77 |
| | Pancreas | 2.36 (0.12) | 2.31 (0.43) | 3.22 (1.19) | 2.11 (0.37) | 2.38 | 1.52 (0.31) |
| | Stomach (%ID) | 0.31 (0.03) | 0.36 (0.07) | 0.56 (0.23) | 0.39 (0.05) | 0.28 (0.04) | 0.24 (0.09) |
| | Intestine | 2.97 (0.37) | 2.06 (0.23) | 2.07 (0.41) | 1.84 (0.19) | 1.44 (0.18) | 1.11 (0.12) |
| % ID/g | Kidney | 10.62 (0.82) | 8.49 (0.31) | 7.51 (1.37) | 6.61 (0.36) | (0.72) 5.90 (0.73) (0.61) 29.87 | (1.34) 4.65 (1.04) |
| | Liver | 9.73 (1.28) | 6.82 (0.78) | 7.06 (0.89) | 4.64 (0.66) | 8.16 (4.59) | 5.79 (0.40) |
| | Heart | 6.22 (0.45) | 3.93 (0.38) | 3.39 (0.69) | 2.61 (0.24) | 2.84 | 1.93 (0.12) |
| | Lung | 16.14 (1.01) | 10.74 (1.38) | 9.60 (1.72) | 7.78 (0.70) | 7.74 (1.49) | 5.21 (1.07) |
| | Brain | 0.63 (0.16) | 0.44 (0.03) | 0.41 (0.06) | 0.33 (0.01) | 0.29 (0.04) | 0.19 (0.04) |
| | Tumor | 3.77 (1.98) | 22.15 (4.90) | 32.89 (7.43) | 25.68 (4.60) | (7.43) | 34.29 (7.39) |
| | Muscle | 0.94 (0.33) | 1.27 (0.19) | 1.19(0.07) | 1.16 (0.39) | 0.95 (0.29) | 0.69 (0.24) |
| Ratio | Tumor/Blood | 0.12 (0.07) | 1.05 (0.24) | 2.05(0.43) | 1.75 (0.30) | 2.64 (0.53) | 4.54 (0.39) |
| | Tumor/Muscle | 5.38 (5.65) | 17.54 (3.93) | 27.41 (4.95) | 23.75 (6.86) | 33.00 (7.79) | 54.22 (22.99) |

**[Table 23]**

| **[¹¹¹In]In-TMDPEI2-PEG48** | Tissue | Time after administration (h) | | | | | |
|---|---|---|---|---|---|---|---|
| | | **4** | **24** | **48** | **72** | **96** | **192** |
| | Blood | 28.59 (2.21) | 16.15(3.06) | 14.15 (1.88) | 10.47 (2.40) | 8.74 (1.13) | 3.85 (0.88) |
| | Spleen | 5.16 (0.50) | 4.63 (2.17) | 4.25 (0.77) | 4.03 (0.82) | 3.75 (0.19) | 3.80 |
| | Pancreas | 3.97 (0.98) | 5.77 (2,12) | 4.49 (0.66) | 4.50 (1.21) | 3.37 (0.50) | 2.22 (0.84) |
| | Stomach (%ID) | 0.39 (0.10) | 0.38 (0.05) | 0.47 (0.07) | 0.33 (0.10) | 0.34 (0.07) | 0.19 (0.07) |
| | Intestine | 2.38 (0.22) | 1.87 (0.48) | 1.61 (0.32) | 1.50 (0.07) | 1.24 (0.14) | 0.81 (0.11) |
| % ID/g | Kidney | 6.80(1.01) | 5.00 (1.15) 8.36 | 4.47 (0.89) | 4.11 (0.67) | 3.54 (0.15) | (0.32) 1.89 (0.27) |
| | Liver | 8.38 (0.74) | (2.59) | 8.09 (1.57) | 8.49 (1.82) | 6.39 (0.72) | 5.10 (0.82) |
| | Heart | 5.61 (0.12) | 3.92 (0.84) | 3.15 (0.50) (1.12) | 2.70 (0.59) | 2.25 (0.22) | 1.27 (0.12) |
| | Lung | 10.65 (2.27) | 8.15 (2.07) | 7.61 | 6.38 (1.47) | 4.76 (0.88) | 2.81 (0.25) |
| | Brain | 0.58 (0.04) | 0.47 (0.14) | 0.35 (0.09) | 0.33 (0.06) | 0.23 (0.05) | 0.12 (0.06) |
| | Tumor | 3.79(2.06) | 12.33 (2.07) | 18.93 (2.01) | 22.00 (7.36) | 23.29 (7.31) | 17.12 (1.37) |
| | Muscle | 0.72(0.12) | 1.31 (0.32) | 1,41 (0.29) | 0.92 (0.14) | 0.89 (0.09) | 0.57 (0.06) |
| Ratio | Tumor/Blood | 0.14 (0.08) | 0.79 (0.22) | 1.35 (0.14) | 2.08 (0.40) | 2.71 (1.03) | 4.68 (1.43) |
| | Tumor/Muscle | 5.35 (3.02) | 9.95 (3.56) | 13.84 (2.88) | 24.85 (11.24) | 26.93 (10.35) | 30.35 (5.72) |

**[Table 24]**

| \|^{III}In\|In **TMDPEG48-3** | Tissue | Time after administration (h) | | | | | |
|---|---|---|---|---|---|---|---|
| | | **4** | **24** | **48** | **72** | **96** | **192** |
| | Blood | 38.28 (3.06) | 24.36 (2.53) | 18.36 (0.64) | 18.55 (0.98) | 12.41 (4.67) | 7.26 (1.89) |
| | Spleen | 5.74 (0.38) | 6.40 (1.43) | 5.96 (0.58) | 5.46 (0.57) | 6.45 (0.93) | 5.30 (1.82) |
| | Pancreas | 2.00 (0.37) | 4.48 (0.71) | 4.08 (1.62) | 2.78 (0.84) | 3,97 (0.18) | 1.98 (0.92) |
| | Stomach (%ID) | 046 (0.09) | 0.45 (0.10) | 0.46 (0.10) | 0.37 (0.12) | 0.40 (0.08) | 0.26 (0.09) |
| | Intestine | 3.28 (0.43) | 2.44 (0.35) | 2.05 (0.24) | 1.83 (0.07) | 1.70 (0.15) | 1.14 (0.26) |
| % ID/g | Kidney | 6.75 (0.55) | 5.66 (0.70) | 4.40 (0.36) | 4.39 (0.30) (0.70) | 3.55 (0.83) | 2.93 (0.58) |
| | Liver | 7.96 (0.63) | 10.31 (2.03) | 8.92 (0.93) | 7.68 (0.98) | 8.69 (1.86) | 7.69 (2.13) |
| | Heart | 5.18 (0.48) | 5.45 (0.58) | 4.16 (0.39) | 3.50 (0.22) | 3.24 (0.28) | 2.24 (0.32) |
| | Lung | 19.36 (2.74) | 12.41 (1.70) | 10.34 (1.13) | 9.39 | 7.02 (2.19) | 5.17 (1.39) |
| | Brain | 0.74 (0.14) | 0.54 (0.06) | 0.43 (0.04) | 0.42 (0.06) | 0.30 (0.07) | 0.19 (0.05) |
| | Tumor | 2.57 (0.77) | 10.13 (2.17) | 17.75 (1.42) | 32.32 (7.06) | 21.97 (5.12) | 30.29 (7.36) |
| | Muscle | 0.83 (0.23) | 1.41 (0.31) | 1.09 (0.15) | 1.23 (0.07) | 0.90 (0.25) | 0.69 (0.20) |
| Ratio | Tumor/Blood | 0.07 (0.02) | 0.42 (0.08) | 0.97 (0.06) | 1.75 (0.41) | 1.87 (0.40) | 4,19 (0,45) |
| | Tumor/Muscle | 3.10 (0.08) | 7.24 (1.13) | 16.43 (2.24) | 26.59 (7.41) | 24.58 (1.39) | 44.82 (4.72) |

As shown in Tables 22 to 24, regarding the radioactivity uptake of the tumor, all the ¹¹¹In-labeled antibodies reached the maximum values at the late stage of administration, the [¹¹¹In]In-TMDPEG24 showed 34.3 ± 7.4%ID/g at 192 hours after administration, the [¹¹¹In]In-MDPEI2-PEG48 showed 23.3 ± 3.3%ID/g at 96 hours after administration, and the [¹¹¹In]In-TMDPEG48-3 showed 32.3 ± 7.1%ID/g at 72 hours after administration. In addition, regarding the amount of radioactivity in the blood, at 24 hours after administration, the [¹¹¹In]In-TMDPEG24 showed 21.1 ± 0.3%ID/g and the [¹¹¹In]In-TMDPEG48-3 showed 24.4 ± 2.5%ID/g, whereas the [¹¹¹In]In-TMDPEI2-PEG48 showed 16.2 ± 3.1%ID/g.

As shown in Fig. 5, regarding the AUC in the tumor, all the radiolabeled antibody drugs showed the same value as [¹¹¹In]In-TMD. Regarding the AUC in blood, [¹¹¹In]In-TMDPEI2-PEG48 showed a slightly lower value compared to the other two RICs. The tumor-to-blood ratio for any RIC did not show a value exceeding the [¹¹¹In]In-TMDPEG48, and the order from higher RIC was [¹¹¹In]In-TMDPEG24, [¹¹¹In]In-TMDPEI2-PEG48, and [¹¹¹In]In-TMDPEG48-3.

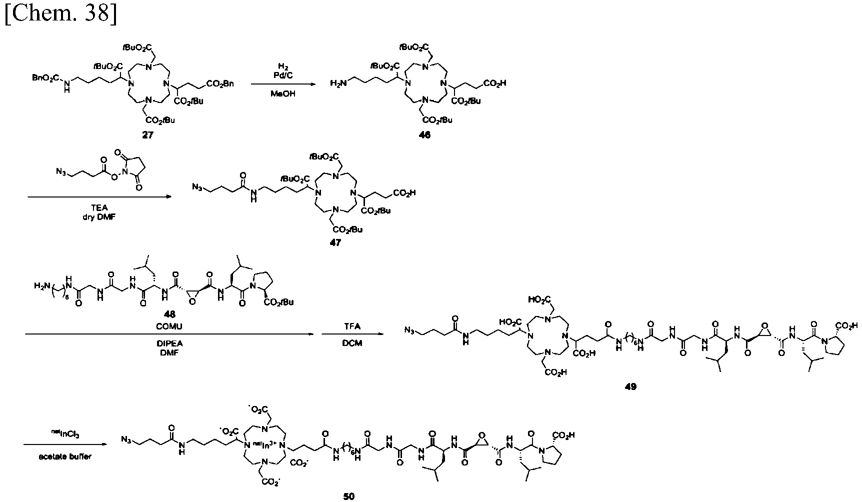

### · Synthesis of Compound 49 and In Complex 50 Thereof (Example 1-7)

### <Synthesis of Compound 46>

Compound 27 (1.25 g, 1.26 mmol) was dissolved in methanol (12 mL), 10% palladium on carbon (624 mg) was added thereto, and the mixture was vigorously stirred at room temperature for 20 hours under a hydrogen gas atmosphere. Subsequently, filtration was performed using a Hyflo Super Cel, and the solvent from the filtrate was distilled off. The residue was purified by the reverse phase HPLC using the COSMOSIL C₁₈ column (5C₁₈-AR-II, 10 mm I.D. × 250 mm) and the mobile phase (elution conditions: 0.1% trifluoroacetic acid aqueous solution/0.1% trifluoroacetic acid acetonitrile solution = 9/1 (0 min) to 3/7 (30 min), flow rate: 4 mL/min) to obtain 200 mg of Compound 46 (yield: 21%). HRMS (electrospray ionization method) of the obtained Compound 46 showed a measured m/z value of 772.5426 (theoretical m/z value: 772.5431 for C₃₉H₇₄N₅O₁₀⁺, [M+H]⁺). The identification data of the obtained Compound 46 by NMR is shown below. ¹H NMR(400MHz, CD₃OD)δ4.53-2.55(m, 26H), 1.86-1.25(m, 44H).

### <Synthesis of Compound 47>

Compound 46 (200 mg, 0.26 mmol) was dissolved in anhydrous DMF (1 mL), succinimidyl 4-azidobutyrate (88 mg, 0.39 mmol) and triethylamine (52 mg, 0.52 mmol) were added thereto, and the mixture was stirred at room temperature for 7 hours. The solvent was distilled off, and the residue was purified by the reverse phase HPLC using the COSMOSIL C₁₈ column (5C₁₈-AR-II, 10 mm I.D. × 250 mm) and a mobile phase (elution conditions: 0.1% trifluoroacetic acid aqueous solution/0.1% trifluoroacetic acid acetonitrile solution = 7/3 (0 min) to 1/9 (30 min), flow rate: 4 mL/min) to obtain 84 mg of Compound 47 (yield: 37%). HRMS (electrospray ionization method) of the obtained Compound 47 showed a measured m/z value of 883.5862 (theoretical m/z value: 883.5863 for C₄₃H₇₉N₈O₁₁⁺, [M+H]⁺). The identification data of the obtained Compound 47 by NMR is shown below. ¹HNMR(400MHz, DMSO-d₆)δ4.63-2.40(m, 28H), 2.16-2.07(m, 2H), 1.82-1.27(m, 46H).

### <Synthesis of Compound 48>

Compound 48 was synthesized according to a previous report (FEBS Lett., 2000, 482, 91-96).

### <Synthesis of Azide-DOTA-Cathepsin B-Substrate (ADCS) (Compound 49)>

Compound 47 (6.9 mg, 9.0 µmol) was dissolved in anhydrous DMF (150 µL), COMU (7.6 mg, 27 µmol) and DIPEA (3.5 mg, 27 µmol) were added thereto at 0°C, and the mixture was stirred at 0°C for 20 minutes. Then, Compound 48 (6.5 mg, 9.0 µmol) was added, and the mixture was stirred at room temperature for 24 hours. The solvent was distilled off, and the residue was dissolved in a mixed solution of trifluoroacetic acid (1.6 mL) and dichloromethane (400 µL) and stirred at room temperature for 24 hours. Thereafter, the solvent was distilled off, and the residue was purified by the reverse phase HPLC using the COSMOSIL PBr column (3PBr, 4.6 mm I.D. × 150 mm) and a mobile phase (elution conditions: 0.01% trifluoroacetic acid aqueous solution/0.01% trifluoroacetic acid acetonitrile solution = 9/1 (0 min) to 1/9 (40 min), flow rate: 1 mL/min) to obtain 0.50 mg of Compound 49 (yield: 4.2%). HRMS (electrospray ionization method) of the obtained Compound 49 showed a measured m/z value of 1308.7157 (theoretical m/z value: 1308.7158 for C₅₈H₉₈N₁₅O₁₉⁺, [M+H]⁺).

### <Synthesis of Azide-[^{nat}In]In-DOTA-Cathepsin B-Substrate ([^{nat}In]In-ADCS) (Compound 50)>

Compound 49 (0.20 mg, 0.15 µmol) was dissolved in acetate buffer solution (1.0 mol/L, pH 5.2, 150 µL), indium(III) chloride (0.34 mg, 1.5 µmol) was added thereto, and then the mixture was heated at 90°C for 20 minutes. Thereafter, the reaction solution was centrifuged to remove insoluble matters. The supernatant was purified by the reverse phase HPLC using the COSMOSIL PBr column (3PBr, 4.6 mm I.D. × 150 mm) and the mobile phase (elution conditions: 0.01% trifluoroacetic acid aqueous solution/0.01% trifluoroacetic acid acetonitrile solution = 9/1 (0 min) to 1/9 (40 min), flow rate: 1 mL/min) to obtain 0.20 mg of Compound 50 (yield: 94%). HRMS (electrospray ionization method) of the obtained Compound 50 showed a measured m/z value of 1420.5953 (theoretical m/z value: 1420.5962 for C₅₈H₉₈InN₁₅O₁₉⁺, [M+2H]⁺).

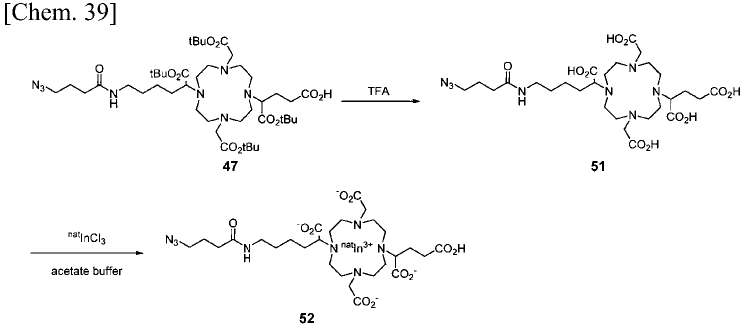

### Synthesis of Compound 51 and In Complex 52 Thereof (Comparative Example 1-4)

### <Synthesis of Azide-DOTA (AD) (Compound 51)>

Compound 47 (3.6 mg, 4.0 µmol) was dissolved in trifluoroacetic acid (1 mL), and the mixture was stirred at room temperature for 71 hours. Thereafter, the solvent was distilled off, and the residue was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 1/9 (40 min), flow rate: 1 mL/min) to obtain 0.44 mg of Compound 51 (yield: 16%). HRMS (electrospray ionization method) of the obtained Compound 51 showed a measured m/z value of 659.3358 (theoretical m/z value: 659.3359 for C₂₇H₄₇N₈O₁₁⁺, [M+H]⁺). The identification data of the obtained Compound 51 by NMR is shown below. ¹HNMR(500MHz, CD₃CN)δ7.93(s, 1H), 3.3(t, J=13.5Hz, 2H), 2.90-2.39(m, 16H), 2.22-2.15(m, 2H), 2.08-2.05(m, 2H), 1.94-1.62(m, 16H), 1.52-1.39(m, 2H).

### <Synthesis of Azide-[^{nat}In]In-DOTA ([^{nat}In]In-AD) (Compound 52)>

Compound 51 (0.40 mg, 0.61 µmol) was dissolved in acetate buffer solution (1.0 mol/L, pH 5.2, 200 µL), indium(III) chloride (1.3 mg, 6.1 µmol) was added thereto, and then the mixture was heated at 90°C for 20 minutes. Thereafter, the reaction solution was centrifuged to remove insoluble matters. The supernatant was purified by the reverse phase HPLC using the COSMOSIL C₁₈ column (5C₁₈-AR-II, 4.6 mm I.D. × 150 mm) and the mobile phase (elution conditions: 0.1% trifluoroacetic acid aqueous solution/0.1% trifluoroacetic acid acetonitrile solution = 9/1 (0 min) to 1/9 (40 min)) to obtain 0.26 mg of Compound 52 (yield: 55%). HRMS (electrospray ionization method) of the obtained Compound 52 showed a measured m/z value of 771.2162 (theoretical m/z value: 771.2163 for C₂₇H₄₄InN₈O₁₁⁺, [M+2H]⁺).

### · Synthesis of Radiolabeled Antibody Drugs (Example 2-7 and Comparative Example 2-4)

### <Introduction of Dibenzocyclooctyne (DBCO) Group to Trastuzumab>

Herceptin (4.0 mg) (manufactured by Roche) was dissolved in PBS (4 mL), and purified and concentrated using Amicon Ultra-4 (recovered fraction: 30 kDa) manufactured by Millipore. A DMSO solution (6.76 µL, 67.6 nmol) of DBCO-PEG4-NHS ester and PBS (78.2 µL) were added to a PBS solution (1.0 mg, 50.2 µL, 6.8 nmol) of trastuzumab as a concentrated solution, and the mixture was incubated under light-shielded conditions for 1 hour. The reaction solution was purified using Zeba Spin Desalting Column (40K MWCO, 0.5 mL), and the obtained filtrate was purified again with Amicon Ultra-4 (recovered fraction: 50 kDa), and then purified again using a Zeba Spin Desalting Column (40K MWCO, 0.5 mL), to obtain trastuzumab into which a DBCO group had been introduced (trastuzumab-DBCO). The purified trastuzumab-DBCO was promptly used for the labeling reaction.

The number of DBCO groups introduced per antibody molecule was calculated according to a previous report (Bioconjug. Chem., 2016, 27, 217-225). The absorbance of the Trastuzumab-DBCO solution at 280 nm and 309 nm was measured, and the concentration of each of trastuzumab and DBCO was calculated assuming that the molar extinction coefficient of trastuzumab at 280 nm was 218 mmol/L⁻¹cm⁻¹ and the molar extinction coefficient of DBCO at 309 nm was 12 mmol/L⁻¹cm⁻¹. The number of DBCO groups introduced per antibody was calculated by dividing the DBCO concentration by the antibody concentration. The number of DBCO groups introduced per antibody was 4.3 ± 0.3.

### <Labeling Reaction>

### <First Labeling Step>

Compounds 49 and 51 (1.0 mg/mL DMSO solution, 1 to 2 µL) were each dissolved in 0.1 mol/L acetate buffer solution (pH 5.9, 80 µL), the [¹¹¹In]InCl₃ solution (70 µL, 5.0 MBq) was added thereto, and then the mixture was heated at 90°C for 20 minutes. The reaction solution was purified by the reverse phase HPLC using the COSMOSIL C₁₈ column (5C₁₈-AR-II, 4.6 mm I.D. × 150 mm) and a mobile phase (elution conditions of reaction solution of Compound 49: H₂O/MeCN/TFA [80/20/0.1 (0 min) to 50/50/0.1 (30 min)], elution conditions of reaction solution of Compound 51: H₂O/MeCN/TFA[92/8/0.1], flow rate: 1.0 mL/min), and the solvent of the fraction containing the target compound was distilled off under an argon gas stream.

In the present specification, radiolabeled compounds, in which Compounds 49 and 51 are coordinated to ¹¹¹In³⁺, are referred to as [¹¹¹In]In-ADCS and [¹¹¹In]In-AD, respectively.

### <Second Labeling Step>

After a 0.1 mol/L phosphate buffer solution (pH 6.7, 300 µL) was added to the residue obtained by the first labeling step, trastuzumab-DBCO (0.6 mg) was added thereto, and the mixture was incubated for 12 hours at room temperature under light-shielded conditions. Thereafter, the reaction solution was purified and concentrated using Amicon Ultra-4 (recovered fraction: 50 kDa) to obtain a target radiolabeled antibody drug. The radiochemical purity (RCP) was measured as follows. Analysis was performed by the size exclusion HPLC using the TSKgel G3000SW_{XL} column (5 µm, 7.8 mm I.D. × 300 mm) and the mobile phase (0.1 mol/L phosphate buffer solution (pH 6.8), flow rate: 0.5 mL/min), and the percentage of the area value of the radiolabeled compound relative to the area values of all detected peaks was defined as RCP (%).

In the present specification, RICs obtained by introducing [¹¹¹In]In-ADCS and [¹¹¹In]In-AD into trastuzumab-DBCO are referred to as [¹¹¹In]In-TADCS and [¹¹¹In]In-TAD, respectively. The radiochemical yield (RCY) of each labeling step in the two-step labeling process was calculated as the percentage of radioactivity of the purified ¹¹¹In-labeled product relative to the amount of radioactivity added at the beginning of each step. In addition, the overall radiochemical yield was calculated as the percentage of radioactivity of the obtained ¹¹¹In-labeled antibody drug relative to the amount of radioactivity of the [¹¹¹In]InCl₃ solution added during the first labeling step.

In Example 2-7 ([¹¹¹In]In-TADCS), the RCY of the first step was 71.6%, the RCY of the second step was 45.1%, and the overall radiochemical yield was 32.3%.

In Comparative Example 2-8 ([¹¹¹In]In-TAD), the RCY of the first step was 78.5%, the RCY of the second step was 56.9%, and the overall radiochemical yield was 43.0%.

Each antibody drug was synthesized via a two-step labeling reaction. The radiochemical purity of all ¹¹¹In-labeled antibody drugs was greater than 95%.

### <Stability Evaluation In Plasma> (Example 3-7)

A PBS solution (740 kBq, 50 µL) of [¹¹¹In]In-ADCS was added to mouse plasma (200 µL), and the mixture was incubated at 37°C for 0, 24, or 96 hours. Subsequently, MeCN (250 µL) was added, the suspension was centrifuged at 1,500 × g for 5 min, and the supernatant was filtered using a Cosmonice Filter S (0.45 µm, 4 mm). The solvent in the filtrate was distilled off under an argon gas stream, and the radiochemical purity of the residue was analyzed by the reverse phase HPLC. The reverse phase HPLC was performed using the COSMOSIL C₁₈ column (5C₁₃-AR-II, 4.6 mm I.D. × 150 mm) and a mobile phase (H₂O/MeCN/TFA = 80/20/0.1 (0 min) to 50/50/0.1 (30 min), flow rate: 1.0 mL/min).

The radiochemical purity of [¹¹¹In]In-ADCS at each time point was measured to be 99.0 ± 0.7% at 0 hours, 99.9 ± 0.2% at 24 hours, and 97.5 ± 0.6% at the 96 hours. This radiochemical purity can be regarded as the stability of [¹¹¹In]In-ADCS. That is, it was shown that [¹¹¹In]In-ADCS maintained a radiochemical purity of 95% or more up to 96 hours, and had high stability in mouse plasma.

### <Cathepsin B Binding Experiment> (Example 4-7 and Comparative Example 4-4)

The cathepsin B binding experiment was generally performed according to a previous report (J. Nucl. Med., 2020, 61, 443-450).

As buffers used for the experiment, a storing buffer (pH 6.0) which is an aqueous solution containing sodium acetate at a final concentration of 50 mmol/L and ethylenediaminetetraacetic acid at 1.0 mmol/L, and a binding assay buffer (pH 5.5) which is an aqueous solution containing trishydroxyaminomethane at a final concentration of 5.0 mmol/L, magnesium chloride at 5.0 mmol/L, and dithiothreitol at 2.0 mmol/L were prepared. A storing buffer solution (600 nM, 10 µL) of cathepsin B derived from human liver was added to a storing buffer (10 µL) containing CA-074 (2 mM) or not containing CA-074 which is a representative inhibitor of cathepsin B, and the mixture was pre-incubated at room temperature for 30 minutes. Thereafter, a binding assay buffer solution of [¹¹¹In]In-ADCS or [¹¹¹In]In-AD (111 kBq, 30 µL) was added thereto. In addition, a cathepsin B non-treated group, in which a storing buffer (20 µL) was added to a binding assay buffer solution (111 kBq, 30 µL) of [¹¹¹In]In-ADCS or [¹¹¹In]In-AD, was also prepared. After incubating these at 37°C for 2 hours, Laemmli sample buffer (2X) (50 µL) was added thereto, and the mixture was further incubated at room temperature for 10 minutes. Each reaction solution (15 µL) and Precision Plus Protein^{™} Dual Xtra Standards (15 µL) as the molecular weight marker were injected into a polyacrylamide gel, and polyacrylamide gel electrophoresis (SDS-PAGE) was performed at 40 mA for 80 minutes. After completion of electrophoresis, the gel was removed, and the [¹¹¹In]InCl₃ solution (1 to 2 kBq, 0.5 µL) was added dropwise to the position of each band of the molecular weight marker. The removed gel was brought into contact with the imaging plate under light-shielded conditions for 20 minutes, and then autoradiographic imaging was performed using Amersham Typhoon scanner.

The imaging results of autoradiography are shown in Fig. 6. In Fig. 6, CTSB refers to cathepsin B. In the group incubated with [¹¹¹In]In-ADCS, a band of RI was observed around 30 kDa only in the CA-074 non-treated group. In the CA-074-treated group, a band was observed around the 10 kDa position, which differed from the molecular weight of [¹¹¹In]In-ADCS (approximately 1.4 kDa). However, since bands were also detected at the same position in the cathepsin B non-treated group, these bands are considered to be derived from unbound [¹¹¹In]In-ADCS. For [¹¹¹In]In-AD, bands were observed at or below the 2 kDa position in both the CA-074 non-treated group and the CA-074-treated group. Since bands were observed at the same position in the cathepsin B non-treated group, it is considered that these bands were derived from [¹¹¹In]In-AD not bound to cathepsin B.

### <Cell Internalization Experiment> (Examples 5-7 and Comparative Example 5-4)

A cell internalization experiment was performed in the same manner as in Examples 4-1 to 4-3. Regarding the medium fraction after 24 and 48 hours of incubation, separation based on molecular size was performed using Amicon Ultra-4 (recovered fraction: 50 kDa), and the radioactivity derived from the radiolabeled antibody drug dissociated from the cell surface and the radioactive component derived from the cell metabolites were separated. The concentrated solution was recovered as a dissociated fraction, and radioactivity was measured with a gamma counter. The radioactivity remaining in the Amicon was defined as the radioactivity of the degraded fraction. Significance testing in cell internalization experiments was performed by combining one-way analysis of variance and Bonferroni post-hoc test with a 95% confidence interval. The percentage of radioactivity of each fraction is shown in Tables 25 and 26. In Tables 25 and 26, "N.D." indicates that no data is acquired. In addition, the results for the percentages of radioactivity of the cell internalized fraction (internalized) and the degraded fraction (degraded) are collectively shown in Fig. 7. For the cell internalized fraction, [¹¹¹In]In-TADCS after incubation for 24 and 48 hours showed a significantly higher value compared to [¹¹¹In]In-TAD, indicating that the intracellular retention of radioactivity is high. For the degraded fraction, [¹¹¹In]In-TADCS after 24 and 48 hours incubation showed a lower value compared to [¹¹¹In]In-TAD, and the difference was significant, especially after 48 hours of incubation.

**[Table 25]**

| **[¹¹¹In]In-TADCS** | | Incubation time (h) | | | | | |
|---|---|---|---|---|---|---|---|
| | | **0** | **1** | **2** | **4** | **24** | **48** |
| % of total bound radioactivity | Medium fraction | 6.0± 0.4 | 7.7 ± 1.6 | 6.9 ± 0.7 | 5.6 ± 0.4 | 9.3 ± 0.9 | 17.2 ± 1.9 |
| | Cell surface fraction | 89.8± 1.2 | 83.9 ± 4.3 | 82.1 ± 0.6 | 76.1 ± 0.4 | 56.8 ± 0.9 | 43.5 ± 3.5 |
| | Cell internalized fraction | 4.2± 0.8 | 8.5 ± 3.2 | 11.0 ± 1.3 | 18.3 ± 0.1 | 34.6 ± 0.6 | 39.4± 5.2 |
| | Dissociated fraction | N.D. | N.D. | N.D, | N.D. | 5.4 ± 0.6 | 9.4 ± 0.3 |
| | Degraded fraction | N.D. | N.D. | N.D. | N.D. | 4.7 ± 1.6 | 6.4 ± 0.7 |
| Ratio | Cell internalized fraction/Medium fraction | 0.70 ± 0.10 | 1.10 ± 0.30 | 1.63 ± 0.37 | 3.28 ± 0.23 | 3.75 ± 0.36 | 2.33 ± 0.55 |
| | Cell internalized fraction/ Degraded fraction | N.D. | N.D. | N.D. | N.D. | 7.99 ± 2.47 | 6.23 ± 1.46 |

**[Table 26]**

| **[¹¹¹In]In·TAD** | | Incubation time (h) | | | | | |
|---|---|---|---|---|---|---|---|
| | | **0** | **1** | **2** | **4** | **24** | **48** |
| % of total bound radioactivity | Medium fraction | 8.1 ± 1.6 | 11.4 ± 1.6 | 9.8 ± 3.6 | 9.3± 1.2 | 13.8 ± 1.0 | 21.3 ± 2.3 |
| | Cell surface fraction | 88.3 ± 1.3 | 79.3 ± 1.4 | 79.6± 1.8 | 71.4 ± 3.0 | 56.9 ± 0.6 | 49.0 ± 0.9 |
| | Cell internalized fraction | 3.6 ± 0.4 | 9.3 ± 1.6 | 10.6 ± 1.8 | 19.3 ± 2.1 | 29.3 ± 0.6 | 29.7 ± 2.7 |
| | Dissociated fraction | N.D. | N.D. | N.D. | N.D. | 5.7 ± 0.6 | 8.8 ± 1.8 |
| | Degraded fraction | N.D. | N.D. | N.D. | N.D. | 8.8 ± 1.8 | 14.7 ± 3.0 |
| Ratio | Cell internalized fraction/Medium fraction | 0.46 ± 0.14 | 0.83 ± 0.22 | 1.24 ± 0.67 | 2.10 ± 0.18 | 2.14 ± 0.21 | 1.41 ± 0.28 |
| | Cell internalized fraction/ Degraded fraction | N.D. | N.D. | N.D. | N.D. | 3.44 ± 0.64 | 2.11 ± 0.70 |

### <Intracellular Cathepsin B Binding Experiment> (Example 6-7)

The intracellular cathepsin B binding experiment was generally performed according to a previous report (J. Nucl. Med., 2020, 61, 443-450). PBS (10 µL) with CA-074Me (1 mM) or without CA-074Me, a cathepsin B inhibitor, was added to the SK-OV-3 cell suspension (1.0 × 10⁷ cells/1 mL). Further, [¹¹¹In]In-TADCS (740 kBq, 100 µg) was added, and the mixture was incubated at 37°C for 24 hours. After incubation, the cell suspension was centrifuged at 860 × g for 3 min and the supernatant was removed. The cell mass was resuspended in 0.2M sodium citrate buffer solution (500 µL) at pH 2. After incubation at 4°C for 3 minutes, centrifugation was performed to remove the supernatant containing the radiolabeled antibody drug dissociated from the cell surface. The acid washing was performed again. A solution (1 µL) prepared by dissolving one tablet of cOmplete^{™} Protease Inhibitor Cocktail in 2 mL of distilled water, and a solution prepared by mixing RIPA buffer (100 µL) were added to the cell mass, and ultrasonication was performed for 1 minute. The suspension was centrifuged at 12,000 × g for 5 minutes and the supernatant was recovered. Laemmli sample buffer (2X) (40 µL) was added to the supernatant (40 µL), and the mixture was incubated at 85°C for 2 minutes. Each solution (15 µL) and Precision Plus Protein^{™} Dual Xtra Standards (15 µL) as the molecular weight marker were injected into a polyacrylamide gel, and SDS-PAGE was performed at 20 mA for 80 minutes. After completion of electrophoresis, the gel was removed, and the [¹¹¹In]InCl₃ solution (0.1 to 0.2 kBq, 0.5 µL) was added dropwise to the position of each band of the molecular weight marker. The removed gel was brought into contact with the imaging plate under light-shielded conditions for 7 days, and then autoradiographic imaging was performed using Amersham Typhoon scanner.

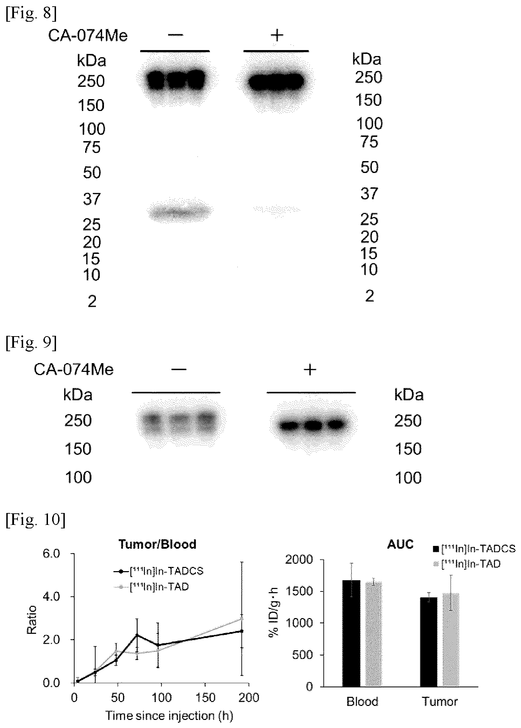

The imaging results of autoradiography are shown in Fig. 8. A band derived from radioactivity was confirmed at the position of a molecular weight of 30 kDa in a CA-074Me non-treated group, while a band was hardly confirmed in a CA-074Me-treated group. In both the CA-074Me non-treated group and the CA-074Me-treated group, radioactivity was detected at the position of a molecular weight of 200 to 250 kDa. Focusing only on the bands at the 200 to 250 kDa position, the results with adjusted contrast are shown in Fig. 9. In the CA-074Me non-treated group, in addition to the band around 200 kDa similar to that in the CA-074Me-treated group, a band was also observed around 250 kDa on the higher molecular weight side. This is considered to be derived from the complex of unmetabolized [¹¹¹In]In-TADCS and cathepsin B.

### <In Vivo Radioactivity Distribution Experiment Using Tumor Transplantation Model Mice> (Example 7-7 and Comparative Example 7-4)

Model mice were prepared by the same method as in Examples 5-1 to 5-3, and an in vivo radioactivity distribution experiment was conducted. The administered radioactivity was 130 kBq and 15 µg for both [¹¹¹In]In-TADCS and [¹¹¹In]In-TAD. These results are shown in Tables 27 to 28, categorized by the radiolabeled antibody drug administered to the mice. In addition, the area under the curve (AUC) of blood and tumor was calculated using GraphPad Prism software based on the results of the in vivo radioactivity distribution experiment without decay correction.

**[Table 27]**

| **[¹¹¹In]In-TADCS** | Tissue | Time after administration (h) | | | | | |
|---|---|---|---|---|---|---|---|
| | | **4** | **24** | **48** | **72** | **96** | **192** |
| | Blood | 44.73 (11.94) | 21.10 (3.70) | 19.86 (4.38) | 13.44 (3.81) | 9.22 (2.90) | 8.56 (4.02) |
| | Spleen | 11.45 (4,71) | 8.79 (2.97) | 11.12 (5.42) | 6.89 (0.95) | 11.67 (8.83) | 6.18 (2.36) |
| | Pancreas | 2.88 (0.84) | 5.28 (1.54) | 5.10 (0.90) | 3.25 (0.81) | 4.31 (0.57) | 2.49 (1.11) |
| | Stomach (%ID) | 0.39 (0.11) | 0.39 (0.06) | 0.40 (0.13) | 0.36 (0.14) | 0.30 (0.07) | 0.33 (0.13) |
| | Intestine | 5.49 (2.79) | 2.43 (0.24) | 2.30 (0.69) | 2.18 (0.42) | 1.71 (0.35) | 1.30 (0.38) |
| % ID/g | Kidney | 11.79 (2,10) | 9.50 (1.39) | 8.85 (2.23) | 6.67 (1.13) | 6.68 (3.43) | 4.80 (0.96) |
| | Liver | 21.11 (7.36) | 14.16 (6.49) | 19.33 (15.91) | 12.51 (1.40) | 24.96 (18.40) | 8.57 (1.66) |
| | Heart | 7.83 (1.83) | 4.86 (2.89) | 3.91 (2.19) | 2.99 (1.01) | 3.39(1.13) | 1.77 (0.28) |
| | Lung | 21.44 (4.99) | 12.78 (4.01) | 12.80 (3.24) | 7.81 (1.61) | 8.08 (3.63) | 6.41 (2.37) |
| | Brain | 1.04 (0.22) | 0.72 (0.37) | 0.58 (0.29) | 0.32 (0.10) | 0.44 (0.24) | 0.21 (0.12) |
| | Tumor | 3.74 (1.59) | 10.45 (1.70) | 20.89 (3.67) | 29.66 (5.94) | 16.21 (2.41) | 20.53 (6.17) |
| | Muscle | 1.47 (0.04) | 2.04 (0.98) | 1.50 (0.44) | 1.17 (0.24) | 0.82 (0.22) | 0.67 (0.33) |
| Ratio | Tumor/Blood | 0.09 (0.04) | 0.51 (0.16) | 1.07 (0.24) | 2.31 (0.76) | 2.00 (1.02) | 2.64 (0.76) |
| | Tumor/Muscle | 2.56 (1.14) | 5.92 (2.59) | 14.49 (3.28) | 25.59 (5.40) | 21.29 (7.31) | 33.03 (7,26) |

**[Table 28]**

| **[¹¹¹In]In-TAD** | Tissue | Time after administration (h) | | | | | |
|---|---|---|---|---|---|---|---|
| | | **4** | **24** | **48** | **72** | **96** | **192** |
| | Blood | 33.22 (4.64) | 21.05 (4.49) | 17.77 (3.30) | 16.00 (4.54) | 3.56 (1.39) | 9.03 (4.59) |
| | Spleen | 5.89 (1.69) | 5.45 (1.06) | 5.79 (2.70) | 5.15 (0.67) | 4.31 (0.65) | 3.29 (1.10) |
| | Pancreas | 3.22 (0.54) | 2.82 (0.77) | 4.73 (0.58) | 2.64 (1.38) | 1.31 (0.24) | 1.46 (0.62) |
| | Stomach (%ID) | 0.36 (0.10) | 0.36 (0.05) | 0.51 (0.16) | 0.31 (0.12) | 0.27 (0.09) | 0.19 (0,04) |
| | Intestine | 2,43 (0.08) | 1.89 (0.18) | 2.06 (0.30) | 1.84 (0.02) | 1.43 (0.16) | 0.93 (0.35) |
| % ID/g | Kidney | 9.07 (2.46) | 7.38 (1.19) | 6.05 (0.62) | 5.69 (1.68) | 4.81 (0.71) | 2.80 (0.96) |
| | Liver | 13.86 (5.80) | 8.43 (1.31) | 10.12 (2.12) | 8.02 (1.80) | 9.10 (0.42) | 4.60 (0.80) |
| | Heart | 7.11 (2.45) | 3.97 (0.79) | 4.40 (0.84) | 2.94 (0.33) | 3.43 (0.41) | 2.11 (0.88) |
| | Lung | 13.94 (4.36) | 13.24 (2.48) | 8.78 (1.33) | 8.41 (1.25) | 8.47 (0.73) | 5.14 (1.80) |
| | Brain | 0.67 (0.06) | 0.48 (0.13) | 0.44 (0.12) | 0.37 (0.08) | 0.38 (0.05) | 0.19 (0.10) |
| | Tumor | 2.06 (1.99) | 11.18 (1.50) | 26.12 (14.19) | 21.80 (7.13) | 20.28 (11.88) | 26.82 (12.30) |
| | Muscle | 1.07 (0.17) | 1.57 (0.42) | 2.22 (0.80) | 1.11 (0.19) | 0.82 (0.22) | 0.60 (0.17) |
| Ratio | Tumor/Blood | 0.06 (0.05) | 0,55 (0.15) | 1.52 (0.98) | 1.38 (0.28) | 1.46 (0.79) | 3.79 (2,63) |
| | Tumor/Muscle | 1.78 (1.45) | 7,72 (3.28) | 11.99 (6.56) | 19.59 (4.45) | 28.32 (20.49) | 46.07 (17.79) |

[¹¹¹In]In-TADCS and [¹¹¹In]In-TAD showed high blood retention and similar accumulation tendencies in tumors and other organs. For tumor accumulation, [¹¹¹In]In-TADCS showed a maximum value at 72 hours after administration, and [¹¹¹In]In-TAD showed a maximum value at 192 hours after administration. The tumor/blood ratio of radioactivity accumulation and the AUC value in tumor and blood are shown in Fig. 10.

### · Synthesis of Radiolabeled Antibody Drugs (Examples 2-8 and 2-9 and Comparative Example 2-5)

### <Introduction of Thiol Group into Humanized SKM9-2 Antibody>

In the present Examples and Comparative Example, the humanized SKM9-2 antibody described in Production Example 3 of WO 2023/277144 was used as an antibody.

A solution of humanized SKM9-2 antibody (891 µg) in 0.1 mol/L borate buffer solution containing 2 mmol/L EDTA (pH 8.0, 72.1 µL) was prepared. In addition, a 2-iminothiolane hydrochloride solution with a concentration of 0.7 mmol/L was prepared by dissolving 2-iminothiolane hydrochloride in the 0.1 mol/L borate buffer solution containing 2 mmol/L EDTA (pH 8.0). Then, the 2-iminothiolane hydrochloride solution was added to the humanized SKM9-2 solution. The amount of the 2-iminothiolane hydrochloride solution added was such that the amount of 2-iminothiolane hydrochloride was 2 molar equivalents with respect to SKM9-2. The reaction solution obtained in this manner was incubated at room temperature under light-shielded conditions for 1 hour. The reaction solution was purified using Zeba Spin Desalting Column (40K MWCO, 0.5 mL) to obtain humanized SKM9-2 into which thiol groups had been introduced (hereinafter also referred to as "SKM9-2-SH"). The purified SKM9-2-SH was promptly used in the following labeling reaction.

### <Labeling Reaction>

To the [¹¹¹In]In-MDPEI2, [¹¹¹In]In-MD, and [¹¹¹In]In-MDPEG48 in a 0.1 mol/L phosphate buffer solution (pH 7, 500 µL) prepared in the same manner as in Example 2-1, Comparative Example 2-1, and Example 2-3, the 0.1 mol/L phosphate buffer solution (pH 7, 100 µL) of SKM9-2-SH (0.4 to 0.5 mg) was added, and the mixture was incubated for 7.5 to 10 hours at room temperature under light-shielded conditions. Thereafter, the reaction solution was purified and concentrated using Amicon Ultra-4 (recovered fraction: 50 kDa) to obtain a target radiolabeled antibody drug. RCP was measured as follows. Analysis was performed by the size exclusion HPLC using the TSKgel SW3000_{XL} column (5 µm, 7.8 mm I.D. × 300 mm) and the mobile phase (0.1 mol/L phosphate buffer solution (pH 7), flow rate: 0.5 mL/min), and the percentage of the area value of the radiolabeled compound relative to the area values of all detected peaks was defined as RCP (%).

In the present specification, RICs obtained by introducing [¹¹¹In]In-MDPEI2, [¹¹¹In]In-MD, and [¹¹¹In]In-MDPEG48 into SKM9-2-SH are referred to as [¹¹¹In]In-SMDPEI2, [¹¹¹In]In-SMD, and [¹¹¹In]In-SMDPEG48, respectively. The RCY of the labeling reaction was calculated as a percentage of radioactivity of the ¹¹¹In-labeled antibody drug after purification relative to the amount of radioactivity of the added ¹¹¹In-labeled chelate linker.

The RCY of Example 2-8 ([¹¹¹In]In-SMDPEI2) was 70.6%, the RCY of Example 2-9 ([¹¹¹In]In-SMDPEG48) was 74.4%, the RCY of Comparative Example 2-5 ([¹¹¹In]In-SMD) was 60.4%, and the RCP of each of the synthesized RICs was all greater than 95%.

Sialylated HEG1-positive malignant pleural mesothelioma cells, ACC-MESO-4 cells (Cancer Sci 2006; 97:387-394), were provided by the RIKEN BioResource Research Center, National Research and Development Agency. The cells were cultured in RPMI-1640 medium (manufactured by Nacalai Tesque, Inc.) containing antibiotics (penicillin and streptomycin) (100 U/mL) and 10% by mass heat-inactivated fetal bovine serum (FBS), at 37°C under a 5% by volume CO₂ atmosphere.

### <Preparation of Tumor Transplantation Model Mice>

ACC-MESO-4 cells (2.0 × 10⁶ cells/mouse) were suspended in RPMI-1640 (100 µL) and subcutaneously transplanted to the back of male SHO mice (5 weeks old) under isoflurane anesthesia. The mice were housed for 4 to 5 weeks, and those with a tumor diameter reaching 0.5 cm were used in the following experiments.

### <In Vivo Radioactivity Distribution Experiment Using Tumor Transplantation Model Mice> (Examples 5-8 and 5-9 and Comparative Example 5-5)

A physiological saline solution (100 µL) containing [¹¹¹In]In-SMDPEI2, [¹¹¹In]In-SMD, and [¹¹¹In]In-SMDPEG48 (148 kBq, 20 µg) was administered via the tail vein of ACC-MESO-4 tumor transplantation model mice, and the mice were euthanized at 4, 24, 48, 72, 96, or 192 hours after administration (n = 3 or 4 each). Blood and each organ were collected, and each weight and radioactivity were measured. The value obtained by dividing the percentage of the amount of radioactivity relative to the administered radioactivity (%ID) by the blood weight or organ weight (g) (%ID/g) was determined as an index indicating the in vivo radioactivity distribution. The results are shown in Tables 29 to 31 for each RIC administered to mice.

In addition, the AUCs for blood and tumor were calculated using GraphPad Prism software based on the average %ID/g values at each time point without decay correction. The results are shown in Table 32.

**[Table 29]**

| **[¹¹¹In]In-SMDPEI2** | Tissue | Time after administration (h) | | | | | |
|---|---|---|---|---|---|---|---|
| | | **4** | **24** | **48** | **72** | **96** | **192** |
| | Blood | 26.77 (3.51) | 14.30 (1.16) | 11.56 (1.32) | 9.69 (0.71) | 8.52 (1.91) | 5.18 (0.96) |
| | Spleen | 3.81 (0.59) | 4.34 (0.81) | 5.36 (1.15) | 4.41 (2.10) | 3.47 | 4.40 (1.74) |
| | Pancreas | 1.02 | 1.10 | 1.09 | 1.10 | 0.99 (0.20) | 0.82 (0.11) |
| | Stomach (%ID) | 0.51 (0.14) | 0.39 (0.10) | 0.34 (0.03) | 0.37 (0.07) | 0.30 (0.06) | 0.22 (0.02) |
| | Intestine | 1.71 (0.28) | 1.12 (0.16) | 0.93 (0.10) | (0.16) 0.85 (0.11) | 0.71 (0.03) | 0.57 (0.14) |
| % ID/g | Kidney | (0.13) 6.05 (0.45) | (0.07) 5.51 (0.69) (0.3 | (0.03) 5.00 (1.14) ( 20.21 | 5.33 (0.31) | 4.56 (0.28) | 3.60 (0.66) (3.86) |
| | Liver | 4.84 (0.66) | 2.82 (0.09) | 2.89 (0.45) | 2.64 (0.31) | (0.70) 2.23 (0.17) | 2.29 (0.65) |
| | Heart | 5.70 (0.63) | 2.97 (0.54) | 2.75 (0.36) | 2.73 (0.40) | 2.20 (0.44) | 1.49 (0.47) |
| | Lung | 12.47 (0.76) | 7.74 1) | 6.46 1.56) | 5.25 (0.33) | 5.42 (0.39) | 2.90 (0.85) |
| | Brain | 0.50 (0.05) | 0.31 (0.03) | 0.24 (0.05) | 0.20 (0.02) | 0.19 (0.02) | 0.14 (0.08) |
| | Tumor | 7.04 (2.45) | 17.46 (6.48) | (4.62) | 18.37 (0.53) | 25.74 (2.19) | 20.99 |
| | Muscle | 0.83 (0.10) | 1.03 (0.24) | 0.91 (0.14) | 0.83 (0.07) | 0.74 (0.16) | 0.45 (0.07) |
| Ratio | Tumor/Blood | 0.26 (0.09) | 1.22 (0.44) | 1.76 (0.44) | 1.90 (0.17) | 3.16 (0.85) | 4.10 (0.71) |
| | Tumor/Muscle | 8.44 (2.90) | 18.29 (9.35) | 22.97 (7.86) | 22.26 (2.59) | 35.87 (7.10) | 47.20 (4.68) |

**[Table 30]**

| **[¹¹¹In]In-SMD** | Tissue | | Ti | me after adm | inistration (h) | | |
|---|---|---|---|---|---|---|---|
| | | **4** | **24** | **48** | **72** | **96** | **192** |
| | Blood | 20.31 (1.25) | 11.69 (1.19) | 10.29 (1.69) | 7.33 (0.75) | 7.94 (1.43) | 5.15 (0.73) |
| | Spleen | 3.69 (0.44) | 3.59 (0.90) | 4.05 (1.01) | 3.32 (0.68) | 3.04 (0.89) | 3.47 (0.61) |
| | Pancreas | 0.71 | 1.15 | 0.97 | 0.95 (0.24) | 1.01 (0.28) | 0.69 |
| | Stomach (%ID) | 0.36 (0.08) | 0.52 (0.18) | 0.35 (0.08) | 0.29 (0.05) | 0.29 (0.03) | 0.22 (0.01) |
| | Intestine | 2.41 (0.67) | (0.16) 1.72 (0.51) | 1.23 (0.12) | 0.87 (0.11) | 0.73 (0.14) | 0.49 (0.10) |
| *%* ID/g | Kidney | (0.13) 4.51 (0.70) | 3.96 (0.25) | (0.22) 3.93 (0.61) | 3.21 | 3.09 (0.33) | (0.05) 2.23 (0.55) |
| | Liver | 3.85 (0.52) | 3.46 (1.19) | 2.96 (0.53) | 2.72 (0.59) | 2.47 (0.54) | 1.57 (0.12) |
| | Heart | 3.68 (0.66) | 2.59 (0.14) | 2.67 (0.06) | 1.92 (0.34) | 2.01 (0.21) | 1.32 (0.25) |
| | Lung | 10.73 (1.25) | 6.71 (1.93) | 5.44 (0.71) | 3.77 (0.29) | 4.57 (0.70) | 3.18 (0.43) |
| | Brain | 0.38 (0.07) | 0.27 (0.04) | 0.24 (0.03) | 0.18 (0.01) | 0.20 (0.04) | 0.13 (0.03) |
| | Tumor | 5.26 (5.31) | 8.87 (1.48) | 14.65 (3.26) | 16.02 (1.26) | 17.02 (1.85) | 14.68 (3.16) |
| | Muscle | 1.18 (0.55) | 0.77 (0.10) | 0.75 (0.10) | 0.66 (0.05) | 0.67 (0.14) | 0.37 (0.04) |
| Ratio | Tumor/Blood | 0.27 (0.29) | 0.76 (0.12) | 1.46 (0.46) | 2.19 (0.21) | 2.17 (0.23) | 2.84 (0.40) |
| | Tumor/Muscle | 4.84 (4.87) | 11.83 (3.50) | 19.43 (2.71) | 24.37 (3.37) | 26.73 (9.02) | 39.44 (8.29) |

**[Table 31]**

| **[¹¹¹In]In-SMDPEG48** | Tissue | Time after administration (h) | | | | | |
|---|---|---|---|---|---|---|---|
| | | **4** | **24** | **48** | **72** | **96** | **192** |
| | Blood | 24.60 (1.82) | 16.47 (1.70) | 13.41 (1.70) | 11.79 (0.29) | 9.30 (2.55) | 6.79 (1.07) |
| | Spleen | 3.67 | 4.19 (1.14) | 4.22 (1.47) | 3.28 (0.13) | 4.41 (1.44) | 3.67 |
| | Pancreas | 0.96 (0.15) | 1.24 (0.21) | 1.17 (0.17) | 1.05 | 1.36 (0.50) | 0.76 (0.11) |
| | Stomach (%ID) | 0.48 (0.09) | 0.46 (0.05) | 0.41 (0.09) | 0.42 (0.10) | 0.40 (0.10) | 0.30 (0.07) |
| | Intestine | 1.60 (0.32) | 1.12 (0.13) | 1.10 (0.12) | 1.06 (0.12) | 1.01 (0.11) | 0.68 (0.06) |
| *% ID*/*g* | Kidney | (1.10) 4.82 (0.80) | 4.74 (0.83) (0.17) | 4.20 (0.92) | (0.05) 3.64 (0.50) | 4.04 (1.11) | (1.39) 2.87 (0.60) |
| | Liver | 3.78 (0.39) | 3.04 | 3.12 (0.98) | 2.93 (0.40) | 3.83 (1.28) | 2.10 (0.23) |
| | Heart | 4.85 (0.39) | 3.55 (0.53) | 2.67 (0.45) | 2.39 (0.64) | 2.46 (0.09) | 1.65 (0.32) |
| | Lung | 11.92 (0.94) | 8.61 (1.32) | 7.78 (1.86) | 7.11 (0.64) | 5.88 (1.90) | 4.39 (0.66) |
| | Brain | 0.48 (0.11) | 0.35 (0.04) | 0.31 (0.06) | 0.26 (0.05) | 0.21 (0.02) | 0.17 (0.03) |
| | Tumor | 5.67 (1.94) | 22.10 (1.17) | 24.18 (5.22) | 26.39 (5.14) | 28.52 (5.82) | 32.32 (8.60) |
| | Muscle | 0.63 (0.14) | 0.83 (0.14) | 0.93 (0.08) | 0.83 (0.15) | 0.87 (0.21) | 0.50 (0.13) |
| Ratio | Tumor/Blood | 0.23 (0.09) | 1.35 (0.14) | 1.80 (0.31) | 2.24 (0.40) | 3.33 (1.44) | 4.71 (0.66) |
| | Tumor/Muscle | 10.11 (6,77) | 27.23 (5.23) | 25.84 (4.15) | 32.41 (7.66) | 33.45 (6.71) | 65.39 (10.10) |

**[Table 32]**

| | Tumor (%ID/g·h) | Blood (%ID/g·h) | Tumor/Blood |
|---|---|---|---|
| [¹¹¹In]In-SMDPEI2 | 1837 | 1217 | 1.51 |
| [¹¹¹In]In-SMD | 1253 | 1038 | 1.21 |
| [¹¹¹In]In-SMDPEG48 | 2301 | 1325 | 1.74 |

As shown in Tables 29 to 31, regarding the radioactivity uptake of the tumor, all the ¹¹¹In-labeled antibodies reached the maximum values at the late stage of administration, [¹¹¹In]In-SMDPEI2 showed 25.7 ± 2.2%ID/g at 96 hours after administration, [¹¹¹In]In-SMD showed 17.0 ± 1.9%ID/g at 96 hours after administration, and [¹¹¹In]In-SMDPEG48 was 32.3 ± 8.6%ID/g at 192 hours after administration. In addition, tumor accumulation at 96 hours after administration of [¹¹¹In]In-SMDPEI2 and [¹¹¹In]In-SMDPEG48 was 25.7 ± 2.2%ID/g and 28.5 ± 5.8%ID/g, respectively, and compared to [¹¹¹In]In-SMD, [¹¹¹In]In-SMDPEI2 and [¹¹¹In]In-SMDPEG48 exhibited excellent tumor accumulation.

As shown in Table 32, regarding the AUC in the tumor, [¹¹¹In]In-SMDPEG48 showed the highest value, and [¹¹¹In]In-SMDPEI2 also showed a higher value compared to [¹¹¹In]In-SMD. Regarding AUC in blood, [¹¹¹In]In-SMDPEI2 and [¹¹¹In]In-SMDPEG48 showed higher values compared to [¹¹¹In]In-SMD. The tumor/blood ratio of AUC was [¹¹¹In]In-SMDPEG48, [¹¹¹In]In-SMDPEI2, and [¹¹¹In]In-SMD from high RIC, and the same tendency as when each chelate linker was applied to trastuzumab was observed.

### <In Vivo Blocking Experiment Using Tumor Transplantation Model Mice>

### (Example 6-9)

A physiological saline solution (100 µL) containing [¹¹¹In]In-SMDPEG48 (148 kBq) and humanized SKM9-2 antibody (2.0 mg/mouse) was administered via the tail vein of ACC-MESO-4 tumor transplantation model mice, and the mice were euthanized at 72 hours after the administration (n = 4). Blood and each organ were collected, and each weight and radioactivity were measured. Significance testing in in vivo blocking experiments was performed using Student's t-test with a 95% confidence interval. The results are shown in Table 33.

**[Table 33]**

| **[¹¹¹In]In-SMDPEG48 + Blocking** | Tissue | Time after administration (h) |
|---|---|---|
| | | **72** |
| | Blood | 6.83 (0.64) |
| | Spleen | 3.21 (0.76) |
| | Pancreas | 1.05 (0.26) |
| | Stomach (%ID) | 0.34 (0.08) |
| | Intestine | 0.82 (0.13) |
| % ID/g | Kidney | 1.87 (0.31) |
| | Liver | 3.57 (0.76) |
| | Heart | 3.24 (0.93) |
| | Lung | 4.22 (0.42) |
| | Brain | 0.19 (0.03) |
| | Tumor | 4.24 (1.94) |
| | Muscle | 0.56 (0.09) |
| Ratio | Tumor/Blood | 0.62 (0.25) |
| | Tumor/Muscle | 7.85 (4.54) |

As shown in Table 33, co-administration of humanized SKM9-2 antibody significantly reduced the tumor accumulation of [¹¹¹In]In-SMDPEG48 (4.2 ± 1.9%ID/g). A significant reduction was also observed in the amount of radioactivity in the blood (6.8 ± 0.6%ID/g), and a significant reduction was also observed in the tumor/blood ratio (0.62 ± 0.25), indicating the target specificity of [¹¹¹In]In-SMDPEG48 in vivo.

According to the present invention, there is provided a radioactive metal-labeled antibody capable of enhancing radioactivity accumulation within cells of target cells.

The present application is based on Japanese Patent Application No. 2023-032317 (filed on March 2, 2023) and Japanese Patent Application No. 2023-203279 (filed on November 30, 2023), the contents of which are entirely incorporated herein.

## Claims

1. A radioactive metal-labeled antibody comprising an antibody labeled with a radioactive metal, wherein
the radioactive metal-labeled antibody further comprises a functional chelate linker conjugated to the antibody, and
the functional chelate linker includes:
a chelating moiety capable of coordinating to a radioactive metal ion; and
a functional unit having a function of enhancing intracellular retention.

2. The radioactive metal-labeled antibody according to claim 1, wherein the functional unit has:
a structure in which ethyleneimine or ethylene glycol is polymerized, or
a structure capable of binding to cathepsin B.

3. The radioactive metal-labeled antibody according to claim 2, wherein the functional unit has a structure in which ethyleneimine is polymerized with a polymerization degree of 1 to 7.

4. The radioactive metal-labeled antibody according to claim 2, wherein the functional unit has a structure in which ethylene glycol is polymerized with a polymerization degree of 11 to 48.

5. The radioactive metal-labeled antibody according to claim 2, wherein the functional unit has a structure represented by Formula (c) below: 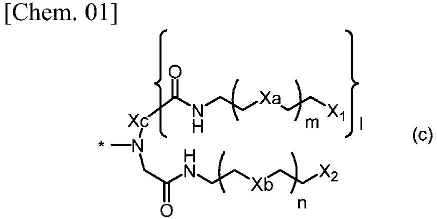
(in Formula (c), X₁ and X₂ each independently represent a hydrogen atom, an alkyl group having 1 to 4 carbon atoms, a hydroxy group, an amino group, or a bond,
Xₐ and X_{b} each independently represent O or NH,
1 represents 0 or 1,
Xc represents a hydrogen atom when 1 is 0, and represents CH₂ when 1 is 1,
m and n each independently represent the polymerization degree of ethyleneimine or ethylene glycol, and
* represents a bond).

6. The radioactive metal-labeled antibody according to claim 5, wherein the functional chelate linker has a structure represented by Formula (d) below: 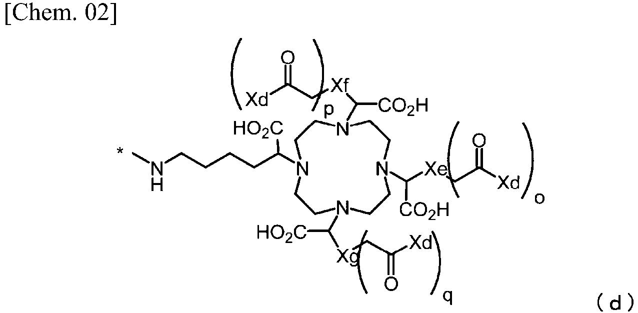
(in Formula (d), o, p, and q each independently represent 0 or 1, and satisfy 1 ≤ o + p + q ≤ 3,
Xd represents a structure represented by the Formula (c),
Xe represents a hydrogen atom when o is 0, and represents CH₂ when o is 1,
Xf represents a hydrogen atom when p is 0, and represents CH₂ when p is 1,
Xg represents a hydrogen atom when q is 0, and represents CH₂ when q is 1, and
* is a bond).

7. The radioactive metal-labeled antibody according to claim 2, wherein the structure capable of binding to cathepsin B is an epoxysuccinyl peptide.

8. The radioactive metal-labeled antibody according to claim 1 or 2, wherein the chelating moiety is interposed between the antibody and the functional unit.

9. The radioactive metal-labeled antibody according to claim 8, wherein
the functional chelate linker further includes an antibody binding unit having a binding site with the antibody,
the chelating moiety has first and second amino acid residues, and
the chelating moiety is bound to the antibody binding unit via the first amino acid residue, and the chelating moiety is bound to the functional unit via the second amino acid residue.

10. The radioactive metal-labeled antibody according to claim 1 or 2, wherein the chelating moiety is DOTA or a derivative thereof.

11. The radioactive metal-labeled antibody according to claim 1 or 2, wherein the radioactive metal is one selected from Al¹⁸F, ⁶⁴Cu, ⁶⁷Cu, ⁶⁸Ga, ⁸⁹Zr, ⁹⁰Y, ^{99m}Tc, ¹¹¹In, ¹⁷⁷Lu, and ²²⁵Ac.

12. The radioactive metal-labeled antibody according to claim 1 or 2, wherein the functional chelate linker is introduced into a thiol group of the antibody.

13. The radioactive metal-labeled antibody according to claim 1 or 2, wherein the antibody is an antibody that recognizes an antigen specifically expressed in a tumor cell.

14. A radioactive pharmaceutical comprising the radioactive metal-labeled antibody according to claim 1 or 2 as an active ingredient.

15. The radioactive pharmaceutical according to claim 14, which is used for diagnosis of cancer or internal radiation therapy for cancer.

16. A compound comprising, in its structure:
a chelating moiety capable of coordinating to a radioactive metal ion;
a functional unit having a function of enhancing intracellular retention; and
an antibody binding unit including a binding site with an antibody.

17. The compound according to claim 16, wherein the chelating moiety is interposed between the antibody binding unit and the functional unit.

18. The compound according to claim 16 or 17, wherein the binding site with the antibody is a maleimide group.
